# EUROPEAN PATENT SPECIFICATION

(11) **EP 3 166 638 B1**
(45) Date of publication and mention of the grant of the patent: **06.08.2025**
(21) Application number: 15733782.5
(22) Date of filing: 12.06.2015
(51) Int. Cl.: A61K 41/00, A61K 47/68, A61K 47/61, A61P 35/00

(54) **PAYLOAD-POLYMER-PROTEIN CONJUGATES**
PAYLOAD-POLYMER-PROTEIN-KONJUGATE
CONJUGUÉS CHARGE UTILE-POLYMÈRE-PROTÉINE

(30) Priority: 13.06.2014 FI 20145551; 20.02.2015 FI 20155113
(43) Date of publication of application: 17.05.2017
(73) Proprietor: Glykos Finland Oy, 00790 Helsinki (FI)
(72) Inventor: HELIN, Jari, 05200 Rajamäki (FI); SAARINEN, Juhani, 00370 Helsinki (FI); EKHOLM, Filip S., 06150 Porvoo (FI); LEPPÄNEN, Anne, 01360 Vantaa (FI)
(74) Representative: Papula Oy
(86) International application number: PCT/FI2015/050423
(87) International publication number: WO 2015/189478

(56) References cited:
- WO-A1-2014/096551
- WO-A2-2009/100194
- GEDDA L ET AL: "DEVELOPMENT AND IN VITRO STUDIES OF EPIDERMAL GROWTH FACTOR-DEXTRAN CONJUGATES FOR BORON NEUTRON CAPTURE THERAPY", BIOCONJUGATE CHEMISTRY, ACS, WASHINGTON, DC, US, vol. 7, no. 5, 1 September 1996 (1996-09-01), pages 584 - 591, XP008044179, ISSN: 1043-1802, DOI: 10.1021/BC9600473
- BRICH Z ET AL: "Preparation and characterization of a water soluble dextran immunoconjugate of doxorubicin and the monoclonal antibody (ABL 364)", JOURNAL OF CONTROLLED RELEASE, ELSEVIER, AMSTERDAM, NL, vol. 19, no. 1-3, 1 March 1992 (1992-03-01), pages 245 - 257, XP023744406, ISSN: 0168-3659, [retrieved on 19920301], DOI: 10.1016/0168-3659(92)90080-B
- LAURENT PETTERSSON M ET AL: "Immunoreactivity of boronated antibodies", JOURNAL OF IMMUNOLOGICAL METHODS, ELSEVIER SCIENCE PUBLISHERS B.V.,AMSTERDAM, NL, vol. 126, no. 1, 24 January 1990 (1990-01-24), pages 95 - 102, XP023987014, ISSN: 0022-1759, [retrieved on 19900124], DOI: 10.1016/0022-1759(90)90016-O
- BARTH R F ET AL: "Neutron capture therapy of epidermal growth factor (+) gliomas using boronated cetuximab (IMC-C225) as a delivery agent", APPLIED RADIATION AND ISOTOPES, ELSEVIER, OXFORD, GB, vol. 61, no. 5, 1 November 2004 (2004-11-01), pages 899 - 903, XP004526469, ISSN: 0969-8043, DOI: 10.1016/J.APRADISO.2004.05.004
- ERIK S. ZIMMERMAN ET AL: "Production of Site-Specific Antibody-Drug Conjugates Using Optimized Non-Natural Amino Acids in a Cell-Free Expression System", BIOCONJUGATE CHEMISTRY, vol. 25, no. 2, 17 January 2014 (2014-01-17), pages 351 - 361, XP055107336, ISSN: 1043-1802, DOI: 10.1021/bc400490z

## Description

### FIELD OF THE INVENTION

The invention relates to a conjugate, a pharmaceutical composition, and a method of preparing the conjugate.

### BACKGROUND OF THE INVENTION

Conjugates of payload molecules such as cytotoxic drugs with proteins, for instance antibodies, may be useful, for instance, in the therapy of cancer. The conjugates currently available utilize various chemistries to conjugate payload molecules to proteins; however, many of them may not be optimal in terms of e.g. activity of the payload molecule, aqueous solubility of the conjugate or the reaction conditions required for conjugation.

For instance, a bulky conjugate or a conjugate having suboptimal solubility may not be efficiently delivered to its target. A payload molecule may not always be efficiently released from the protein and/or delivered into cells or into various parts of cells. The activity, such as toxicity, of the payload molecule may be reduced as a result of the conjugation. In some cases, linkage of the payload molecule may not be stable towards chemical or biochemical degradation during manufacturing or in physiological conditions, e.g. in blood, serum, plasma or tissues. Furthermore, conjugation of the payload molecule to one or more random positions and/or chemical groups of the protein may impair the pharmacokinetic properties of the conjugate or the specificity of the protein, such as an antibody, towards its target.

Brich Z. et al., Journal of Controlled Release (1992), vol. 19, no. 1-3, pages 245-257 disclose an immunoconjugate consisting of the monoclonal antibody ABL 364 and the cytostatic drug doxorubicin using oxidized dextran as a carrier.

Pettersson M.L. et al., Journal of Immunological Methods (1990), vol. 126, no. 1, pages 95-102 disclose dextran-boronated monoclonal antibodies.

Gedda L. et al., Bioconjugate Chemistry (1996), vol. 7, no. 5, pages 584-591 disclose a conjugate of EGF and sulfhydryl boron hydride (BSH) to an allylated dextran.

### SUMMARY OF THE INVENTION

The conjugate according to the present invention is characterized by what is presented in claim 1.

The method for preparing the conjugate according to the present invention is characterized by what is presented in claim 8.

The pharmaceutical composition according to the present invention is characterized by what is presented in claim 16.

The conjugate or pharmaceutical composition for use as a medicament according to the present invention is characterized by what is presented in claim 17.

The conjugate or pharmaceutical composition for use in the treatment of cancer according to the present invention is characterized by what is presented in claim 17.

### BRIEF DESCRIPTION OF THE DRAWINGS

The accompanying drawings, which are included to provide a further understanding of the invention and constitute a part of this specification, illustrate embodiments of the invention and together with the description help to explain the principles of the invention. In the drawings:
**Figure 1****.** Proton-NMR spectrum of BSH-dextran. The boron linked protons resonate between 0.8-2.0 ppm, and the boron load of BSH-dextran can be estimated by comparing the integral of boron-protons to the integral of dextran protons. Unreacted allyl groups yield signals at 4.22, 5.29, 5.39 and 5.99 ppm. Sharp signal at 2.225 ppm is acetone (internal standard).
**Figure 2****.** Gel filtration analysis of BSH-Dex-conjugates.
   A. Anti-EGFR1-Fab-BSH(800B)-Dex. Conjugate elutes at 7.8 ml when analysed with Yarra SEC-3000 gel filtration column. By comparison, anti-EGFR1-Fab elutes at 9.1 ml. B. Anti-EGFR1-Fab2-BSH(800B)-Dex. Conjugate elutes at 6.9 ml when analysed with Yarra SEC-3000 gel filtration column. By comparison, anti-EGFR1-Fab2 elutes at 8.4 ml.

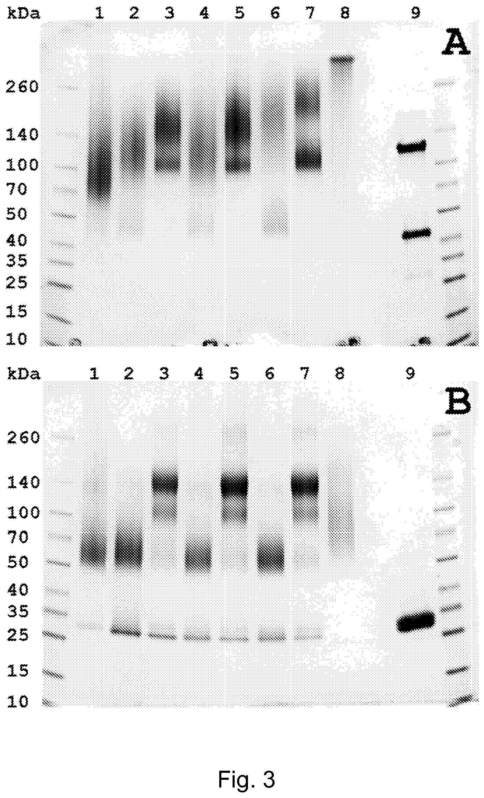

**Figure 3****.** SDS-PAGE analysis of fluorescently labeled anti-EGFR1 Fab/F(ab')2 boron conjugates with different amounts of boron in nonreducing (panel A) and reducing (panel B) conditions. Anti-EGFR1-Fab-BSH-Dex conjugates: Lane 1 (900B), lane 2 (700B), lane 4 (560B), lane 6 (360B). Anti-EGFR1-F(ab')2-BSH-Dex conjugates: Lane 3 (700B), lane 5 (560B), lane 7 (360B). Lane 8 is Anti-EGFR1-Fab-Dex and lane 9 is a control containing a mixture of anti-EGFR1-F(ab')2 and Fc fragments (Fab fragments migrate like Fc fragments on the gel). Gel staining with Coomassie Blue.

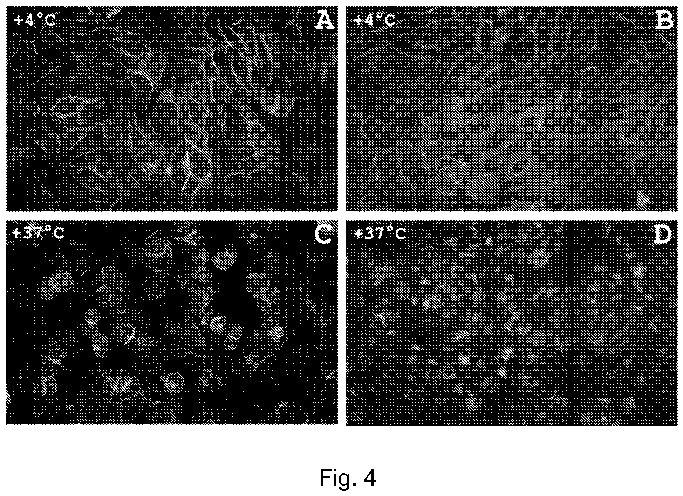

**Figure 4****.** Cell surface binding and internalization of fluorescently labeled anti-EGFR1-F(ab')2 (Panels A and C) and anti-EGFR1-F(ab')2-BSH(900B)-Dex (Panels B and D) by HSC-2 cells. Incubations have been performed at +4 °C (binding to the cell surface) and at +37 °C (binding to cell surface and internalization). Analysis has been carried out by fluorescence microscopy.

### DETAILED DESCRIPTION

Any references in the description to methods of treatment refer to the compounds, pharmaceutical compositions and medicaments of the present invention for use in a method for treatment of the humal or animal body by therapy.

The present invention relates to a conjugate according to claim 1 represented by the formula

[D-L-Y-(CH₂)ₙ-O]ₘ-P-T

wherein
T is an antibody;
P is a polymer selected from the group consisting of dextran, hyaluronic acid, hydroxyethyl starch, and cyclodextrin;
m is at least 1;
n is in the range of 2 to 10;
each Y is independently selected from the group consisting of S, NH and 1,2,3-triazolyl, wherein 1,2,3-triazolyl is optionally substituted;
each L is independently absent or comprises a linker group covalently joining D and Y; and
each D is a payload molecule.

Dextran, hyaluronic acid, hydroxyethyl starch, and cyclodextrin comprise at least one hydroxyl group. The presence of the at least one hydroxyl group allows the linking of one or more substituents to the polymer as described herein. Many of these polymers also comprise saccharide units that may be further modified, e.g. oxidatively cleaved, to introduce functional groups to the polymer. P may thus also be a polymer derivative.

In this specification, the term "saccharide unit" should be understood as referring to a single monosaccharide moiety.

In this specification, the term "saccharide" should be understood as referring to a monosaccharide, disaccharide or an oligosaccharide.

The value of m may depend e.g. on the polymer, on the payload molecule, the linker group, and the method of preparing the conjugate. Typically, a large value of m may led to higher efficiency of the conjugate; on the other hand, a large value of m may in some cases affect other properties of the conjugate, such as pharmacokinetic properties or solubility, adversely. In an embodiment, m is in the range of 1 to about 300, or in the range of about 10 to about 200, or in the range of about 20 to about 100, or in the range of about 20 to about 150. In an embodiment, m is in the range of 1 to about 20, or in the range of 1 to about 15 or in the range of 1 to about 10. In an embodiment, m is 1, 2, 3, 4, 5, 6, 7, 8, 9, 10, 11, 12, 13, 14, 15, 16, 17, 18, 19, or 20. In an embodiment, m is 2-16. In an embodiment, m is in the range of 2 to 10. In other embodiments, n is in the range of 2 to 6; 2 to 5; 2 to 4; 2 or 3; or 3 or 4.

In an embodiment, about 25-45% of carbons of the polymer bearing a hydroxyl group are substituted by a substituent of the formula D-L-Y-(CH₂)ₙ-O-.

In embodiments in which the polymer comprises a plurality of saccharide units, the ratio of m to the number of saccharide units of the polymer may be e.g. 1:20 to 1:3 or 1:4 to 1:2.

In an embodiment, n is 2, 3, 4, 5, 6, 7, 8, 9 or 10. In an embodiment, n is in the range of 2 to 9, or in the range of 3 to 8, or in the range of 4 to 7, or in the range of 2 to 6, or in the range of 2 to 5, or in the range of 2 to 4.

Each n may, in principle, be independently selected. Each n in a single conjugate may also be the same.

In an embodiment, Y is S.

In an embodiment, Y is NH.

In an embodiment, Y is 1,2,3-triazolyl. In this specification, the term "1,2,3-triazolyl" should be understood as referring to 1,2,3-triazolyl, or to 1,2,3-triazolyl which is substituted. In an embodiment, the 1,2,3-triazolyl is a group formed by click conjugation comprising a triazole moiety. Click conjugation should be understood as referring to a reaction between an azide and an alkyne yielding a covalent product - 1,5-disubstituted 1,2,3-triazole - such as copper (I)-catalysed azide-alkyne cycloaddition reaction (CuAAC). Click conjugation may also refer to copper-free click chemistry, such as a reaction between an azide and a cyclic alkyne group such as dibenzocyclooctyl (DBCO). "1,2,3-triazolyl" may thus also refer to a group formed by a reaction between an azide and a cyclic alkyne group, such as DBCO, wherein the group comprises a 1,2,3-triazole moiety.

The linker group may, in principle, be any linker group that can be incorporated in the conjugate according to one or more embodiments of the invention. Linkers that may, in principle, be utilised are described e.g. in Dosio et al., Toxins 2011, 3, 848-883, and Sammet et al., Pharm. Pat. Analyst 2012, 1(1), 2046-8954.

L may comprise one or more linker groups or moieties. It may also comprise one or more groups formed by a reaction between two functional groups. A skilled person will realize that various different chemistries may be utilized when preparing the conjugate, and thus a variety of different functional groups may be reacted to form groups comprised by L. In an embodiment, the functional groups are selected from the group consisting of sulfhydryl, amino, alkenyl, alkynyl, azidyl, aldehyde, carboxyl, maleimidyl, succinimidyl and hydroxylamino. A skilled person is capable of selecting the functional groups so that they may react in certain conditions.

In this specification, the term "alkenyl" should be understood as referring to a C₂-C₁₈ hydrocarbon containing normal, secondary, tertiary or cyclic carbon atoms with at least one site of unsaturation, i.e., a carbon-carbon, sp² double bond. Examples include, but are not limited to ethylene or vinyl (CH=CH₂), allyl (CH₂CH=CH₂), cyclopentenyl (C₅H₇), and 5-hexenyl (CH₂CH₂CH₂CH₂CH=CH₂). The term "alkenyl" should also be understood as referring to an alkenylene, an unsaturated, branched or straight chain or cyclic hydrocarbon radical of 2-18 carbon atoms, and having two monovalent radical centers derived by the removal of two hydrogen atoms from the same or two different carbon atoms of a parent alkene. Typical alkenylene radicals include, but are not limited to 1,2-ethylene (CH=CH).

The term "alkynyl" should be understood as referring to a C₂-C₁₈ hydrocarbon containing normal, secondary, tertiary or cyclic carbon atoms with at least one site of unsaturation, i.e. a carbon-carbon, sp triple bond. Examples include, but are not limited to acetylenic (C≡CH) and propargyl (CH₂C≡CH). The term "alkynyl" should also be understood as referring to an alkynylene, an unsaturated, branched or straight chain or cyclic hydrocarbon radical of 2-18 carbon atoms, and having two monovalent radical centers derived by the removal of two hydrogen atoms from carbon atoms of a parent alkyne. Typical alkynylene radicals include (but are not limited to) acetylene (C≡C), propargyl (CH₂C≡C), and 4-pentynyl (CH₂CH₂CH₂C≡C). In an embodiment, alkynyl is dibenzylcyclooctyne (DBCO).

In an embodiment, alkynyl is CH≡C, CH≡CCH₂ or DBCO.

In this specification, "succinimidyl" may refer to N-hydroxysuccinimidyl (NHS) or N-hydroxysulfosuccinimidyl (sulfo-NHS).

In an embodiment, each L is independently absent or is a linker group covalently joining D and Y.

In an embodiment, L comprises a peptide from 2 to 5 amino acids in length. In an embodiment, L is or consists of a peptide from 2 to 5 amino acids in length.

In an embodiment, L comprises a linker group cleavable by a lysosomal hydrolase. Such linker groups are known in the art. Linker groups cleavable by a lysosomal hydrolase can be hydrolysed *in vitro* or *in vivo.* This may allow for the release the payload molecule in active form inside a cell.

In an embodiment, L is a linker group cleavable by a lysosomal hydrolase.

In an embodiment, the linker group cleavable by a lysosomal hydrolase is selected from the group consisting of a peptide cleavable by cathepsin and a cleavable carbohydrate unit.

In this specification, "peptide cleavable by cathepsin" refers to a peptide having an amino acid sequence that is recognized and cleaved by a protease, for example, a cathepsin B. A peptide cleavable by cathepsin may be recognized by more than one cathepsin, including related proteases.

In an embodiment, "peptide cleavable by cathepsin B" refers to a peptide having an amino acid sequence that comprises at least one cathepsin B cleavage site.

In an embodiment, the peptide comprises an amino acid sequence cleavable by a lysosomal peptidase. Such an amino acid sequence may be e.g. L-Gly-L-Gly, L-Val-L-Cit, L-Phe-L-Cit, L-Leu-L-Cit, L-Ile-L-Cit, L-Trp-L-Cit, L-Phe-L-Leu, L-Phe-L-Lys, L-Val-L-Lys, L-Ala-L-Lys, L-Leu-L-Ala-L-Leu, L-Leu-L-Ala-L-Ala, L-Ala-L-Leu-L-Ala-L-Leu, L-Arg-L-Leu-L-Val-L-Gly-L-Phe-L-Glu, L-Arg-L-Leu-L-Val-L-Gly-L-Trp-L-Glu, L-Arg-L-Leu-L-Val-L-Gly-L-Phe-L-Asp, L-Arg-L-Leu-L-Val-L-Gly-β-(2-naphthyl)-L-Ala-L-Glu, L-Arg-L-Leu-L-Val-L-Gly-L-Phe-L-α-aminoadipic acid, L-Arg-L-Leu-L-Arg-L-Gly-L-Phe-L-Glu, L-Leu-L-Arg-L-Gly-L-Phe-L-Glu, L-Arg-L-Ile-L-Ile-L-Glu-L-Gly-L-Ile-L-Glu, L-Arg-L-Ile-L-Glu-L-Gly-L-Ile-L-Glu, L-Ile-L-Glu-L-Gly-L-Ile-L-Glu, L-Arg-L-Leu-L-Glu-L-Gly-L-Ile-L-Glu, L-Leu-L-Glu-L-Gly-L-Ile-L-Glu, L-Leu-L-Arg-L-Gly-L-Ile-L-Glu, L-Gly-L-Phe-L-Gly-L-Ser-L-Val-L-Gln-L-Phe-L-Ala-L-Gly-L-Phe, L-Asp-L-Asp-L-Asp-L-Lys-L-Ile-L-Val, L-Gln-L-Arg-L-Val-L-Met-L-Phe-L-Thr, L-Glu-L-Val-L-Asp-L-Leu-L-Leu-L-Ile, L-Ser-L-Arg-L-Ser-L-Phe-L-Asn-L-Gln, L-Gln-L-Ala-L-Ser-L-Arg-L-Ser-L-Phe, L-Cys-L-Pro-L- Val-L-Thr-L-Tyr-L-Gly, or fragment thereof, and the like.

This specification also discloses variants of the linker peptides disclosed above, including chemical equivalents. Such equivalents include peptides that perform substantially the same function as the specific peptides disclosed herein in substantially the same way. For example, the peptide L-Cys-L-Pro-L-Val-L-Thr-L-Tyr-L-Gly comprises a cathepsin B cleavage site. Thus, a variant of L-Cys-L-Pro-L-Val-L-Thr-L-Tyr-L-Gly will also be recognized and cleaved by cathepsin B. For example, equivalents include, without limitation, conservative amino acid substitutions.

In an embodiment, the variant linker peptide has at least 50%, at least 60%, at least 70%, at least 80%, at least 90%, and 95% sequence identity to the peptide L-Gly-L-Gly, L-Val-L-Cit, L-Phe-L-Cit, L-Leu-L-Cit, L-Ile-L-Cit, L-Trp-L-Cit, L-Phe-L-Leu, L-Phe-L-Lys, L-Val-L-Lys, L-Ala-L-Lys, L-Leu-L-Ala-L-Leu, L-Leu-L-Ala-L-Ala, L-Ala-L-Leu-L-Ala-L-Leu, L-Arg-L-Leu-L-Val-L-Gly-L-Phe-L-Glu, L-Arg-L-Leu-L-Val-L-Gly-L-Trp-L-Glu, L-Arg-L-Leu-L-Val-L-Gly-L-Phe-L-Asp, L-Arg-L-Leu-L-Val-L-Gly-β-(2-naphthyl)-L-Ala-L-Glu, L-Arg-L-Leu-L-Val-L-Gly-L-Phe-L-α-aminoadipic acid, L-Arg-L-Leu-L-Arg-L-Gly-L-Phe-L-Glu, L-Leu-L-Arg-L-Gly-L-Phe-L-Glu, L-Arg-L-Ile-L-Ile-L-Glu-L-Gly-L-Ile-L-Glu, L-Arg-L-Ile-L-Glu-L-Gly-L-Ile-L-Glu, L-Ile-L-Glu-L-Gly-L-Ile-L-Glu, L-Arg-L-Leu-L-Glu-L-Gly-L-Ile-L-Glu, L-Leu-L-Glu-L-Gly-L-Ile-L-Glu, L-Leu-L-Arg-L-Gly-L-Ile-L-Glu, L-Gly-L-Phe-L-Gly-L-Ser-L-Val-L-Gln-L-Phe-L-Ala-L-Gly-L-Phe, L-Asp-L-Asp-L-Asp-L-Lys-L-Ile-L-Val, L-Gln-L-Arg-L-Val-L-Met-L-Phe-L-Thr, L-Glu-L-Val-L-Asp-L-Leu-L-Leu-L-Ile, L-Ser-L-Arg-L-Ser-L-Phe-L-Asn-L-Gln, L-Gln-L-Ala-L-Ser-L-Arg-L-Ser-L-Phe, or L-Cys-L-Pro-L-Val-L-Thr-L-Tyr-L-Gly.

In an embodiment, the lysosomal peptidase is cathepsin. In an embodiment, the cathepsin is cathepsin B. Peptides or amino acid sequences cleavable by cathepsin or cathepsin B are found and their amenability to cleavage by a cathepsin or capthepsin B can be tested, for example, using methods described in Stachowiak et al. (2004) Fluorogenic peptide substrates for carboxypeptidase acticity of cathepsin B, Acta Biochimica Polonica, 51:81-92; Dubowchik et al. (2002), Cathepsin B-labile dipeptide linkers for lysosomal release of doxorubicin from internalizing immunoconjugates: model studies of enzymatic drug release and antigen-specific in vitro anticancer activity, Bioconjug Chem, 13:855-869; Kim et al. (2013) New strategy for selective and sensitive assay of cathepsin B using a dityrosine-based material, Anal Biochem, 435:166-173; Lohmüller et al (2003) Toward Computer-Based Cleavage Site Prediction of Cysteine Endopeptidases, Biol. Chem., 384:899-909.

The term "a cleavable carbohydrate unit" as used in this specification refers to a carbohydrate unit cleavable by a lysosomal or endosomal glycohydrolase.

The linker group may be chosen based on the trafficking pathway utilized by the conjugate. In particular, the linker group such as a peptide may be cleavable by a protease such as cathepsin B that is present in an intracellular compartment, such as lysosome or endosome in the cell where the conjugate is processed.

In an embodiment, the linker group comprises a peptide having an amino acid sequence comprising two cathepsin cleavage sites. In certain embodiments, the linker group comprises a peptide having an amino acid sequence comprising at least two cathepsin cleavage sites. In an embodiment, the two cathepsin cleavage sites are recognized and cleaved by the same cathepsin. In other embodiments, the linker group comprises a peptide comprising three or more cathepsin cleavage sites.

In an embodiment, the peptide comprises a self-immolative group between the peptide and the payload molecule, such as p-aminobenzyloxycarbonyl (PABC) group. The term "self-immolative" refers to a functional chemical moiety that is capable of covalently linking together chemical moieties (i.e., D, a payload molecule to a peptide linker L) and that will spontaneously separate from e.g. payload molecule if its bond to the peptide linker is cleaved.

In an embodiment, the linker group comprises a group represented by the formula -(CH₂)ᵣ-, wherein r is in the range of 1 to 10. In an embodiment, r is 1, 2, 3, 4, 5, 6, 7, 8, 9 or 10. In an embodiment, r is in the range of 2 to 9, or in the range of 3 to 8, or in the range of 4 to 7, or in the range of 1 to 6, or in the range of 2 to 5, or in the range of 1 to 4.

In an embodiment, the linker group is hydrophilic.

In an embodiment, the linker group comprises at least one OH group.

In an embodiment, the linker group comprises at least one moiety derived from one or more saccharide units.

In an embodiment, L comprises a structure represented by the formula

Y'-(O-CH₂-CH₂)_{q}-Y'

wherein q is in the range of 1 to 20; and
each Y' is independently a group formed by a reaction between two functional groups.

In an embodiment, q is 1, 2, 3, 4, 5, 6, 7, 8, 9, 10, 11, 12, 13, 14, 15, 16, 17, 18, 19, or 20. In an embodiment, q is 2-16. In an embodiment, q is in the range of 2 to 10. In other embodiments, q is in the range of 2 to 6; 2 to 5; 2 to 4; 2 or 3; or 3 or 4.

In an embodiment, each Y' is independently a group formed by a reaction between two functional groups selected from sulfhydryl, amino, alkenyl, alkynyl, azidyl, aldehyde, carboxyl, maleimidyl, succinimidyl and hydroxylamino.

In an embodiment, L comprises a saccharide. The saccharide may be a monosaccharide, disaccharide or trisaccharide. The saccharide may also be an oligosaccharide comprising from 1 to about 20, or from 1 to 10, or from 1 to 8, or from 1 to 6, or from 1 to 5, or from 1 to 4, or from 1 to 3, or from 1 to 2, or 1, 2, 3, 4 or 5 saccharide units.

In an embodiment, the saccharide comprises a sialic acid unit. The sialic acid may be a terminal saccharide unit in the saccharide.

In an embodiment, the saccharide comprises a sialyllactose. The sialyllactose may be sialylα2,6lactose or sialylα2,3lactose.

The payload molecule is a toxic payload molecule or a boron compound.

The toxic payload molecule may, in principle, be any toxic molecule suitable for conjugation according to one or more embodiments of the invention. It may be any compound that results in the death of a cell, or induces cell death, or in some manner decreases cell viability. The toxic payload molecule can be any of many small molecule drugs, including, but not limited to, dolastatins; auristatins; epothilones; daunorubicins and doxorubicins; alkylating agents, such as thiotepa and cyclophosphamide (CYTOXAN^{™}); alkyl sulfonates such as busulfan, improsulfan and piposulfan; aziridines, such as benzodopa, carboquone, meturedopa, and uredopa; ethylenimines and methylamelamines including altretamine, triethylenemelamine, trietylene-phosphoramide, triethylenethiophosphaoramide and trimethylolomelamine; acetogenins (especially bullatacin and bullatacinone); camptothecins (including the synthetic analogue topotecan); bryostatin; callystatin; CC-1065 (including its adozelesin, carzelesin and bizelesin synthetic analogues); cryptophycins (particularly cryptophycin 1 and cryptophycin 8); duocarmycin (including the synthetic analogues, KW-2189 and CBI-TMI); eleutherobin; pancratistatin; sarcodictyins; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics, such as the enediyne antibiotics (e.g. calicheamicins, especially calicheamicin γ1; dynemicin, including dynemicin A; esperamicin; as well as neocarzinostatin chromophore and related chromoprotein enediyne antiobiotic chromomophores), aclacinomysins, actinomycin, authramycin, azaserine, bleomycins, cactinomycin, carabicin, caminomycin, carzinophilin; chromomycins, dactinomycin, detorubicin, 6-diazo-5-oxo-L-norleucine, other doxorubicin derivatives including morpholino-doxorubicin, cyanomorpholino-doxorubicin, 2-pyrrolino-doxorubicin and deoxydoxorubicin, epirubicin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites, such as methotrexate and 5-fluorouracil (5-FU); folic acid analogues, such as denopterin, methotrexate, pteropterin, trimetrexate; purine analogs, such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-fluorouracil; androgens, such as calusterone, dromostanolone propionate, epitiostanol, mepitiostane, testolactone; anti-adrenals, such as aminoglutethimide, mitotane, trilostane; folic acid replenisher, such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; maytansinoids, such as maytansine, ansamitocins, DM-1, DM-4; mitoguazone; mitoxantrone; mopidamol; nitracrine; pentostatin; phenamet; pirarubicin; podophyllinic acid; 2-ethylhydrazide; procarbazine; PSK^{®}; razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; capecitabine; anti-hormonal agents that act to regulate or inhibit hormone action on tumours, such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens, such as flutamide, nilutamide, bicalutamide, leuprolide, and goserelin; siRNA; and pharmaceutically acceptable salts, acids or derivatives of any of the above as well as analogues and derivatives thereof, some of which are described below.

In an embodiment, the toxic payload molecule is a dolastatin, auristatin, doxorubicin, maytansinoid, epirubicin, duocarmycin, or any analogue or derivative thereof.

In an embodiment, the toxic payload molecule is dolastatin 10, dolastatin 15 or any derivative thereof.

In an embodiment, the toxic payload molecule is auristatin F or any derivative thereof.

In one embodiment, the toxic payload molecule is dolastatin 10, dolastatin 15, or auristatin F.

In one embodiment, the toxic payload molecule is dolastatin 10.

In one embodiment, the toxic payload molecule is dolastatin 15.

In one embodiment, the toxic payload molecule is auristatin F.

Dolastatins that can be used in the present invention are well known in the art and can be isolated from natural sources according to known methods or prepared synthetically according to known methods.

Examples of suitable dolastatins include monomethyl and desmethyl dolastatins 10, 15, C, D and H, monomethyl and desmethyl isodolastatin H, and analogues and derivatives thereof. These dolastatins contain a primary or secondary amine at the N-terminus. Dolastatins 10 and 15 are the most potent toxic payload molecules among the naturally occurring dolastatins. Monomethyl and desmethyl dolastatins 10 and 15 can be prepared by chemical synthesis according to standard peptide synthesis chemistry.

Auristatins that can be used in the present invention include (but are not limited to) monomethyl and desmethyl auristatins E, F, EB, EFP, PY, PYE, PE, PHE, TP, 2-AQ and 6-AQ, e.g. described in U.S. Pat. No. 5,635,483; Int. J. Oncol. 15:367-72 (1999); Mol. Cancer Ther. 3:921-32 (2004); U.S. application Ser. No. 11/134,826; U.S. Patent Publication Nos. 20060074008 and 2006022925; and Pettit, G.R., et al. (2011) J. Nat. Prod. 74:962-8.

In an embodiment, monomethyl and desmethyl auristatin and dolastatin 10 derivatives are represented by the formula: 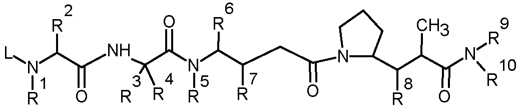 wherein L is either H, or may be understood as referring to L or Y of formula I; R¹, R⁵ and R⁹ are each independently either H or C₁-C₈ alkyl; R², R³ and R⁶ are each independently either H, C₁-C₈ alkyl, C₃-C₈ carbocycle, aryl, C₁-C₈ alkyl-aryl, C₁-C₈ alkyl-(C₃-C₈ carbocycle), C₃-C₈ heterocycle or C₁-C₈ alkyl-(C₃-C₈ heterocycle); R⁴ is either H or CH₃; or R³ and R⁴ jointly form a carbocyclic ring with the carbon to which they are attached and have the formula -(CRₐR_{b})ᵣ-, wherein Rₐ and R_{b} are independently selected from H, C₁-C₈ alkyl and C₃-C₈ carbocycle; and r is selected from the group consisting of 2, 3, 4, 5 and 6; R⁷ and R⁸ are each independently selected from H, OH, C₁-C₈ alkyl, C₃-C₈ carbocycle and O(C₁-C₈ alkyl); R¹⁰ is either CX₂-CX₂-aryl, CX₂-CX₂-(substituted aryl), CX₂-CX₂-(C₃-C₈ heterocycle), CX₂-(C₃-C₁₀ heterocycle), CX₂-CX₂-(C₃-C₈ carbocycle), C(=O)O(C₁-C₄ alkyl) or CH(CH₂R¹²)C(=O)ZR¹¹; each occurrence of X is independently either H, OH, C₁-C₈ alkyl, C₃-C₈ carbocycle, C₃-C₈ heterocycle, 2-thiazole or O(C₁-C₈ alkyl); Z is either O, S, NH or N(C₁-C₈ alkyl) ; R¹¹ is either H, C₁-C₂₀ alkyl, aryl, C₃-C₈ heterocycle, (R¹³O)ₘ-R¹⁴ or (R¹³O)ₘ-CH(R¹⁵)₂; R¹² is either aryl or C₃-C₈ heterocycle; m is an integer ranging from 1-1000; R¹³ is C₂-C₈ alkyl; R¹⁴ is H or C₁-C₈ alkyl; each occurrence of R¹⁵ is independently H, COOH, (CH₂)ₙ-N(R¹⁶)₂, (CH₂)ₙ-SO₃H or (CH₂)ₙ-SO₃-C₁-C₈ alkyl; each occurrence of R¹⁶ is independently H, C₁-C₈ alkyl or (CH₂)ₙ-COOH; and n is an integer in the range from 0 to 6.

In an embodiment, monomethyl and desmethyl auristatins and dolastatin 10 derivatives are represented by the formula: 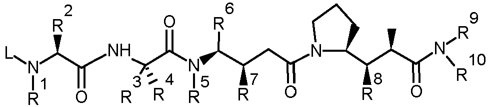 wherein the substituents are as described above.

In an embodiment, monomethyl and desmethyl auristatins and dolastatin 10 derivatives are represented by the formula: 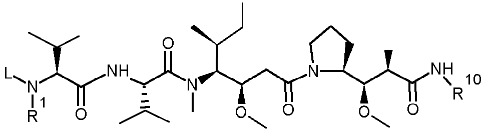 wherein the substituents are as described above.

In an embodiment, monomethyl and desmethyl auristatin F derivatives are represented by the formula: 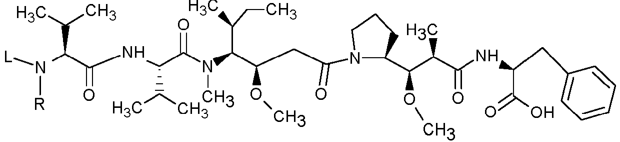 wherein L is either H, or may be understood as referring to L or Y of formula I; and R is either H or CH₃.

In an embodiment, monomethyl and desmethyl dolastatin 10 derivatives are represented by the formula: 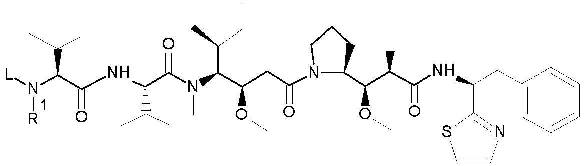 wherein L is either H, or may be understood as referring to L or Y of formula I; and R¹ is either H or CH₃.

In one embodiment, monomethyl and desmethyl dolastatin 15 analogues and derivatives are represented by the formula: 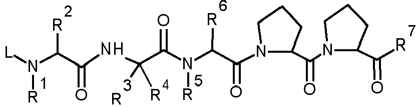 wherein L, R¹, R², R³, R⁴, R⁵ and R⁶ are as described above; R⁷ is either OH, NH₂, NHR⁸ or NR⁸R⁹; R⁸ and R⁹ are each independently either H, C₁-C₈ alkyl, C₃-C₈ carbocycle, aryl, C₁-C₈ alkyl-aryl, C₁-C₈ alkyl-(C₃-C₈ carbocycle), C₃-C₈ heterocycle, C₁-C₈ alkyl-(C₃-C₈ heterocycle), benzyl or tert-butyl; or R⁸ and R⁹ jointly form a heterocyclic ring with the nitrogen to which they are attached and have the formula -(CRₐR_{b})ₙ-, wherein Rₐ and R_{b} are independently selected from H, C₁-C₈ alkyl, C₃-C₈ carbocycle, aryl, C₁-C₈ alkyl-aryl, C₁-C₈ alkyl-(C₃-C₈ carbocycle), C₃-C₈ heterocycle, C₁-C₈ alkyl- (C₃-C₈ heterocycle), O(C₁-C₈ alkyl), a double bond with neighboring carbon atom, or they jointly form a carbonyl group; and n is selected from 2, 3, 4, 5 and 6.

In an embodiment, monomethyl and desmethyl dolastatin 15 analogues and derivatives are represented by the formula: 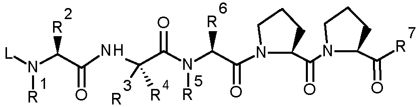 wherein the substituents are as described above.

In an embodiment, the monomethyl or desmethyl dolastatin 15 analogue or derivative is selected from the group consisting of monomethyl and desmethyl dolastatin 15, monomethyl and desmethyl cemadotin, monomethyl and desmethyl tasidotin, and monomethyl and desmethyl P5 (the corresponding dimethyl compounds are described in Bai et al. 2009. Mol. Pharmacol. 75:218-26).

In an embodiment, monomethyl and desmethyl dolastatin 15 analogues and derivatives are represented by the formula: 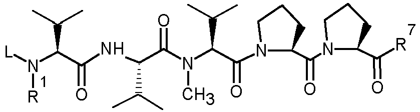 wherein the substituents are as described above.

In an embodiment, monomethyl and desmethyl dolastatin 15 derivatives are represented by the formula: 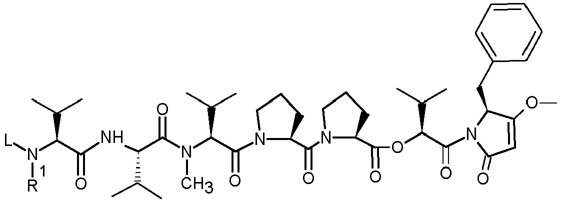 wherein L is either H, or may be understood as referring to L or Y of formula I; and R¹ is either H or CH₃.

The toxic payload molecule according to the present invention may also be daunorubicin or doxorubicin. The primary amine group of the daunosamine moiety can be used, or daunorubicin or doxorubicin of the present invention can be modified to comprise another primary or secondary amine moiety. Preferred doxorubicin and daunorubicin payload molecules useful in the present invention are according to the formula: 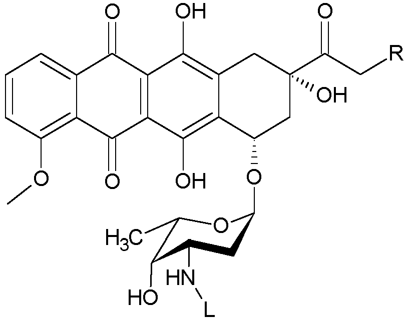 wherein R is either H or OH; and L is either H, or may be understood as referring to L or Y of formula I.

In an embodiment, the toxic payload molecule is a maytansinoid.

In an embodiment, the toxic payload molecule is maytansine, an ansamitocin, DM1 or DM4 (also known as DM-4).

In an embodiment, the toxic payload molecule is DM1. DM1 is also known as DM-1 and mertansine.

In an embodiment, the toxic payload molecule is a rubicin. Suitable rubicins may be e.g. daunorubicins, doxorubicins, detorubicin, other doxorubicin derivatives including morpholino-doxorubicin, cyanomorpholino-doxorubicin, 2-pyrrolino-doxorubicin, deoxydoxorubicin, epirubicin, esorubicin, idarubicin, rodorubicin, zorubicin, and pirarubicin.

In an embodiment, the toxic payload molecule is epirubicin.

In an embodiment, the toxic payload molecule is duocarmycin. Suitable duocarmyxins may be e.g. duocarmycin A, duocarmycin B1, duocarmycin B2, duocarmycin C1, duocarmycin C2, duocarmycin D, duocarmycin SA, duocarmycin MA, and CC-1065. The term "duocarmycin" should be understood as referring also to synthetic analogs of duocarmycins, such as adozelesin, bizelesin, carzelesin, KW-2189 and CBI-TMI.

In an embodiment, the duocarmycin is a duocarmycin suitable for conjugating to L or to Y. In an embodiment, the duocarmycin comprises an amino group or another suitable chemical group for conjugating the duocarmycin to L or to Y. In an embodiment, the amino group is a free amino group.

One skilled in the art of toxic payload molecules will readily understand that each of the toxic payload molecules described herein can be modified in such a manner that the resulting compound still retains the specificity and/or activity of the starting compound. The skilled person will also understand that many of these compounds can be used in place of the toxic payload molecules described herein. Thus, the toxic payload molecules of the present invention should be understood as including any analogues and derivatives of the compounds described herein.

In an embodiment, the payload molecule is a toxic payload molecule selected from the group consisting of dolastatin, auristatin, doxorubicin, maytansinoid, epirubicin, duocarmycin, and any analogue or derivative thereof.

In this specification, the term "boron compound" may refer to any compound containing boron atoms. Suitable boron compounds may be e.g. BSH, carboranes, decaborane or any derivatives thereof. A single molecule of a boron compound typically contains at least one nonradioactive boron-10 atom.

In an embodiment, the boron compound is BSH. "BSH", "B₁₂H₁₁-SH" and "Na₂B₁₂H₁₁SH" should be understood as referring to sodium borocaptate, also known as sodium mercaptododecaborate and sulfhydryl boron hydride. "B₁₂H₁₁²⁻" thus refers to the boron hydride moiety of BSH.

In an embodiment, the boron compound is a carborane. In an embodiment, the boron compound is a derivative of decaborane.

In this specification, the term "fluorescent label molecule" may refer to any fluorophore which can re-emit light upon light excitation. Such a fluorescent label molecule may be suitable for detection, visualization and/or quantitation. Suitable fluorescent label molecules may be e.g. FITC, TRITC, and the Alexa and Cy dyes.

In this specification, the term "radioactive molecule" may refer to any molecule that comprises a radioactive atom. The term "radioactive molecule" may refer to a radioactive molecule suitable for radiation therapy. It may also refer to a radioactive molecule suitable for detection, visualization and/or quantitation.

The payload molecule may be selected so that it comprises a functional group that allows for conjugating it to L or to Y. The payload molecule may also be modified prior to reacting it with other components of the conjugate. In an embodiment, the payload molecule comprises a primary or secondary amine moiety. In an embodiment, a toxic payload molecule is modified to comprise a primary or secondary amine moiety. In an embodiment, the amine-modified payload molecule essentially retains the activity of the original payload molecule after conjugation.

In an embodiment, the polymer has a molecular mass in the range of about 3 to about 2000 kDa, or about 5 to about 200 kDa, or about 10 to about 100 kDa, or about 30 to about 300 kDa. In this context, the molecular mass of the polymer should be understood as referring to the polymer not containing any substituents of the formula [D-L-Y-(CH₂)ₙ-O]- or the protein.

In an embodiment, the conjugate is represented by the formula

[D-L-Y'-(CH₂)ₒ-S-(CH₂)ₙ-O]ₘ-P-T

wherein
T is an antibody;
P is a polymer selected from the group consisting of dextran, hyaluronic acid, hydroxyethyl starch, and cyclodextrin;
m is at least 1;
n is in the range of 2 to 10;
each L is independently absent or a linker group covalently joining D and Y;
each D is a payload molecule;
o is in the range of 1 to 10; and
each Y' is independently selected from the group consisting of NH and 1,2,3-triazolyl, wherein 1,2,3-triazolyl is optionally substituted,
wherein saccharide units of the polymer have been cleaved by oxidative cleavage of a bond between two adjacent carbons substituted by a hydroxyl group.

In an embodiment, o is 3, 4, 5, 6, 7, 8, 9 or 10. In an embodiment, o is in the range of 3 to 4, or in the range of 3 to 5, or in the range of 3 to 6, or in the range of 3 to 7, or in the range of 3 to 8, or in the range of 3 to 9. Each o may, in principle, be independently selected. Each o in a single conjugate may also be the same.

In an embodiment, Y' is selected from the group consisting of NH and 1,2,3-triazolyl.

In an embodiment, P is a polymer derivative comprising at least one saccharide unit, and the polymer derivative is bound to the protein via a bond formed by a reaction between at least one aldehyde group formed by oxidative cleavage of a saccharide unit of the polymer derivative and an amino group of the protein.

In an embodiment, the saccharide unit is a D-glucosyl, D-mannosyl, D-galactosyl, L-fucosyl, D-N-acetylglucosaminyl, D-N-acetylgalactosaminyl, D-glucuronidyl, or D-galacturonidyl unit, or a sulphated derivative thereof.

In an embodiment, the D-glucosyl is D-glucopyranosyl.

The polymer is selected from the group consisting of dextran, hyaluronic acid, hydroxyethyl starch, and cyclodextrin. These polymers have the added utility that they may be oxidatively cleaved so that aldehyde groups are formed.

In an embodiment, the polymer is dextran.

In this specification, "dextran" should be understood as referring to a branched glucan composed of chains of varying lengths, wherein the straight chain consists of a α-1,6 glycosidic linkages between D-glucosyl (D-glucopyranosyl) units. Branches are bound via α-1,3 glycosidic linkages and, to a lesser extent, via α-1,2 and/or α-1,4 glycosidic linkages. A portion of a straight chain of a dextran molecule is depicted in the schematic representation below.

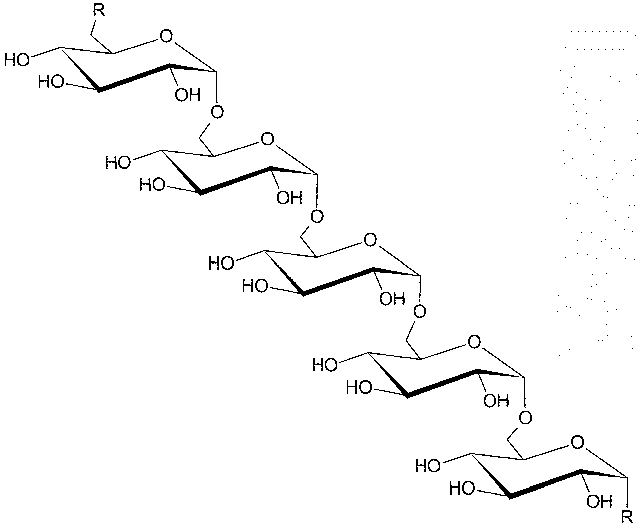

"D-glucosyl unit" should be understood as referring to a single D-glucosyl molecule. Dextran thus comprises a plurality of D-glucosyl units. In dextran, each D-glucosyl unit is bound to at least one other D-glucosyl unit via a α-1,6 glycosidic linkage, via a α-1,3 glycosidic linkage or via both.

Each D-glucosyl unit of dextran comprises 6 carbon atoms, which are numbered 1 to 6 in the schematic representation below. The schematic representation shows a single D-glucosyl unit bound to two other D-glucosyl units (not shown) via α-1,6 glycosidic linkages.

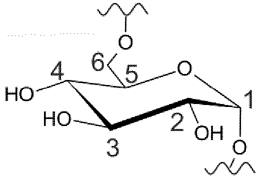

Carbons 2, 3 and 4 may be substituted by free hydroxyl groups. In D-glucosyl units bound to a second D-glucosyl unit via a α-1,3 glycosidic linkage, wherein carbon 3 of the D-glucosyl unit is bound via an ether bond to carbon 1 of the second D-glucosyl unit, carbons 2 and 4 may be substituted by free hydroxyl groups. In D- glucosyl units bound to a second D-glucosyl unit via a α-1,2 or α-1,4 glycosidic linkage, wherein carbon 2 or 4 of the D-glucosyl unit is bound via an ether bond to carbon 1 of the second D-glucosyl unit, carbons 3 and 4 or 2 and 3, respectively, may be substituted by free hydroxyl groups.

A skilled person will understand that other polymers described in this specification also contain free hydroxyl groups bound to one or more carbon atoms and have also other similar chemical properties.

Carbohydrate nomenclature is essentially according to recommendations by the IUPAC-IUB Commission on Biochemical Nomenclature (e.g. Carbohydrate Res. 1998, 312, 167; Carbohydrate Res. 1997, 297, 1; Eur. J. Biochem. 1998, 257, 293).

In this specification, the term "Ficoll" refers to an uncharged, highly branched polymer formed by the copolymerisation of sucrose and epichlorohydrin.

In an embodiment, the polymer is a dextran derivative comprising at least one D-glucosyl unit;
n is in the range of 3 to 10;
Y is S;
L is absent;
D is B₁₂H₁₁²⁻;
the dextran derivative comprises at least one aldehyde group formed by oxidative cleavage of a D-glucosyl unit; and
the dextran derivative is bound to the protein via a bond formed by a reaction between at least one aldehyde group of the dextran and an amino group of the protein.

In an embodiment, the term "dextran derivative" may be understood as referring to dextran, wherein at least one carbon selected from carbon 2, 3 or 4 of the at least one D-glucosyl unit is substituted by a substituent of the formula

-O-(CH₂)ₙ-S-B₁₂H₁₁²⁻

wherein n is in the range of 3 to 10; and
the dextran derivative is bound to the protein via a bond formed by a reaction between at least one aldehyde group formed by oxidative cleavage of a D-glucosyl unit of the dextran derivative and an amino group of the protein. The dextran derivative may furher contain other modifications to the basic dextran structure, e.g. as described below.

One or more, e.g. one, two or three carbons of a saccharide unit may be substituted by a substituent of the formula -O-(CH₂)ₙ-S-B₁₂H₁₁²⁻.

Saccharide units of the polymer, for instance the D-glucosyl units of dextran, may be cleaved by oxidative cleavage of a bond between two adjacent carbons substituted by a hydroxyl group. The oxidative cleavage cleaves vicinal diols, such as D-glucosyl and other saccharide units in which two (free) hydroxyl groups occupy vicinal positions. Saccharide units in which carbons 2, 3 and 4 are substituted by free hydroxyl groups may thus be oxidatively cleaved between carbons 2 and 3 or carbons 3 and 4. Thus a bond selected from the bond between carbons 2 and 3 and the bond between carbons 3 and 4 may be oxidatively cleaved. D-glucosyl units and other saccharide units of dextran and other polymers may be cleaved by oxidative cleavage using an oxidizing agent such as sodium periodate, periodic acid and lead(IV) acetate, or any other oxidizing agent capable of oxidatively cleaving vicinal diols.

Oxidative cleavage of a saccharide unit forms two aldehyde groups, one aldehyde group at each end of the chain formed by the oxidative cleavage. In the conjugate, the aldehyde groups may in principle be free aldehyde groups. However, the presence of free aldehyde groups in the conjugate is typically undesirable. Therefore the free aldehyde groups may be capped or reacted with an amino group of the protein, or e.g. with a tracking molecule.

In an embodiment, the polymer derivative is bound to the protein via a bond formed by a reaction between at least one aldehyde group formed by oxidative cleavage of a saccharide unit of the polymer derivative and an amino group of the protein.

In an embodiment, the polymer derivative may also be bound to the protein via a group formed by a reaction between at least one aldehyde group formed by oxidative cleavage of a saccharide unit of the polymer derivative and an amino group of the protein.

The aldehyde group formed by oxidative cleavage readily reacts with an amino group in solution, such as an aqueous solution. The resulting group or bond formed may, however, vary and is not always easily predicted and/or characterised. The reaction between at least one aldehyde group formed by oxidative cleavage of a saccharide unit of the polymer derivative and an amino group of the protein may result **e.g. in** the formation of a Schiff base. Thus the group via which the polymer derivative is bound to the protein may be **e.g.** a Schiff base (imine) or a reduced Schiff base (secondary amine).

In an embodiment, the conjugate is internalised by a cell expressing the target molecule after the conjugate (or the protein part of the conjugate, exemplary protein part may be an antibody) is bound to the target molecule. In other words, after binding to its target molecule on the target cell, for example, in a tumor cell, the conjugate may be internalized by the target cell as a result of the binding. The effect of this is that the conjugate may be taken up by the target cell.

The protein is an antibody. The antibody may be selected based on the selective binding it confers in order to allow for delivering the payload molecule to specific target cells. In this specification, the term "target molecule" should be understood as referring to any target molecule as defined in this specification.

In an embodiment, the protein is capable of binding a target molecule.

In an embodiment, the protein is an antibody capable of binding to a target molecule selected from the group consisting of CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, epidermal growth factor receptor 1 (EGFR1), epidermal growth factor receptor 2 (HER2/neu), HER3, HER4 receptor, LFA-1, Mac1, p150.95, VLA-4, ICAM-1, VCAM, EpCAM, alpha₄/beta₇ integrin, alpha v/beta3 integrin including either alpha or beta subunits thereof, tissue factor (TF), tumor necrosis factor alpha (TNF-α), epidermal growth factor (EGF), human vascular endothelial growth factor (VEGF), glycoprotein IIb/IIIa, TGF-beta, alpha interferon (alpha-IFN), IL-8, IL-2 receptor, IgE, HIV-1 envelope glycoprotein gp120, cancer-associated high-mannose type N-glycans, blood group antigen Apo2, death receptor, flk2/flt3 receptor, obesity (OB) receptor, mpl receptor, CTLA-4, transferrin receptor, Lewis y, GD3, and a target molecule binding fragment thereof.

The term "antibody capable of binding to a target molecule" should be understood as referring to an antibody that specifically binds the target molecule. The term "specifically binding" refers to the ability of the antibody to discriminate between the target molecule and any other protein to the extent that, from a pool of a plurality of different proteins as potential binding partners, only the target molecule is bound or significantly bound. As examples only, specific binding and/or kinetic measurements may be assayed by e.g. by utilizing surface plasmon resonance-based methods on a Biacore apparatus, by immunological methods such as ELISA or by e.g. protein microarrays.

The term "a target molecule binding fragment thereof" should be understood as referring to any fragment of an antibody that is capable of specifically binding the target molecule.

The antibody may be e.g. an scFv, a single domain antibody, an Fv, a VHH antibody, a diabody, a tandem diabody, a Fab, a Fab', a F(ab')₂, a Db, a dAb-Fc, a taFv, a scDb, a dAb₂, a DVD-Ig, a Bs(scFv)₄-IgG, a taFv-Fc, a scFv-Fc-scFv, a Db-Fc, a scDb-Fc, a scDb-C_{H}3, or a dAb-Fc-dAb. Furthermore, the antibody or a target molecule binding fragment thereof may be present in monovalent monospecific, multivalent monospecific, bivalent monospecific, or multivalent multispecific forms.

In an embodiment, the antibody is a human antibody or a humanized antibody. In this context, the term "human antibody", as it is commonly used in the art, is to be understood as meaning antibodies having variable regions in which both the framework and complementary determining regions (CDRs) are derived from sequences of human origin. In this context, the term "humanized antibody", as it is commonly used in the art, is to be understood as meaning antibodies wherein residues from a CDR of an antibody of human origin are replaced by residues from a CDR of a nonhuman species (such as mouse, rat or rabbit) having the desired specificity, affinity and capacity.

Target molecules or cancer target molecules (antigens) for the protein and the conjugate may include CD proteins, such as CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, and CD138; members of the ErbB receptor family, such as epidermal growth factor receptor 1 (EGFR), epidermal growth factor receptor 2 (HER2/neu), HER3 or HER4 receptor; cell adhesion molecules, such as LFA-1, Mac1, p150.95, VLA-4, ICAM-1, VCAM, EpCAM, alpha₄/beta₇ integrin, and alpha v/beta3 integrin including either alpha or beta subunits thereof; growth factors, such as human vascular endothelial growth factor (VEGF); tissue factor (TF); tumor necrosis factor alpha (TNF-α);; glycoprotein IIb/IIIa; TGF-beta; alpha interferon (alpha-IFN); an interleukin, such as IL-8; an interleukin receptor, such as IL-2 receptor; IgE; respiratory syncytial virus (RSV); HIV-1 envelope glycoprotein gp120, cancer-associated high-mannose type N-glycans; blood group antigens Apo2, death receptor; flk2/flt3 receptor; obesity (OB) receptor; mpl receptor; CTLA-4; transferrin receptor; cancer-associated glycan structure, such as Lewis y or GD3; or protein C.

In an embodiment, the target molecule is selected from the group consisting of CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, epidermal growth factor receptor 1 (EGFR), epidermal growth factor receptor 2 (HER2/neu), HER3 or HER4 receptor, LFA-1, Mac1, p150.95, VLA-4, ICAM-1, VCAM, EpCAM, alpha₄/beta₇ integrin, alpha v/beta3 integrin including either alpha or beta subunits thereof (e.g. anti-CD11a, anti-CD18 or anti-CD11b antibodies), tissue factor (TF), tumor necrosis factor alpha (TNF-α), human vascular endothelial growth factor (VEGF), glycoprotein IIb/IIIa, TGF-beta, alpha interferon (alpha-IFN), IL-8, IL-2 receptor, IgE, respiratory syncytial virus (RSV), HIV-1 envelope glycoprotein gp120, cancer-associated high-mannose type N-glycans, blood group antigen Apo2, death receptor, flk2/flt3 receptor, obesity (OB) receptor, mpl receptor, CTLA-4, transferrin receptor, Lewis y, GD3 and protein C.

Antibodies that may be used are antibodies to CD2, CD3, CD4, CD5, CD6, CD11, CD19, CD20, CD22, CD26, CD30, CD33, CD37, CD38, CD40, CD44, CD52, CD56, CD79, CD105, CD138, EphA receptors (e.g., EphA2 receptor), EphB receptors, EGFR, EGFRvIII, HER2, HER3, trastuzumab, pertuzumab mesothelin, cripto, alpha beta₆ integrins, VEGF, VEGFR, folate receptor (for example, FOLR1), transferrin receptor, Lewis y, GD3, or EpCAM.

In an embodiment, the protein is the antibody bevacizumab, tositumomab, etanercept, trastuzumab, adalimumab, alemtuzumab, gemtuzumab ozogamicin, efalizumab, rituximab, infliximab, abciximab, basiliximab, palivizumab, omalizumab, daclizumab, cetuximab, panitumumab, epratuzumab, 2G12, lintuzumab, nimotuzumab, ibritumomab tiuxetan, abagovomab, actoxumab, adecatumumab, afutuzumab, altumomab, amatuximab, anifrolumab, apolizumab, atinumab, atlizumab, atorolimumab, bapineuzumab, basiliximab, bavi-tuximab, belimumab, benralizumab, bertilimumab, be-silesomab, bezlotoxumab, bimagrumab, bivatuzumab, blinatumomab, blosozumab, brentuximab, briakinumab, brodalumab, canakinumab, cantuzumab, caplacizumab, capromab, carlumab, catumaxomab, CC49, cedelizumab, cixutumumab, clazakizumab, clenoliximab, clivatuzumab, conatumumab, concizumab, crenezumab, CR6261, dacetuzumab, dalotuzumab, daratumumab, demcizumab, denosumab, detumomab, drozitumab, duligotumab, dupilumab, dusigitumab, ecromeximab, eculizumab, edobacomab, edrecolomab, eldelumab, elotuzumab, elsilimomab, enavatuzumab, enlimomab, enokizumab, enoticumab, ensituximab, epitumomab, epratuzumab, ertumaxomab, etaracizumab, etrolizumab, evolocumab, exbivirumab, fanolesomab, faralimomab, farletuzumab, fasinumab, felvizumab, fezakinumab, ficlatuzumab, figitumumab, flanvotumab, fontolizumab, foralumab, foravirumab, fresolimumab, fulranumab, futuximab, galiximab, ganitumab, gantenerumab, gavilimomab, gevokizumab, girentuximab, glembatumumab, golimumab, gomiliximab, guselkumab, ibalizumab, icrucumab, imciromab, imgatuzumab, inclacumab, indatuximab, intetumumab, inolimomab, inotuzumab, ipilimumab, iratumumab, itolizumab, ixekizumab, keliximab, labetuzumab, lambrolizumab, lampalizumab, lebrikizumab, lemalesomab, lerdelimumab, lexatumumab, libivirumab, ligelizumab, lintuzumab, lirilumab, lodelcizumab, lorvotuzumab, lucatumumab, lumiliximab, mapatumumab, margetuximab, maslimomab, mavrilimumab, matuzumab, mepolizumab, metelimumab, milatuzumab, minretumomab, mitumomab, mogamulizumab, morolimumab, motavizumab, moxetumomab, muromonab, namilumab, narnatumab, natalizumab, nebacumab, necitumumab, nerelimomab, nesvacumab, nimotuzumab, nivolumab, obinutuzumab, ocaratuzumab, ocrelizumab, odulimomab, ofatumumab, olaratumab, olokizumab, onartuzumab, oregovomab, orticumab, otelixizumab, oxelumab, ozanezumab, ozoralizumab, pagibaximab, panobacumab, parsatuzumab, pascolizumab, pateclizumab, patritumab, pemtumomab, perakizumab, pertuzumab, pi-dilizumab, pinatuzumab, pintumomab, placulumab, po-latuzumab, ponezumab, priliximab, pritoxaximab, pritumumab, quilizumab, racotumomab, radretumab, rafivirumab, ramucirumab, raxibacumab, regavirumab, reslizumab, rilotumumab, robatumumab, roledumab, romosozumab, rontalizumab, rovelizumab, ruplizumab, samalizumab, sarilumab, satumomab, secukinumab, seribantumab, setoxaximab, sevirumab, sibrotuzumab, sifalimumab, siltuximab, simtuzumab, siplizumab, sirukumab, solanezumab, solitomab, sonepcizumab, sontuzumab, stamulumab, suvizumab, tabalumab, taca-tuzumab, talizumab, tanezumab, taplitumomab, tefiba-zumab, tenatumomab, teneliximab, teplizumab, tepro-tumumab, TGN1412, ticilimumab, tildrakizumab, tiga-tuzumab, tocilizumab, toralizumab, tovetumab, tralokinumab, TRBS07, tregalizumab, tremelimumab, tucotuzumab, tuvirumab, ublituximab, urelumab, urtoxazumab, ustekinumab, vantictumab, vapaliximab, vatelizumab, vedolizumab, veltuzumab, vepalimomab, vesencumab, visilizumab, volociximab, vorsetuzumab, votumumab, zalutumumab, zanolimumab, zatuximab, ziralimumab, 2G12 (anti-HIV-1 envelope glycoprotein gp120), or zolimomab. However, these antibodies are provided as examples only, to which the invention is not limited; a skilled person will appreciate that the antibody of the invention is not limited to any particular antibody or form thereof.

In an embodiment, the protein is an anti-EGFR1 antibody or an EGFR1 binding fragment thereof.

"EGFR1" should in this specification be understood as referring to human epidermal growth factor receptor 1 (EGFR1) having a sequence set forth in SEQ ID NO: 1.

"Anti-EGFR1 antibody" should be understood as referring to an antibody that specifically binds EGFR1. The term "specifically binding" refers to the ability of the antibody to discriminate between EGFR1 and any other protein to the extent that, from a pool of a plurality of different proteins as potential binding partners, only EGFR1 is bound or significantly bound. As examples only, specific binding and/or kinetic measurements may be assayed by e.g. by utilizing surface plasmon resonance-based methods on a Biacore apparatus, by immunological methods such as ELISA or by e.g. protein microarrays.

"An EGFR1 binding fragment thereof" should be understood as referring to any fragment of an anti-EGFR1 antibody that is capable of specifically binding EGFR1.

In an embodiment, anti-EGFR1 antibody is cetuximab, imgatuzumab, matuzumab, nimotuzumab, necitumumab, panitumumab, or zalutumumab.

In an embodiment, the anti-EGFR1 antibody is cetuximab.

In an embodiment, cetuximab has a sequence set forth in SEQ ID NO:s 2 and 3.

In an embodiment, cetuximab comprises or consists of the sequences set forth in SEQ ID NO:s 2 and 3.

In an embodiment, the anti-EGFR1 antibody is nimotuzumab.

In an embodiment, nimotuzumab has a sequence set forth in SEQ ID NO:s 4 and 5.

In an embodiment, nimotuzumab comprises or consists of the sequences set forth in SEQ ID NO:s 4 and 5.

In an embodiment, the anti-EGFR1 antibody fragment comprises a Fab fragment of cetuximab. In an embodiment, the anti-EGFR1 Fab fragment has a sequence set forth in SEQ ID NO:s 6 and 3. In an embodiment, the anti-EGFR1 Fab fragment comprises or consists of a sequence set forth in SEQ ID NO:s 6 and 3.

In an embodiment, the anti-EGFR1 antibody comprises a F(ab')2 fragment of cetuximab. In an embodiment, the anti-EGFR1 F(ab')2 fragment has a sequence set forth in SEQ ID NO:s 7 and 3. In an embodiment, the anti-EGFR1 F(ab')2 fragment comprises or consists of a sequence set forth in SEQ ID NO:s 7 and 3.

In an embodiment, the anti-IgE antibody is omalizumab.

In an embodiment, the antibody fragment comprises Fab fragment of the antibody. In an embodiment, the Fab fragment is an anti-EGFR1 Fab fragment.

In an embodiment, the antibody fragment comprises the F(ab')2 fragment. In an embodiment, the F(ab')2 fragment is an anti-EGFR1 F(ab')2 fragment.

The protein typically contains at least one amino group, such as an N-terminal amine group and/or the amino group of a lysine residue.

In an embodiment, the amino group of the protein is the amino group of a lysine residue of the protein.

In an embodiment, conjugate comprises at least one tracking molecule bound to the polymer or to the protein. The tracking molecule may be bound to the polymer (or polymer derivative) or to the protein directly, via a bond, or indirectly, via **e.g.** a suitable linker group.

"Tracking molecule" refers in this specification to a detectable molecule. Such a detectable molecule may be e.g. a radioisotope, such as ¹⁴C, a compound comprising a radioisotope, a radionuclide, a compound comprising a radionuclide, a fluorescent label molecule (such as FITC, TRITC, the Alexa and Cy dyes, etc.), a chelator, such as DOTA (1,4,7,10-tetraazacyclododecane-1,4,7,10-tetraacetic acid), or an MRI active molecule, such as gadolinium-DTPA (gadolinium-diethylenetriaminepentacetate). Procedures for accomplishing the binding of the tracking molecule to the polymer or to the protein are well known to the art. A tracking molecule may allow for locating the conjugate after it has been administered to a patient and targeted to specific cells. In this way, it is possible e.g. to direct the low energy thermal neutron irradiation to the location of the targeted conjugate in boron neutron capture therapy.

In an embodiment, the tracking molecule is bound to the polymer derivative via a bond or a group formed by a reaction between at least one aldehyde group formed by oxidative cleavage of a saccharide unit of the dextran derivative and a group of the tracking molecule. A suitable group of the tracking molecule may be e.g. an amino group.

In an embodiment, the tracking molecule comprises a linker group. The linker group may be any linker group described in this specification.

In an embodiment, P is a polymer derivative comprising at least one saccharide unit; and the conjugate comprises at least one tracking molecule bound to the polymer derivative via a bond or a group formed by a reaction between at least one aldehyde group formed by oxidative cleavage of a saccharide unit of the polymer derivative and a group of the tracking molecule.

It is possible that one or more aldehyde groups formed by oxidative cleavage of a saccharide unit of the polymer derivative is not reacted with an amino group of the protein or with a tracking molecule.

In an embodiment, the polymer derivative comprises at least one aldehyde group formed by oxidative cleavage of a saccharide unit of the polymer derivative which is capped.

The at least one aldehyde group may be capped by a suitable group, such as a reduced Schiff base.

The at least one aldehyde group may also be capped by a group formed by a reaction between the at least one aldehyde group and a hydrophilic capping agent, such as ethanolamine, lysine, glycine or Tris.

In an embodiment, ethanolamine comprises ¹⁴C.

In an embodiment, the polymer derivative comprises at least one aldehyde group formed by oxidative cleavage of a saccharide unit of the polymer derivative that is not reacted with an amino group of the protein or with a tracking molecule and which is capped.

In an embodiment, essentially all of the aldehyde groups formed by oxidative cleavage of one or more saccharide units of the polymer derivative are capped.

In an embodiment, the polymer derivative comprises a plurality of aldehyde groups formed by oxidative cleavage of a saccharide unit of the polymer derivative, wherein essentially all of the aldehyde groups formed by oxidative cleavage of one or more saccharide units of the polymer derivative are capped.

In an embodiment, the polymer is substituted by at least one substituent selected from the group consisting of

-O-(CH₂)ₚCH=CH₂ or -O-(CH₂)ₚC≡CH,

wherein p is in the range of 1 to 8.

While such an embodiment is typically not desirable, it may occur as a side product, when said substituent has not reacted. At least one carbon of the polymer may be thus substituted. At least one saccharide unit of the polymer may be thus substituted.

In an embodiment, p is 1, 2, 3, 4, 5, 6, 7 or 8. In an embodiment, p is in the range of 1 to 2, or in the range of 1 to 3, or in the range of 1 to 4, or in the range of 1 to 5, or in the range of 1 to 6, or in the range of 1 to 7.

In an embodiment, the conjugate comprises an anti-EGFR1 antibody or an EGFR1 binding fragment thereof and at least one dextran derivative, wherein
the dextran derivative comprises at least one D-glucosyl unit, wherein at least one carbon selected from carbon 2, 3 or 4 of the at least one D-glucosyl unit is substituted by a substituent of the formula

   -O-(CH₂)ₙ-S-B₁₂H₁₁²⁻
wherein n is in the range of 3 to 10; and
the dextran derivative is bound to the anti-EGFR1 antibody or an EGFR1 binding fragment thereof via a bond formed by a reaction between at least one aldehyde group formed by oxidative cleavage of a D-glucosyl unit of the dextran derivative and an amino group of the anti-EGFR1 antibody or an EGFR1 binding fragment thereof.

In an embodiment, the conjugate comprises about 300 boron atoms (300B), about 400 boron atoms (400B), about 500 boron atoms (500B), about 600 boron atoms (600B), about 700 boron atoms (700B), about 800 boron atoms (800B), about 900 boron atoms (900B), about 1000 boron atoms (1000B), about 1100 boron atoms (1100B), about 1200 boron atoms, about 1300 boron atoms (1300B), about 1400 boron atoms (1400B), or about 1500 boron atoms (1500B). E.g "900B" refers to a conjugate carrying per one mole of protein one mole of dextran, that carries ca. 900 moles of boron atoms in BSH molecules.

In an embodiment, the conjugate is suitable for use in boron neutron capture therapy. "Boron neutron capture therapy" (BNCT) should be understood as referring to targeted radiotherapy, wherein nonradioactive boron-10 is irradiated with low energy thermal neutrons to yield biologically destructive alpha particles and lithium-7 nuclei. The nonradioactive boron-10 may be targeted by incorporating it in a tumor localizing drug such as a tumor localizing conjugate.

The present invention also relates to a method for preparing the conjugate according to the claims, comprising the steps of:
a) reacting at least one hydroxyl group of a polymer selected from the group consisting of dextran, hyaluronic acid, hydroxyethyl starch, and cyclodextrin with an alkylating agent to obtain alkylated polymer,
   wherein the alkylating agent has a structure represented by the

      X-(CH₂)ₙCH=CH₂ or X-(CH₂)ₙC≡CH
   wherein n is in the range from 1 to 8, and X is Br, Cl, or I;
b) conjugating a payload molecule and optionally a linker group with the modified polymer obtainable from step a) to obtain a payload molecule-modified polymer conjugate; and
c) conjugating the alkylated polymer obtainable from step a) or the payload molecule-modified polymer conjugate obtainable from step b) with a protein to obtain a conjugate.

The steps b) and c) may, in principle, be executed in this order or in a different order, depending on the chemistry utilized in the steps. The steps b) and c) may also be carried out simultaneously.

In an embodiment, n is 1, 2, 3, 4, 5, 6, 7 or 8. In an embodiment, n is in the range of 1 to 2, or in the range of 1 to 3, or in the range of 1 to 4, or in the range of 1 to 5, or in the range of 1 to 6, or in the range of 1 to 7.

In an embodiment, the polymer has a molecular mass in the range of about 3 to about 2000 kDa, or about 10 to about 100 kDa, or about 5 to about 200 kDa, or about 10 to about 250 kDa. The polymer having a molecular mass in said range should be understood as referring to polymer that has not been subjected to steps a)-c), i.e. polymer that has not been alkylated.

In this context, the term "alkylation" or "alkylating" should be understood as referring to the transfer of an alkenyl or an alkynyl group to the polymer to give an alkyl (alkenyl or alkynyl) ether. In other words, at least one hydroxyl group of the polymer becomes an alkyloxy (alkenyloxy or alkynyloxy) group. In an embodiment, "alkylating" refers to alkenylating. In an embodiment, "alkylating" refers to alkynylating.

One or more of hydroxyl groups of the polymer may react in the alkylation reaction. If the polymer comprises saccharide units, one or more, or a plurality of, saccharide units of polymer may be alkylated.

In embodiments in which the polymer is dextran, in step a), one or more of hydroxyl groups bound to carbons 2, 3 or 4 of at least one D-glucosyl unit of dextran may react in the alkylation reaction. One or more, or a plurality of, D-glucosyl units of dextran may be alkylated.

In an embodiment, the alkylating agent has a structure represented by the formula

X-(CH₂)ₙCH=CH₂

wherein n is in the range from 1 to 8, and X is Br, Cl, or I.

In an embodiment, the alkylating agent has a structure represented by the formula

X-(CH₂)ₙC≡CH

wherein n is in the range from 1 to 8, and X is Br, Cl, or I.

In an embodiment, the alkylating agent is allyl bromide.

In an embodiment, the alkylating agent is propargyl bromide.

In an embodiment, the polymer is dextran, and the dextran is alkenylated in step a) using an alkenylating agent, wherein the alkenylating agent has a structure according to the formula

X-(CH₂)ₙCH=CH₂

wherein n is in the range from 1 to 8, and X is Br, Cl, or I.

In an embodiment, at least one carbon selected of at least one saccharide unit of the alkylated polymer obtainable from step a) is substituted by a substituent of the formula

-O-(CH₂)ₙCH=CH₂ or -O-(CH₂)ₙC≡CH,

wherein n is in the range of 1 to 8.

In an embodiment, n is 1, 2, 3, 4, 5, 6, 7 or 8. In an embodiment, n is in the range of 1 to 2, or in the range of 1 to 3, or in the range of 1 to 4, or in the range of 1 to 5, or in the range of 1 to 6, or in the range of 1 to 7.

In step a), the molar ratio of the alkylating agent to the polymer may be suitably selected in order to obtain an alkylated polymer comprising a desired number of alkyl groups. In an embodiment, about 25-45% of hydroxyl groups of the polymer are alkylated by the alkylating agent. In an embodiment, the alkylated polymer comprises about one alkyl group per 2 to 4 saccharide units of the polymer. In this context, alkyl group refers to the group added to the polymer by the reaction with the alkylating agent. The number of alkyl groups may depend e.g. on the hydrophobicity of the payload molecule. In an embodiment, about 1-25% of hydroxyl groups of the polymer are alkylated by the alkylating agent. In an embodiment, the alkylated polymer comprises about one alkyl group per 3 to 20 saccharide units of the polymer.

In an embodiment, the method comprises the step
b1) reacting the modified polymer obtainable from step a) with a compound comprising a functional group capable of reacting with an alkene or an alkyne.

In an embodiment, the functional group capable of reacting with an alkene or an alkyne is selected from the group consisting of sulfhydryl, amino, and azidyl.

In an embodiment, the modified polymer obtainable from step a) is reacted with a compound comprising a functional group capable of reacting with an alkene or an alkyne in the presence of a catalyst in step b1).

In an embodiment, the catalyst is a radical initiator. The radical initiator is capable of catalyzing the reaction between the functional group capable of reacting with an alkene or an alkyne and with the alkenyl or alkynyl group(s) of the modified polymer.

In this specification, "radical initiator" should be understood as referring to an agent capable of producing radical species under mild conditions and promote radical reactions. The term "radical initiator" may also refer to UV (ultraviolet) light. UV light irradiation is capable of generating radicals, e.g. in the presence of a suitable photoinitiator. Suitable radical initiators include, but are not limited to, inorganic peroxides such as ammonium persulfate or potassium persulfate, organic peroxides, and UV light. In an embodiment, the radical initiator is selected from the group consisting of ammonium persulfate, potassium persulfate and UV light.

In an embodiment, the catalyst is copper(I).

In an embodiment, the catalyst is a Grubbs catalyst.

In an embodiment, the method comprises the step
b1) reacting the modified polymer obtainable from step a) with a compound comprising a functional group capable of reacting with an alkene or an alkyne and a secondary functional group.

In an embodiment, the compound comprising a functional group capable of reacting with an alkene or an alkyne and a secondary functional group comprises a linker group. The linker group may be any linker group described in this specification.

In an embodiment, the functional group capable of reacting with an alkene or an alkyne is selected from the group consisting of sulfhydryl, amino, and azidyl; and the secondary functional group is independently selected from the group consisting of sulfhydryl, amino, alkenyl, alkynyl, azidyl, aldehyde, carboxyl, maleimidyl, succinimidyl and hydroxylamino.

In an embodiment, the secondary and/or tertiary functional group is amino.

In an embodiment, the secondary and/or tertiary functional group is CH=CH₂ or CH=CHCH₂.

In an embodiment, the secondary and/or tertiary functional group is CH≡C, CH≡CCH₂ or DBCO.

In an embodiment, the secondary and/or tertiary functional group is azidyl.

In an embodiment, the secondary compound comprising a functional group capable of reacting with the secondary functional group and a tertiary functional group comprises a linker group. The linker group may be any linker group described in this specification.

In an embodiment, the compound comprising a functional group capable of reacting with an alkene or an alkyne has a structure according to formula Z-(CH₂)ₚ-SH, wherein Z is sulfhydryl, amino, alkenyl, alkynyl, azidyl, aldehyde, carboxyl, maleimidyl, succinimidyl or hydroxylamino; and p is in the range from 1 to 10.

In an embodiment, p is 3, 4, 5, 6, 7, 8, 9 or 10. In an embodiment, p is in the range of 3 to 4, or in the range of 3 to 5, or in the range of 3 to 6, or in the range of 3 to 7, or in the range of 3 to 8, or in the range of 3 to 9.

In an embodiment, Z is selected from the group consisting of sulfhydryl, amino, alkenyl, alkynyl and azidyl.

In an embodiment, Z is amino.

In an embodiment, Z is CH=CH₂ or CH=CHCH₂.

In an embodiment, Z is CH≡C, CH≡CCH₂ or DBCO.

In an embodiment, Z is azidyl.

In an embodiment, the compound comprising a functional group capable of reacting with an alkene or an alkyne and a secondary functional group is cysteamine.

In an embodiment, the compound comprising a functional group capable of reacting with an alkene or an alkyne and a secondary functional group comprises a saccharide. The saccharide may be a monosaccharide, disaccharide or trisaccharide. The saccharide may also be an oligosaccharide comprising from 1 to about 20, or from 1 to 10, or from 1 to 8, or from 1 to 6, or from 1 to 5, or from 1 to 4, or from 1 to 3, or from 1 to 2, or 1, 2, 3, 4 or 5 saccharide units.

In an embodiment, the saccharide comprises a sialic acid unit. The sialic acid may be a terminal saccharide unit in the saccharide.

In an embodiment, the saccharide comprises a sialyllactose. The sialyllactose may be sialylα2,6lactose or sialylα2,3lactose.

In an embodiment, the modified polymer obtainable from step a) is reacted with a compound comprising a functional group capable of reacting with an alkene or an alkyne and a secondary functional group in the presence of a catalyst in step b1).

In an embodiment, the catalyst is a radical initiator. In an embodiment, the catalyst is copper(I). In an embodiment, the catalyst is a Grubbs catalyst.

In an embodiment, the method comprises the step
b1') reacting the modified polymer obtainable from step b1) with a secondary compound comprising a functional group capable of reacting with the secondary functional group and a tertiary functional group.

It should be understood that the secondary compound comprises at least two functional groups; first, a functional group capable of reacting with the secondary functional group, and second, a tertiary functional group.

The secondary and/or tertiary functional group may in principle be any functional group that may be utilized to conjugate a payload molecule, a linker group, a compound comprising a payload molecule and a linker group, or a protein. It may be selected e.g. so that the product of the method, i.e. a glycoprotein-donor molecule conjugate, may be linked to a molecule comprising the linker group and the toxic payload molecule by utilizing click conjugation such as copper(I)-catalysed azide-alkyne cycloaddition reaction (CuAAC). Click conjugation such as copper-free click chemistry may also be utilized. The secondary functional group may also be e.g. a sulfhydryl, amino, aldehyde, carboxyl, maleimidyl, succinimidyl or hydroxylamino group.

In an embodiment, the secondary and/or tertiary functional group is independently selected from the group consisting of sulfhydryl, amino, alkenyl, alkynyl, azidyl, aldehyde, carboxyl, maleimidyl, succinimidyl and hydroxylamino.

In an embodiment, the secondary compound comprising a functional group capable of reacting with the secondary functional group and a tertiary functional group comprises a linker group. The linker group may be any linker group described in this specification.

In an embodiment, the method comprises the step b2) reacting the modified polymer obtainable from step a), b1) or b1') with a payload molecule.

In an embodiment, the modified polymer obtainable from step a) or b1) is reacted with a payload molecule in the presence of a catalyst in step b2).

The payload molecule may comprise a functional group that allows it to react with the modified polymer obtainable from step a) or b1). Such functional groups may be e.g. amino, sulfhydryl, alkenyl, alkynyl, azidyl, aldehyde, carboxyl, maleimidyl, succinimidyl or hydroxylamino.

In an embodiment, the payload molecule comprises an alkenyl group. In such an embodiment, in step b2) the modified polymer obtainable from step a) or b1) is reacted with the payload molecule comprising an alkenyl group. This reaction between two alkenes is a cross metathesis reaction, resulting in a product also comprising a double bond. Such a reaction may be carried out in the presence of a Grubbs catalyst.

The payload molecule may also be modified so that it comprises a functional group that allows it to react with the modified polymer obtainable from step a) or b1).

In an embodiment, the catalyst is a radical initiator. In an embodiment, the catalyst is copper(I). In an embodiment, the catalyst is a Grubbs catalyst.

In an embodiment, the method comprises the step b2) of reacting sodium borocaptate (BSH) with the alkenylated dextran obtainable from step a) to obtain BSH-dextran. In other words, in step b2), the sulfhydryl group of BSH may react with an alkenyl group of the alkenylated dextran to form BSH-dextran to give a thioether. One or more BSH molecules may react with the alkenylated dextran. Therefore, BSH-dextran obtainable from step b) may contain a plurality of BSH moieties (i.e. groups of the formula -S-B₁₂H₁₁²⁻). The the sulfhydryl groups of BSH may react with alkenyl groups of a single alkenylated D-glucosyl unit containing more than one alkenyl group or with alkenyl groups of two or more alkenylated D-glucosyl units.

In an embodiment, the method comprises the step b2) reacting the modified polymer obtainable from step a) or b1) with a compound comprising
a functional group capable of reacting with the alkyl (alkenyl or alkynyl) group of the alkylated polymer or with the secondary functional group, and
a linker group and/or a payload molecule. Said compound may thus comprise the payload molecule, or it may comprise a functional group that may further be reacted with a payload molecule or with a compound comprising the payload molecule in order to conjugate the payload molecule to the modified polymer obtainable from step b2). Said compound may also comprise a linker group. The linker group may be any linker group described in this specification. The at least two functional groups of said compound may be selected independently of each other, or they may be the same.

In an embodiment, the functional group capable of reacting with the alkyl group of the alkylated polymer or with the secondary functional group is selected from the group consisting of amino, sulfhydryl, alkenyl, alkynyl, azidyl, aldehyde, carboxyl, maleimidyl, succinimidyl and hydroxylamino.

In an embodiment, the functional group that may further be reacted with a payload molecule or with a compound comprising the payload molecule is selected from the group consisting of amino, sulfhydryl, alkenyl, alkynyl, azidyl, aldehyde, carboxyl, maleimidyl, succinimidyl and hydroxylamino.

In an embodiment, modified polymer obtainable from step a) or b1) is reacted with the compound comprising a functional group capable of reacting with the alkyl group of the alkylated polymer or with the secondary functional group, and a linker group and/or a payload molecule in the presence of a catalyst in step b2).

In an embodiment, the catalyst is a radical initiator. In an embodiment, the catalyst is copper(I). In an embodiment, the catalyst is a Grubbs catalyst.

In step b), the weight ratio or the molar ratio of the payload molecule to the modified polymer may be suitably selected in order to obtain conjugates in which the number of payload molecules (i.e. m in the formula [D-L-Y-(CH₂)ₙ-O]ₘ-P-T) per polymer moiety (of the polymer derivative) varies. The number of payload moieties per polymer moiety may be measured e.g. by nuclear magnetic resonance as described in Example 2 or by inductively coupled plasma mass spectrometry (ICP-MS) as described in Example 9.

In an embodiment, the ratio of the payload molecule to the modified polymer present in step b) is in the range of 1:5 to 2:1, or in the range of 1:4 to 1:1 by weight, or in the range of 1:2 to 3:4 by weight. In an embodiment, the molar ratio of the payload molecule to the modified polymer present in step b) is in the range of 1:5 to 2:1, or in the range of 1:4 to 1:1, or in the range of 1:2 to 3:4. Typically, the higher the ratio of payload molecule to modified polymer, the higher the number of payload molecules per polymer moiety of the payload molecule-modified polymer conjugate obtainable from step b).

In this context, the linker group may be any linked group described in this specification.

The payload molecule may also be any payload molecule described in this specification.

The payload molecule is a toxic payload molecule or a boron compound.

In an embodiment, the payload molecule is a toxic payload molecule selected from the group consisting of dolastatin, auristatin, doxorubicin, maytansinoid, epirubicin, duocarmycin, and any analogue or derivative thereof.

In an embodiment, the method comprises the steps of:
a) reacting at least one hydroxyl group of a polymer selected from the group consisting of dextran, hyaluronic acid, hydroxyethyl starch, and cyclodextrin with an alkylating agent to obtain alkylated polymer,
wherein the alkylating agent has a structure represented by the formula

   X-(CH₂)ₙCH=CH₂ or X-(CH₂)ₙC≡CH
wherein n is in the range from 1 to 8, and X is Br, Cl, or I;
b1) reacting the modified polymer obtainable from step a) with a compound comprising a functional group capable of reacting with an alkene or an alkyne and a secondary functional group;
b1') reacting the modified polymer obtainable from step b1) with a secondary compound comprising a functional group capable of reacting with the secondary functional group and a tertiary functional group; and
b2) reacting the modified polymer obtainable from step b1') with a payload molecule.

In step b2), the tertiary functional group of the secondary compound and a group of the payload molecule react.

There are several possibilities for conjugating the alkylated polymer obtainable from step a) or the payload molecule-modified polymer conjugate obtainable from step b) with a protein to obtain a conjugate in step c).

The method comprises the steps
c1) oxidatively cleaving at least one saccharide residue of the payload molecule-modified polymer conjugate obtainable from step b), b1), b1') or b2) so that aldehyde groups are formed; and
c2) reacting the oxidatively cleaved payload molecule-modified polymer conjugate obtainable from step c1) with a protein to obtain a conjugate.

The step b) may, in principle, be executed before or after steps c1) and c2), depending on the chemistry utilized in the steps. The steps b), c1) and c2) may in some embodiments also be carried out simultaneously.

As described above, a bond between two carbons that are substituted by a hydroxyl group may be oxidatively cleaved in step c1). In the oxidative cleavage, the saccharide ring is opened between vicinal diols, leaving two aldehyde groups. Aldehyde groups of the oxidatively cleaved payload molecule-modified polymer conjugate obtainable from step c) may react with a protein to obtain a conjugate. The aldehyde groups may react with a suitable group such as an amino group.

The at least one saccharide residue of the payload molecule-modified polymer conjugate may, in principle, be oxidatively cleaved using any oxidizing agent capable of oxidatively cleaving the saccharide unit between two vicinal carbons substituted by free hydroxyl groups. The oxidizing agent may also be selected so that it essentially specifically oxidatively cleaves the at least one saccharide residue of the payload molecule-modified polymer conjugate. Such an oxidizing agent may not oxidize other groups or moieties of the payload molecule-modified polymer conjugate.

In an embodiment, the at least one saccharide residue of the payload molecule-modified polymer conjugate obtainable from step b), b1), b1') or b2) is oxidatively cleaved in step c1) using an oxidizing agent selected from the group consisting of sodium periodate, periodic acid and lead(IV) acetate.

In an embodiment, the at least one saccharide residue of the payload molecule-modified polymer conjugate obtainable from step b), b1), b1') or b2) is oxidatively cleaved in step c1) in an aqueous solution.

In an embodiment, the protein is modified by introducing therein a functional group prior to step c). Such a functional group may be e.g. sulfhydryl, amino, alkenyl, alkynyl, azidyl, aldehyde, carboxyl, maleimidyl, succinimidyl or hydroxylamino.

In an embodiment, the functional group is aldehyde.

In an embodiment, the functional group is CH=CH₂ or CH=CHCH₂.

In an embodiment, the functional group is CH≡C, CH≡CCH₂ or DBCO.

In an embodiment, the functional group is azidyl.

In an embodiment, the method comprises the steps
c3) optionally modifying the protein by adding a functional group capable of reacting with the modified polymer obtainable from step a) or with the payload molecule-modified polymer conjugate obtainable from step b), b1) or b2); and
c4) reacting the protein or the modified protein obtainable from step c3) with the modified polymer obtainable from step a) or with the payload molecule-modified polymer conjugate obtainable from step b), b1) or b2).

In an embodiment, the method comprises the steps
c3) optionally modifying the protein by adding a functional group capable of reacting with the secondary functional group and/or with the tertiary functional group; and
c4) reacting the protein or the modified protein obtainable from step c3) with the modified polymer obtainable from step a) or with the payload molecule-modified polymer conjugate obtainable from step b), b1) or b2).

The step b) may, in principle, be executed before or after steps c3) and c4), depending on the chemistry utilized in the steps. The steps b), c3) and c4) may in some embodiments also be carried out simultaneously.

The functional group capable of reacting with the modified polymer obtainable from step a) or with the payload molecule-modified polymer conjugate obtainable from step b), b1) or b2) may in principle be any functional group described in this specification. In an embodiment, the functional group capable of reacting with the modified polymer obtainable from step a) or with the payload molecule-modified polymer conjugate obtainable from step b), b1) or b2) selected from the group consisting of sulfhydryl, amino, alkenyl, alkynyl, azidyl, aldehyde, carboxyl, maleimidyl, succinimidyl and hydroxylamino.

The functional group capable of reacting with the modified polymer obtainable from step a) or with the payload molecule-modified polymer conjugate obtainable from step b), b1) or b2) may be selected independently of the functional group capable of reacting with the alkyl group of the alkylated polymer or with the secondary functional group, or it may be the same.

In an embodiment, the method comprises the steps of:
a) reacting at least one hydroxyl group of a polymer selected from the group consisting of dextran, hyaluronic acid, hydroxyethyl starch, and cyclodextrin with an alkylating agent to obtain alkylated polymer,
   wherein the alkylating agent has a structure represented by the formula

      X-(CH₂)ₙCH=CH₂ or X-(CH₂)ₙC≡CH
   wherein n is in the range from 1 to 8, and X is Br, Cl, or I;
b1) reacting the modified polymer obtainable from step a) with a compound comprising a functional group capable of reacting with an alkene or an alkyne and a secondary functional group;
b1') reacting the modified polymer obtainable from step b1) with a secondary compound comprising a functional group capable of reacting with the secondary functional group and a tertiary functional group;
b2) reacting the modified polymer obtainable from step b1') with a payload molecule;
c1) oxidatively cleaving at least one saccharide residue of the payload molecule-modified polymer conjugate obtainable from step b2) so that aldehyde groups are formed; and
c2) reacting the oxidatively cleaved payload molecule-modified polymer conjugate obtainable from step c1) with a protein to obtain a conjugate.

In an embodiment, the method comprises the steps of:
a) reacting at least one hydroxyl group of a polymer selected from the group consisting of dextran, hyaluronic acid, hydroxyethyl starch, and cyclodextrin with an alkylating agent to obtain alkylated polymer,
wherein the alkylating agent has a structure represented by the formula

   X-(CH₂)ₙCH=CH₂ or X-(CH₂)ₙC≡CH
wherein n is in the range from 1 to 8, and X is Br, Cl, or I;
b1) reacting the modified polymer obtainable from step a) with a compound comprising a functional group capable of reacting with an alkene or an alkyne and a secondary functional group;
b1') reacting the modified polymer obtainable from step b1) with a secondary compound comprising a functional group capable of reacting with the secondary functional group, and a tertiary functional group;
b2) reacting the modified polymer obtainable from step b1') with a payload molecule;
c3) optionally modifying the protein by adding a functional group capable of reacting with the secondary functional group and/or with the tertiary functional group; and
c4) reacting the protein or the modified protein obtainable from step c3) with the payload molecule-modified polymer conjugate obtainable from step b2).

In this embodiment, it is also possible to perform steps b2) and c4) simultaneously; so that step b2) is performed prior to step c4); or so that step c4) is performed prior to step b2).

In this context, the protein is an antibody.

In an embodiment, the protein is an antibody capable of binding to a target molecule selected from the group consisting of CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, epidermal growth factor receptor 1 (EGFR1), epidermal growth factor receptor 2 (HER2/neu), HER3, HER4 receptor, LFA-1, Mac1, p150.95, VLA-4, ICAM-1, VCAM, EpCAM, alpha₄/beta₇ integrin, alpha v/beta3 integrin including either alpha or beta subunits thereof, tissue factor (TF), tumor necrosis factor alpha (TNF-α), epidermal growth factor (EGF), human vascular endothelial growth factor (VEGF), glycoprotein IIb/IIIa, TGF-beta, alpha interferon (alpha-IFN), IL-8, IL-2 receptor, IgE, HIV-1 envelope glycoprotein gp120, cancer-associated high-mannose type N-glycans, blood group antigen Apo2, death receptor, flk2/flt3 receptor, obesity (OB) receptor, mpl receptor, CTLA-4, transferrin receptor, Lewis y, GD3, and a target molecule binding fragment thereof.

The protein typically contains at least one amino group, such as the N-terminal amine group and/or the amino group of a lysine residue. In step c2), the aldehyde groups of the oxidatively cleaved payload molecule-modified polymer conjugate obtainable from step c1) may thus react with the at least one amino group of the protein. The protein may also comprise other groups, such as at least one carboxyl group or at least one thiol group.

In an embodiment, the at least one amino group of the protein reacts in step c2) with at least one aldehyde group of the oxidatively cleaved payload molecule-modified polymer obtainable from step c1).

In an embodiment, the amino group of the protein is the amino group of a lysine residue of the protein.

In an embodiment, the oxidatively cleaved payload molecule-modified polymer is reacted with the protein by incubating the oxidatively cleaved payload molecule-modified polymer and the polymer in room temperature in an aqueous phosphate buffer having a pH of about 6 to 8 in step c2).

In an embodiment, the method comprises the step e) of reacting the modified polymer obtainable from step a), the payload molecule-modified polymer conjugate obtainable from step b), b1) or b2) or the conjugate obtainable from step c) or c1) with a tracking molecule.

In this context, the tracking molecule may be any tracking molecule described in this specification.

The tracking molecule may be selected so that it comprises a functional group capable of reacting with the modified polymer obtainable from step a) or with the payload molecule-modified polymer conjugate obtainable from step b), b1) or b2) or the conjugate obtainable from step c) or c1). The tracking molecule may also be modified by adding such a functional group.

In an embodiment, the tracking molecule comprises a linker group. The linker group may be any linker group described in this specification.

The tracking molecule may react with at least one aldehyde group of the oxidatively cleaved payload molecule-modified polymer conjugate obtainable from step c1). A suitable group of the tracking molecule that may react with the at least one aldehyde group may be e.g. an amino group.

In an embodiment, the method comprises the step f) of capping unreacted aldehyde groups of the oxidatively cleaved payload molecule-modified polymer conjugate obtainable from step c2) or e).

In an embodiment, the unreacted aldehyde groups are capped using a hydrophilic capping agent, such as ethanolamine, lysine, glycine or Tris.

In an embodiment, the hydrophilic capping agent is selected from the group consisting of ethanolamine, lysine, glycine and Tris.

In an embodiment, ethanolamine comprising ¹⁴C is a tracking molecule.

The capping may be stabilized using a reducing agent, such as NaCNBH₃. A capping group such as a reduced Schiff base may thus be formed.

The conjugate may be purified e.g. by gel filtration, for instance as described in Example 4.

In an embodiment, one or more steps selected from steps a), b), b1), b2), c), c1), and c2) are performed in an aqueous solution. A suitable aqueous solution may be e.g. an aqueous phosphate buffer having a pH of about 6 to 8.

In an embodiment, all of the steps a), b), b1), b2), c), c1) and/or c2) are performed in an aqueous solution.

In an embodiment, the method comprises the steps of
a') alkenylating at least one hydroxyl group of dextran to obtain alkenylated dextran;
b2') reacting sodium borocaptate (BSH) with the alkenylated dextran obtainable from step a) to obtain BSH-dextran;
c1') oxidatively cleaving at least one D-glucopyranosyl residue of the BSH-dextran so that aldehyde groups are formed;
c2') reacting the oxidatively cleaved BSH-dextran obtainable from step c) with a protein to obtain a conjugate.

In an embodiment, the protein is an anti-EGFR1 antibody or an EGFR1 binding fragment thereof.

In an embodiment, the dextran has a molecular mass in the range of about 3 to about 2000 kDa, or about 10 to about 100 kDa, or about 5 to about 200 kDa. The dextran having a molecular mass in said range should be understood as referring to dextran that has not been subjected to steps a')-c2'), i.e. dextran that has not been alkenylated.

In this context, the term "alkenylation" or "alkenylating" should be understood as referring to the transfer of an alkenyl group to a D-glucosyl unit of dextran to give an alkenyl ether. In other words, at least one hydroxyl group of the D-glucosyl unit of dextran becomes an alkenyloxy group.

In step a'), one or more of hydroxyl groups bound to carbons 2, 3 or 4 of at least one D-glucosyl unit of dextran may react in the alkenylation reaction. One or more, or a plurality of, D-glucopyranosyl units of dextran may be alkenylated.

In an embodiment, dextran is alkenylated in step a') using an alkenylating agent, wherein the alkenylating agent has a structure according to the formula

X-(CH₂)ₙCH=CH₂

wherein n is in the range from 1 to 8, and X is Br, Cl, or I.

In an embodiment, n is 1, 2, 3, 4, 5, 6, 7 or 8. In an embodiment, n is in the range of 1 to 2, or in the range of 1 to 3, or in the range of 1 to 4, or in the range of 1 to 5, or in the range of 1 to 6, or in the range of 1 to 7.

In an embodiment, the alkenylating agent is allyl bromide.

In an embodiment, at least one carbon selected from carbon 2, 3 or 4 of at least one D-glucosyl unit of the alkenylated dextran obtainable from step a) is substituted by a substituent of the formula

-O-(CH₂)ₙCH=CH₂,

wherein n is in the range of 1 to 8.

In an embodiment, n is 1, 2, 3, 4, 5, 6, 7 or 8. In an embodiment, n is in the range of 1 to 2, or in the range of 1 to 3, or in the range of 1 to 4, or in the range of 1 to 5, or in the range of 1 to 6, or in the range of 1 to 7.

In step b2'), the sulfhydryl group of BSH may react with an alkenyl group of the alkenylated dextran to form BSH-dextran to give a thioether. One or more BSH molecules may react with the alkenylated dextran. Therefore, BSH-dextran obtainable from step b2') may contain a plurality of BSH moieties (i.e. groups of the formula -S-B₁₂H₁₁²⁻). The the sulfhydryl groups of BSH may react with alkenyl groups of a single alkenylated D-glucosyl unit containing more than one alkenyl group or with alkenyl groups of two or more alkenylated D-glucosyl units.

Thus the BSH-dextran obtainable from step b2') may be a dextran derivative in which at least one carbon selected from carbon 2, 3 or 4 of the at least one D-glucosyl unit is substituted by a substituent of the formula

-O-(CH₂)ₙ-S-B₁₂H₁₁²⁻

wherein n is in the range of 3 to 10.

In an embodiment, BSH-dextran obtainable from step b2') comprises about 10 to about 300 or about 20 to 150 substituents of the formula -O-(CH₂)ₙ-S-B₁₂H₁₁²⁻, wherein n is in the range of 3 to 10.

In an embodiment, BSH is reacted with the alkenylated dextran obtainable from step a') in the presence of a radical initiator in step b2'). The radical initiator is capable of catalyzing the reaction between the sulfhydryl group(s) of BSH and with the alkenyl group(s) of alkenylated dextran.

In an embodiment, BSH is reacted with the alkenylated dextran obtainable from step a') in the presence of a radical initiator selected from the group consisting of ammonium persulfate, potassium persulfate and UV light in step b2').

In step b2'), the weight ratio or the molar ratio of BSH to alkenylated dextran obtainable from step a2') may be suitably selected in order to obtain conjugates in which the number of BSH moieties (i.e. the number of substituents of the formula -O-(CH₂)ₙ-S-B₁₂H₁₁²⁻) per dextran moiety (of the dextran derivative) varies. The number of BSH moieties per dextran moiety of the BSH-dextran may be measured e.g. by nuclear magnetic resonance as described in Example 2.

In an embodiment, the ratio of BSH to alkenylated dextran present in step b2') is in the range of 1:5 to 2:1, or in the range of 1:4 to 1:1 by weight, or in the range of 1:2 to 3:4 by weight. Typically, the higher the ratio of BSH to alkenylated dextran, the higher the number of BSH moieties per dextran moiety of the BSH-dextran.

The ratio of the radical initiator, such as ammonium persulfate or potassium persulfate, may also be varied in step b2'). In an embodiment, the ratio of the radical initiator to BSH and/or to dextran present in step b) is in the range of 1:5 to 2:1, or in the range of 1:4 to 1:1 by weight, or in the range of 1:2 to 3:4 by weight.

In an embodiment, the ratio of the radical initiator to alkenylated dextran in step b2') is in the range of 1:5 to 2:1, or in the range of 1:4 to 1:1 by weight, or in the range of 1:2 to 3:4 by weight.

As described above, a bond selected from the bond between carbons 2 and 3 and the bond between carbons 3 and 4 may be oxidatively cleaved in step c1'). In the oxidative cleavage, the D-glucosyl ring is opened between vicinal diols, leaving two aldehyde groups. Aldehyde groups of the oxidatively cleaved BSH-dextran obtainable from step c1') may react with a protein to obtain a conjugate. The aldehyde groups may react with a suitable group such as an amino group.

In an embodiment, the at least one D-glucosyl residue of the BSH-dextran is oxidatively cleaved in step c1') using an oxidizing agent selected from the group consisting of sodium periodate, periodic acid and lead(IV) acetate.

In an embodiment, the at least one D-glucopyranosyl residue of the BSH-dextran is oxidatively cleaved in step c1') in an aqueous solution.

The present invention also relates to a conjugate obtainable by the method according to one or more embodiments of the invention.

The present invention further relates to a pharmaceutical composition comprising the conjugate according to one or more embodiments of the present invention.

The pharmaceutical composition of the present invention may further comprise a pharmaceutically acceptable carrier. Examples of suitable pharmaceutically acceptable carriers are well known in the art and may include e.g. phosphate buffered saline solutions, water, oil/water emulsions, wetting agents, and liposomes. Compositions comprising such carriers may be formulated by methods well known in the art. The pharmaceutical composition may further comprise other components such as vehicles, additives, preservatives, other pharmaceutical compositions administrated concurrently, and the like.

In an embodiment, the pharmaceutical composition comprises an effective amount of the conjugate according to one or more embodiments of the invention.

In an embodiment, the pharmaceutical composition comprises a therapeutically effective amount of the conjugate according to one or more embodiments of the invention.

The term "therapeutically effective amount" or "effective amount" of the conjugate should be understood as referring to the dosage regimen for modulating the growth of cancer cells and/or treating a patient's disease. In BNCT this happens when cancer cells are bombarded with neutron radiation. The therapeutically effective amount may be selected in accordance with a variety of factors, including the age, weight, sex, diet and medical condition of the patient, the severity of the disease, and pharmacological considerations, such as the activity, efficacy, pharmacokinetic and toxicology profiles of the particular conjugate used. The therapeutically effective amount can also be determined by reference to standard medical texts, such as the Physicians Desk Reference 2004. The patient may be male or female, and may be an infant, child or adult.

The term "treatment" or "treat" is used in the conventional sense and means attending to, caring for and nursing a patient with the aim of combating, reducing, attenuating or alleviating an illness or health abnormality and improving the living conditions impaired by this illness, such as, for example, with a cancer disease.

In an embodiment, the pharmaceutical composition comprises a composition for e.g. oral, parenteral, transdermal, intraluminal, intraarterial, intrathecal, intra-tumoral (i.t.), and/or intranasal administration or for direct injection into tissue. Administration of the pharmaceutical composition may be effected in different ways, e.g. by intravenous, intraperitoneal, subcutaneous, intramuscular, intra-tumoral, topical or intradermal administration.

The present invention further relates to the conjugate according to one or more embodiments of the present invention or the pharmaceutical composition comprising the conjugate according to one or more embodiments of the present invention for use as a medicament.

In an embodiment, the medicament is for the intra-tumor treatment of head-and-neck cancer.

In an embodiment, the medicament is for the intravenous treatment of head-and-neck cancer.

In an embodiment, the medicament is for the intra-tumor and intravenous treatment of head-and-neck cancer.

The present invention further relates to the conjugate according to one or more embodiments of the present invention or the pharmaceutical composition comprising the conjugate according to one or more embodiments of the present invention for use in the treatment of cancer.

In an embodiment, the cancer is selected from the group consisting of head-and-neck cancer, leukemia, lymphoma, breast cancer, prostate cancer, ovarian cancer, colorectal cancer, gastric cancer, squamous cancer, small-cell lung cancer, multidrug resistant cancer and testicular cancer.

In an embodiment, the cancer is a head-and-neck cancer.

The present invention further relates to the conjugate according to one or more embodiments of the present invention or the pharmaceutical composition comprising the conjugate according to one or more embodiments of the present invention for use as a medicament for boron neutron capture therapy.

In an embodiment, the medicament is for the intra-tumor treatment of head-and-neck cancer by boron neutron capture therapy.

In an embodiment, the medicament is for the intravenous treatment of head-and-neck cancer by boron neutron capture therapy.

In an embodiment, the medicament is for the intra-tumor and intravenous treatment of head-and-neck cancer by boron neutron capture therapy.

The present invention further relates to the conjugate according to one or more embodiments of the present invention or the pharmaceutical composition comprising the conjugate according to one or more embodiments of the present invention for use in boron neutron capture therapy.

The present invention further relates to the conjugate according to one or more embodiments of the present invention or the pharmaceutical composition comprising the conjugate according to one or more embodiments of the present invention for use in the treatment of cancer by boron neutron capture therapy.

The use of the conjugate or the pharmaceutical composition according to one or more embodiments of the present invention in the manufacture of a medicament is also disclosed.

The conjugate or the pharmaceutical composition according to one or more embodiments of the present invention in the manufacture of a medicament for boron neutron capture therapy is also disclosed.

The use of the conjugate or the pharmaceutical composition according to one or more embodiments of the present invention in the manufacture of a medicament for the treatment of cancer is also disclosed.

In an embodiment, the cancer is a head-and-neck cancer.

In an embodiment, the cancer is selected from the group consisting of head-and-neck cancer, leukemia, lymphoma, breast cancer, prostate cancer, ovarian cancer, colorectal cancer, gastric cancer, squamous cancer, small-cell lung cancer, multidrug resistant cancer and testicular cancer.

The use of the conjugate or the pharmaceutical composition according to one or more embodiments of the present invention in the manufacture of a medicament for the treatment of cancer by boron neutron capture therapy is also disclosed.

A method of treating and/or modulating the growth of and/or prophylaxis of tumor cells in humans or animals is also disclosed, wherein the the conjugate according to one or more embodiments of the present invention or the pharmaceutical composition according to one or more embodiments of the invention is administered to a human or animal in an effective amount.

In one embodiment, the tumor cells are selected from the group consisting of leukemia cells, lymphoma cells, breast cancer cells, prostate cancer cells, ovarian cancer cells, colorectal cancer cells, gastric cancer cells, squamous cancer cells, small-cell lung cancer cells, head-and-neck cancer cells, multidrug resistant cancer cells, and testicular cancer cells, or metastatic, advanced, drug- or hormone-resistant, or multidrug resistant cancer cells, or versions thereof.

In an embodiment, the tumor cells express EGFR1.

In an embodiment, the conjugate or the pharmaceutical composition according to one or more embodiments of the invention is administered to a human in an effective amount in boron neutron capture therapy.

In an embodiment, the concentration of boron is analysed in tumor cells after administering the conjugate or the pharmaceutical composition.

In an embodiment, the concentration of boron is analysed in blood after administering the conjugate or the pharmaceutical composition.

In an embodiment, the concentration of boron is analysed in muscle, or in other non-tumor tissue, after administering the conjugate or the pharmaceutical composition.

The concentration of boron in tumor cells, in blood or in both may be analysed or measured e.g. by inductively coupled plasma mass spectrometry (ICP-MS) or by inductively coupled plasma atomic emission spectroscopy (ICP-AES) (e.g. Example 9). These methods measure the amount (in moles) or concentration of boron atoms in the sample.

The concentration of boron in tumor cells, in blood or in both may also be analysed or measured indirectly, e.g. by using an embodiment of the conjugate comprising a tracking molecule and analysing or measuring the concentration of the tracking molecule. For instance, if the tracking molecule is fluorescent or radioactive, the fluorescence or radioactivity of the tracking molecule may be measured or visualised.

In an embodiment, the concentration of boron is analysed in tumor cells and in blood after administering the conjugate or the pharmaceutical composition, and the ratio of the concentration of boron in tumor cells to the concentration of boron in blood is higher than 1:1, 2:1, 3:1, 4:1, 5:1, 6:1, 7:1, , 8:1, 9:1, 10:1, 11:1, 12:1, 13:1, 15:1, 20:1, 30:1, 40:1, 50:1, 60:1, 70:1, 80:1, 90:1, 100:1, 110:1, 120:1, 130:1, 140:1, 150:1, 200:1, 210:1, 220:1, 230:1, 240:1, or 250:1.

In an embodiment, the concentration of boron is analysed in tumor cells and in a muscle, or in other non-tumor tissue, after administering the conjugate or the pharmaceutical composition, and the ratio of the concentration of boron in tumor cells to the concentration of boron in a muscle, or other non-tumor tissue, is higher than 1:1, 2:1, 3:1, 4:1, 5:1, 6:1, 7:1, 8:1, 9:1, 10:1, 11:1, 12:1, 13:1, 15:1, 20:1, 30:1, 40:1, 50:1, 60:1, 70:1, 80:1, 90:1, 100:1, 110:1, 120:1, 130:1, 140:1, 150:1, 200:1, 210:1, 220:1, 230:1, 240:1, or 250:1.

In an embodiment, the ratio of the concentration of boron in tumor cells to the concentration of boron in blood, in a muscle, or in other non-tumor tissue is the molar ratio of boron atoms in tumor cells to the boron atoms in blood, in a muscle, or in other non-tumor tissue.

A method for modulating the growth of a cell population expressing EGFR1 protein is also disclosed, wherein the method comprises the step of
contacting the conjugate according to one or more embodiments of the invention or the pharmaceutical composition according to one or more embodiments of the invention with the cell population expressing EGFR1 protein.

In an embodiment, the cell population expressing EGFR1 protein is a cancer cell population or a tumor cell population.

In this context, the term "a cancer cell population" should be understood as referring to one or more cancer cell populations.

The conjugate may be contacted *in vitro, in vivo* and/or *ex vivo* to with the cell population, for example, cancer cells, including, for example, cancer of the blood, plasma, lung, breast, colon, prostate, kidney, pancreas, brain, bones, ovary, testes, and lymphatic organs; more preferably lung, colon prostrate, plasma, blood or colon cancer; "Modulating the growth of cancer cell populations" includes inhibiting the proliferation of cell populations from dividing to produce more cells; reducing the rate of increase in cell division as compared, for example, to untreated cells; killing cell populations; and/or preventing cell populations (such as cancer cells) from metastasizing. The growth of cell populations may be modulated *in vitro, in vivo* or *ex vivo.*

In an embodiment, the cancer is selected from the group consisting of head-and-neck cancer, leukemia, lymphoma, breast cancer, prostate cancer, ovarian cancer, colorectal cancer, gastric cancer, squamous cancer, small-cell lung cancer, multidrug resistant cancer and testicular cancer.

A method of treating and/or modulating the growth of and/or prophylaxis of tumor cells in humans is also disclosed, wherein the conjugate or the pharmaceutical composition according to one or more embodiments of the invention is administered to a human in an effective amount.

In an embodiment, the effective amount is a therapeutically effective amount.

In an embodiment, the conjugate of the pharmaceutical composition according to one or more embodiments of the invention is administered intra-tumorally to a human in an effective amount.

In an embodiment, the conjugate of the pharmaceutical composition according to one or more embodiments of the invention is administered intravenously to a human in an effective amount.

In an embodiment, the conjugate of the pharmaceutical composition according to one or more embodiments of the invention is administered intra-tumorally and intravenously to a human in an effective amount.

In an embodiment, the conjugate or the pharmaceutical composition according to one or more embodiments of the invention is administered intra-tumorally into head-and-neck tumor in an effective amount.

In an embodiment, the conjugate or the pharmaceutical composition according to one or more embodiments of the invention is administered to a human in an effective amount in boron neutron capture therapy.

In an embodiment, the tumor cells are selected from the group consisting of leukemia cells, lymphoma cells, breast cancer cells, prostate cancer cells, ovarian cancer cells, colorectal cancer cells, gastric cancer cells, squamous cancer cells, small-cell lung cancer cells, head-and-neck cancer cells, multidrug resistant cancer cells, and testicular cancer cells, metastatic, advanced, drug- or hormone-resistant, multidrug resistant cancer cells, and versions thereof.

A method of treating cancer in humans is also disclosed, wherein the conjugate or the pharmaceutical composition according to one or more embodiments of the invention is administered to a human in an effective amount.

In an embodiment, the conjugate or the pharmaceutical composition according to one or more embodiments of the invention is administered to a human in an effective amount in boron neutron capture therapy.

In an embodiment, the effective amount is a therapeutically effective amount.

In an embodiment, the conjugate of the pharmaceutical composition according to one or more embodiments of the invention is administered intra-tumorally to a human in a therapeutically effective amount in boron neutron capture therapy.

In an embodiment, the conjugate of the pharmaceutical composition according to one or more embodiments of the invention is administered intravenously to a human in a therapeutically effective amount in boron neutron capture therapy.

In an embodiment, the conjugate of the pharmaceutical composition according to one or more embodiments of the invention is administered intra-tumorally and intravenously to a human in a therapeutically effective amount in boron neutron capture therapy.

In an embodiment, the conjugate or the pharmaceutical composition according to one or more embodiments of the invention is administered intra-tumorally into head-and-neck tumor in a therapeutically effective amount in boron neutron capture therapy.

In an embodiment, the cancer is selected from the group consisting of head-and-neck cancer, leukemia, lymphoma, breast cancer, prostate cancer, ovarian cancer, colorectal cancer, gastric cancer, squamous cancer, small-cell lung cancer, multidrug resistant cancer and testicular cancer.

The embodiments of the invention described hereinbefore may be used in any combination with each other. Several of the embodiments may be combined together to form a further embodiment of the invention. A product, a use or a method to which the invention is related may comprise at least one of the embodiments of the invention described hereinbefore.

The conjugate according to one or more embodiments of the invention has a number of advantageous properties.

The polymer allows for conjugating a relatively large number of payload molecules to the protein. The polymer has low toxicity and low immunogenicity; yet it is well soluble in aqueous solutions.

The conjugates according to one or more embodiments of the invention comprising a boron compound are relatively nontoxic in the absence of low energy neutron irradiation.

The conjugate according to one or more embodiments of the invention has low antigenicity.

It contains a high number of boron-10 atoms per conjugate molecule. Further, it exhibits relatively good aqueous solubility.

The conjugate according to one or more embodiments of the invention also exhibits good pharmacokinetics. It has suitable retention in blood, high uptake in cells to which it is targeted and low uptake in cells and organs to which it is not targeted.

Its production process is relatively simple and can be performed in aqueous solutions.

The conjugate according to one or more embodiments of the invention is sufficiently stable towards chemical or biochemical degradation during manufacturing or in physiological conditions, e.g. in blood, serum, plasma or tissues.

### EXAMPLES

In the following, the present invention will be described in more detail. Reference will now be made in detail to the embodiments of the present invention, examples of which are illustrated in the accompanying drawings. The description below discloses some embodiments of the invention in such detail that a person skilled in the art is able to utilize the invention based on the disclosure. Not all steps of the embodiments are discussed in detail, as many of the steps will be obvious for the person skilled in the art based on this specification.

### EXAMPLE 1. Allylation of dextran

200 mg Dextran 70 kD (Sigma) was dissolved in 2 ml of 0.6 M NaOH. 250 µl of allyl bromide (Sigma) was added, and the reaction was allowed to proceed for 3 h at 60°C. The reaction mixture was then neutralized with 1M acetic acid and the product was isolated by precipitation with 10 volumes of cold acetone (-20°C). Precipitate was collected by centrifugation and washed twice with acetone. The allylated dextran (Scheme 1) was subjected to ¹H-NMR analysis, which showed that the level of allylation was ca. 36%.

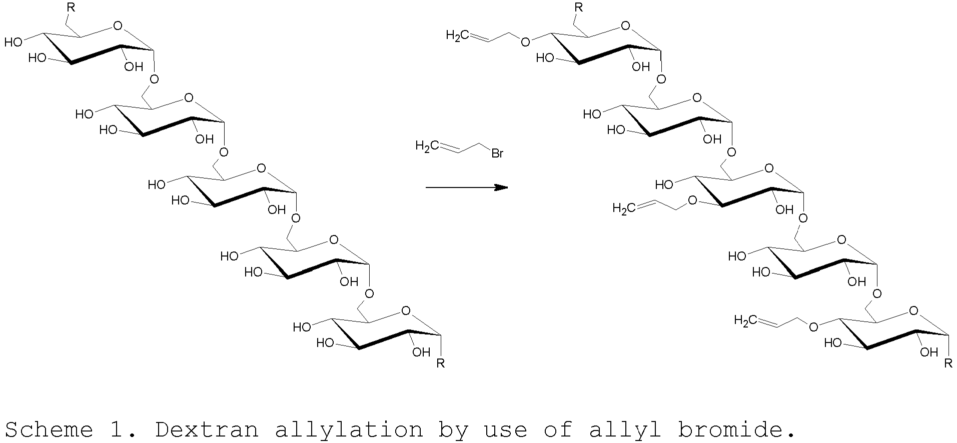

### EXAMPLE 2. Addition of BSH to allyl dextran

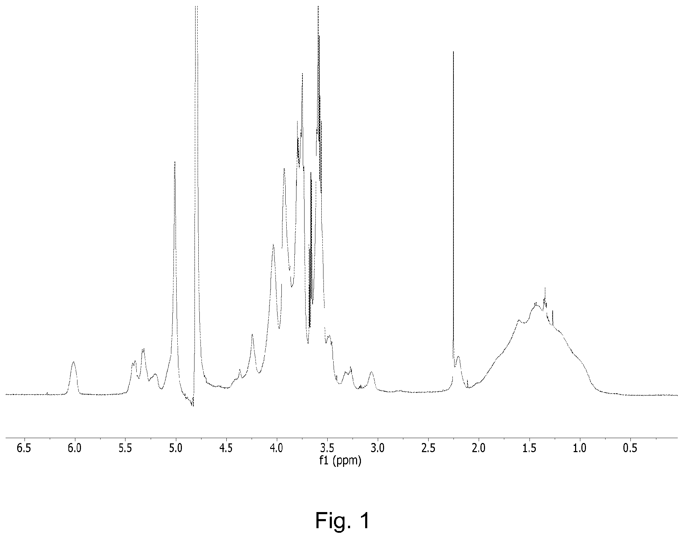

50 mg allyl dextran 70 kD prepared as described in Example 1, 50 mg ammonium persulfate and 50 mg sodium borocaptate (BSH; Katchem Ltd, Czech Republic) were dissolved in 0.5 ml H₂O.
The reaction was allowed to proceed for 2 h at 50°C. The reaction product, BSH-dextran (Scheme 2), was isolated with ultrafiltration using centrifugal filter (Amicon, 10K cut-off). ¹H-NMR analysis showed that on average 100 BSH units were linked to allyl dextran, corresponding to 1200 boron atoms per dextran chain (Fig. 1). With minor modifications, e.g. by use of lower allylation level in dextran, BSH dextran with ca. 900 borons or 800 borons per dextran chain were obtained.

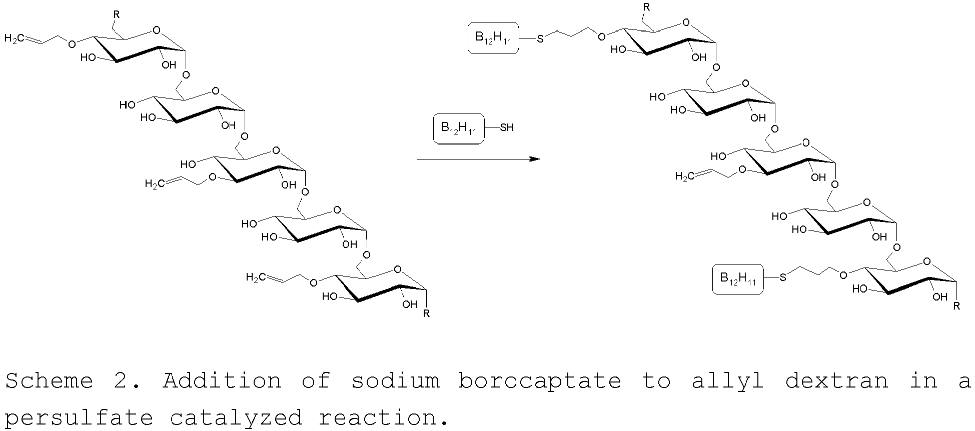

By varying the amount of BSH and persulfate in the reaction described above, it was possible to prepare BSH-dextrans with a clearly lower BSH level: 1) In a reaction containing 20 mg allyl dextran, 15 mg ammonium persulfate and 15 mg BSH, the isolated BSH-dextran was found to contain ca. 700 boron atoms per dextran chain. 2) In a reaction containing 20 mg allyl dextran, 10 mg ammonium persulfate and 10 mg BSH, the isolated BSH-dextran was found to contain ca. 560 boron atoms per dextran chain. 3) In a reaction containing 20 mg allyl dextran, 5 mg ammonium persulfate and 5 mg BSH, the isolated BSH-dextran was found to contain ca. 360 boron atoms per dextran chain.

### EXAMPLE 3. Oxidation of BSH-dextran

50 mg of BSH-dextran prepared as described in Example 2 was dissolved in 3 ml of 25 mM NaIO₄ in 0.1 M sodium acetate, pH 5.5. The reaction tube was covered with aluminium foil and incubated at RT overnight. The reaction product, Oxidized BSH-dextran (Scheme 3), was isolated with ultrafiltration using centrifugal filter (Amicon, 10K cut-off).

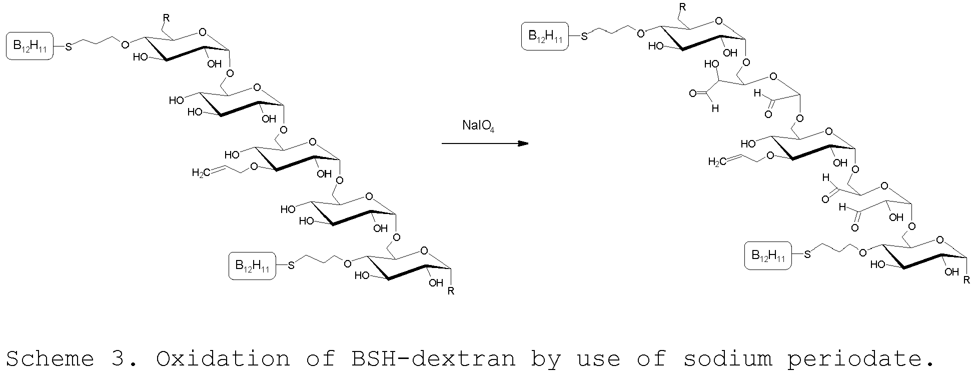

### Example 4. Conjugation of oxidized BSH-dextran to anti-EGFR1 Fab/F(ab')2

2 mg (40 nmol) of anti-EGFR1 Fab in 2 ml of phosphate buffered saline (PBS) was mixed with 5.1 mg (60 nmol) of oxidized BSH-dextran (Example 3) in 1.6 ml of PBS. Reaction was allowed to proceed overnight at RT. 400 µl of 0.5 M NaCNBH₃ was added to the reaction to stabilize the aldehyde-lysine linkages and the reaction was incubated for 2 hours at RT. 800 µl of 0.2 M ethanolamine-HCl pH 8 was added and the reaction was incubated for 1 hour at RT. 400 µl of 0.5 M NaCNBH₃ was added to stabilize ethanolamine capping and the reaction was incubated for 2 hours at RT. Low molecular weight reagents were removed by Amicon centrifugal filter unit (MWCO 30K) according to manufacturer's instructions using PBS as washing eluent.

2 mg (40 nmol) of anti-EGFR1 F(ab')2 in 2 ml of phosphate buffered saline (PBS) was mixed with 2.56 mg (30 nmol) of oxidized BSH-dextran (Example 3) in 1.6 ml of PBS. Conjugate was stabilized, capped and purified by ultrafiltration as above.

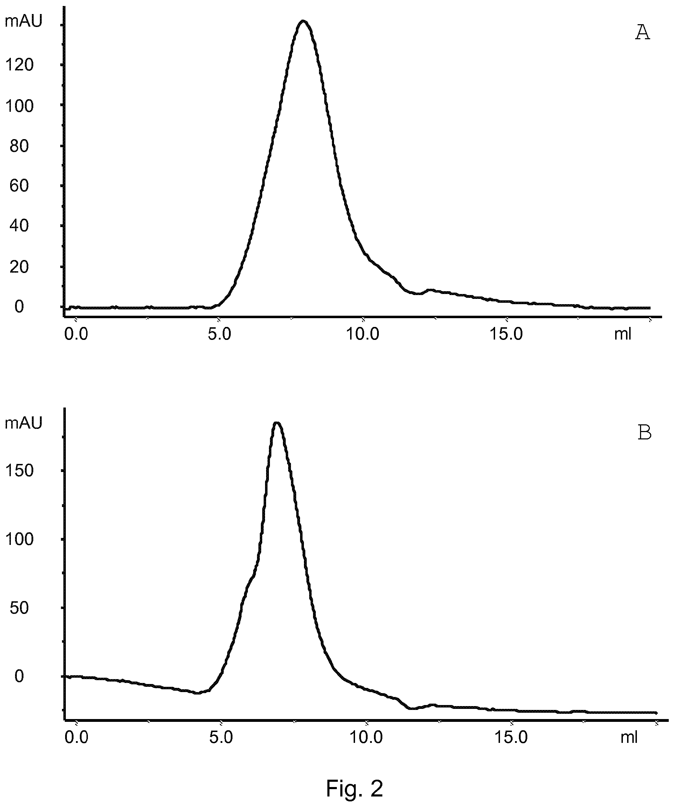

Both conjugates were analyzed by Äkta purifier (GE Healthcare) with Yarra 3 µm SEC-3000 gel filtration column (300 x 7.8 mm; Phenomenex) using 10 % acetonitrile (ACN)-50 mM Tris-HCl, pH 7.5 as elution buffer (Fig. 2).

### Example 5. Generation of Anti-EGFR1-Fab and -F(ab')2 , and control-Fab and -F(ab')2 fragments

Fab and F(ab')2 fragments were generated either from commercial cetuximab (Erbitux, Roche) or cetuximab produced in CHO cells (Freedom CHO-S kit, Invitrogen). Freedom CHO-S Kit (Life Technologies) was used for the development of stable cell lines producing cetuximab. The work was done according to manufacturer's instructions. Optimized nucleotide sequences encoding the heavy and light chain sequences were purchased from GeneArt (Life Technologies) and cloned separately into pCEP4 expression vectors (Life Technologies). For stable expression, the FreeStyle^{™} CHO-S cells were transfected with linearized 1:1 light chain and heavy chain vectors. Transfectants were selected with puromycin and methotrexate after which clone isolation was done by limited dilution cloning. Cloned cell lines were scaled up and assessed for productivity.

Control-Fab and -F(ab')2 fragments were generated from commercial omalizumab (anti-IgE) (Xolair, Novartis).

Anti-EGFR1 Fab fragments were prepared by digesting antibody with immobilized papain (Pierce) according to manufacturer's instructions with minor modifications. The used ratio of enzyme to substrate was 1:60 (w/w) and incubation time was 7 h. Fab fragments were separated from undigested IgG and Fc fragments with a column of immobilized protein A (Thermo Scientific) according to manufacturer's instructions.

Anti-EGFR1 F(ab')2 fragments were prepared by digesting antibody with either FragIT MaxiSpin (Genovis) according to manufacturer's instructions or with Fabricator enzyme (Genovis) according to manufacturer's instructions with minor modifications. Fabricator enzyme digestion was performed with 120 Units of enzyme per mg of antibody in 50 mM sodium phosphate buffer pH 6.6 and incubation time was 1 h at +37°C. F(ab')2 fragments were purified with an immobilized HiTrap protein L column (GE Healthcare) according to manufacturer's instructions. Reaction buffer was changed to PBS with Amicon Ultra concentrator (Millipore) (10 kDa cutoff).

The generated fragments were identified with SDS-PAGE and the protein concentration of each fragment was determined by measuring UV absorbance at 280 nm.

### EXAMPLE 6. SDS-PAGE analysis of boron conjugates

Boron conjugates of anti-EGFR1 Fab and F(ab')2 fragments were analyzed using SDS-PAGE in order to verify that the conjugations have been successful and that unconjugated Fab or F(ab')2 fragments are not present after conjugation. Figure 3 shows an SDS-PAGE analysis of anti-EGFR1 Fab/F(ab')2 boron conjugates with different amounts of boron in a gradient gel (Bio-Rad, 4-15 %) under nonreducing (panel A) and reducing (panel B) conditions. The results of panel A show that conjugation has been complete (or near complete) because unconjugated Fab or F(ab')2 fragments were not visible. BSH is a negatively charged molecule and when conjugated to a protein the migration velocity of a conjugate is faster on a gel than expected based on its theoretical molecular weight. The example of Figure 3 (Panel A) indicates that conjugates with a high amount of boron migrate faster on a nonreducing gel than conjugates with lower amount of boron (e.g. compare lanes 1, 2, 4 and 6). The results of Figure 3 (Panel A) also indicate that most of the conjugates are separated into two bands on a nonreducing gel implying that the samples contain a mixture of two different kinds of conjugates. SDS-PAGE analysis of boron conjugates in reducing conditions (Figure 3, panel B) show that all Fab conjugates with different amounts of boron migrate similarly on the gel under reducing conditions (Lanes 1, 2, 4, 6). Likewise, reduced F(ab')2 conjugates with different amounts of boron migrate identically (Lanes 3, 5, 7). In general, reduced boron conjugates migrate faster on the gel than nonreduced conjugates.

### EXAMPLE 7. In vitro internalization assays of boron conjugates

### AlexaFluor488 labeling of boron conjugates

5 µg AlexaFluor488 carboxylic acid, succinimidyl ester label (Invitrogen) was incubated with 100 µg of boron conjugates (anti-EGFR1-Fab, anti-EGFR1-F(ab')2, anti-EGFR1-mAb, control-Fab, control-F(ab')2, control-mAb) or corresponding nonconjugated compounds for 15 min at room temperature in a buffer containing 10 µl 1 M NaHCO₃, pH 9 in 100 µl PBS. After incubation excess label was removed by changing the buffer to PBS with Amicon Ultra concentrator (Millipore) (10 kDa cutoff). Protein concentration of each compound was determined by measuring UV absorbance at 280 nm and the degree of labeling was calculated according to the manufacturer's instructions (Invitrogen).

### Tritium labeling of boron conjugates

After removal of toluene solvent by evaporation, 100 µCi tritium labeled N-Succinimidyl propionate (Perkin Elmer) was incubated with 100 µg of anti-EGFR1-Fab-BSH(800B)-Dex, anti-EGFR1-F(ab')2-BSH(800B)-Dex, anti-EGFR1-mAb and control-mAb in a buffer containing 20 µl 1 M Na-borate buffer, pH 8.8 in 100 µl PBS. Reaction was allowed to proceed overnight at room temperature and then excess label was removed by changing the buffer to PBS with an Amicon Ultra concentrator (10 kDa cutoff). The amount of radioactivity was measured with scintillation counter in the presence of scintillation fluid cocktail (Ultima Gold, Perkin Elmer). The amount of tritium label in compounds was calculated as cpm/µg protein.

### Cell culture

HSC-2 cells (human squamous cell carcinoma of mouth, JCRP Cellbank, Japan) and FaDu cells (human squamous cell carcinoma of pharynx, ATCC) were cultured in T75 flasks in Eagle's minimal essential medium with 2 % glutamine, 10 % fetal bovine serum and 1 % penicillin/streptomycin. HEK (Human Embryonic Kidney, ATCC) cells were cultured in T75 flasks in Dulbecco's Modified Eagle Medium with 2 % glutamine, 10 % fetal bovine serum and 1 % penicillin/streptomycin.

### Internalization assay visualized in fluorescence microscopy

HSC-2 cells (5x10⁴) were seeded on a chamber slide and allowed to grow for 24 h. Then the cells were incubated for 3h at +37°C or at +4°C in 100µl media containing 10 µg/ml AlexaFluor488 labeled BSH-conjugates. After incubation cells were washed two times with PBS and fixed with 4 % paraformaldehyde for 20 min. Mounting media (Prolong Gold antifade reagent with DAPI) was added and the cells were covered with microscopy cover slips. Cells were photographed with fluorescence microscopy (Zeiss Axio Scope A1; ProgRes C5, JENOPTIK AG).

Internalization of anti-EGFR1-F(ab')2-BSH(900B)-Dex and nonconjugated anti-EGFR1-F(ab')2 by HSC-2 tumor cell line was analyzed by fluorescence microscopy (Figure 4). The experiment was carried out at +4°C (compounds bind to the cell surface but cannot be internalized) and at +37°C (cells are able to internalize the surface-bound compounds). Both nonconjugated anti-EGFR1-F(ab')2 and the boron conjugate bound to the cell surface at +4°C (Panels A and B) and were internalized at +37°C (Panels C and D). In fact, the boron conjugate was internalized more efficiently than nonconjugated anti-EGFR1-F(ab')2. Internalization assay with anti-EGFR1-Fab-BSH(900B)-Dex and EGFR1-mAb-BSH(900B)-Dex and corresponding nonconjugated anti-EGFR1-Fab and anti-EGFR1-mAb gave very similar results to the data presented in Figure 4 (not shown). The effect of boron load for internalization was examined using boron conjugates (anti-EGFR1-Fab-BSH-Dex and anti-EGFR1-F(ab')2-BSH-Dex) with different amounts of boron. The results indicated that conjugates with more boron were internalized more efficiently by HSC-2 cells than conjugates with low boron load at +37°C (not shown). Control-F(ab')2-BSH(900B)-Dex was internalized only very weakly (not shown).

### Internalization assay (FACS)

HSC-2, FaDu and HEK cells (2x10⁵) were seeded on a 24 well plate and allowed to grow for 24 h. Then the cells were incubated for 3 h at +37°C in 300 µl media containing 5 µg/ml AlexaFluor488 labeled compounds. After incubation cells were washed two times with PBS and detached by incubating with 100 µl Trypsin-EDTA for 10 min at +37°C. Cells were neutralized by adding 300 µl of media and resuspended in PBS and analyzed using a flow cytometer (FACS LRS II). The mean fluorescence intensity of each sample was calculated using FACS Diva software. The data presented in Tables 1-3 is expressed as "Normalized mean fluorescence intensity" where the fluorescence intensity has been normalized to the degree of labeling for each compound.

### Assays with FACS

Internalization of fluorescently labeled boron conjugates (900 boron atoms) and nonconjugated Ab fragments by human HNC cancer cell line HSC- 2 was evaluated using FACS. The results represent internalized plus cell surface bound compounds that occurs when cells have been incubated at +37°C (Table 4). Anti-EGFR1-Fab-BSH-Dex was internalized more efficiently than other boron conjugates or nonconjugated anti-EGFR1-Fab. Other anti-EGFR1 boron conjugates (anti-EGFR1-F(ab')2-BSH-Dex and anti-EGFR1-mAb-BSH-Dex) were internalized equally well to nonconjugated anti-EGFR1-Fab and anti-EGFR1-F(ab')2 . Boron conjugates of control-F(ab')2 and -mAb were internalized very weakly.

**Table 1. Cell surface binding and internalization of fluorescently labeled boron conjugates and nonconjugated compounds by HSC-2 cells. Analysis has been carried out by FACS and fluorescence intensity has been normalized to the degree of labeling for each compound.**

| | **HSC-2** |
|---|---|
| Sample | Normalized mean fluorescence intensity |
| Anti-EGFR1-Fab-BSH(900B)-Dex | 158700 |
| Anti-EGFR1-F(ab')2-BSH(900B)-Dex | 81100 |
| Control-F(ab')2-BSH(900B)-Dex | 2200 |
| Anti-EGFR1-mAb-BSH(900B)-Dex | 92700 |
| Control-mAb-BSH(900B)-Dex | 8200 |
| Anti-EGFR1-Fab | 99500 |
| Anti-EGFR1-F(ab')2 | 93100 |
| Anti-EGFR1-mAb | 21300 |
| Control-mAb | 700 |

Boron conjugates with different amounts of boron (360-900 boron atoms) were synthesized from anti-EGFR1 F(ab')2 and -Fab to study the effect of boron load in the internalization process. This Example shows an internalization assay with fluorescently labeled conjugates using human HNC cancer cell line HSC-2 and a control human cell line HEK. The results from flow cytometric analysis represent internalized plus cell surface bound compounds that occurs when cells have been incubated at +37°C (Table 2). Internalization of all boron conjugates of anti-EGFR1 Ab fragments was very similar as analyzed by flow cytometry. However, an experiment with microscopy revealed that conjugates with more boron were internalized more efficiently than conjugates with low boron load (not shown).

**Table 2. Cell surface binding and internalization of fluorescently labeled boron conjugates with different amounts of boron by HSC-2 and HEK cells. Analysis has been carried out by flow cytometry and fluorescence intensity has been normalized to the degree of labeling for each compound.**

| | **HSC-2** | **HEK** |
|---|---|---|
| **Sample** | Normalized mean fluorescence intensity | |
| **Anti-EGFR1-Fab-BSH(900B)-Dex** | 33900 | |
| **Anti-EGFR1-Fab-BSH(700B)-Dex** | 48300 | 590 |
| **Anti-EGFR1-Fab-BSH(560B)-Dex** | 48000 | 860 |
| **Anti-EGFR1-Fab-BSH(360B)-Dex** | 37000 | 470 |
| **Anti-EGFR1-F(ab')2 -BSH(700B)-Dex** | 41900 | 600 |
| **Anti-EGFR1-F(ab')2 -BSH(560B)-Dex** | 48400 | 530 |
| **Anti-EGFR1-F(ab')2 -BSH(360B)-Dex** | 43100 | 470 |
| **Anti-EGFR1-mAb** | 10700 | 110 |

Internalization of fluorescently labeled boron conjugates (1200 or 800 boron atoms) and nonconjugated Ab fragments by human HNC cancer cell lines (HSC-2 and FaDu) and a control cell line HEK was evaluated using flow cytometry. The results represent internalized plus cell surface bound compounds that occurs when cells have been incubated at + 37°C (Table 3). Anti-EGFR1-Fab-BSH(1200B)-Dex and nonconjugated anti-EGFR1-Fab showed strongest internalization by HSC-2 and FaDu cells. Internalization by FaDu cells has been consistently weaker than by HSC-2 cells, likely due to the smaller amount of EGFR1 receptors at the cell surface. Control boron conjugates (control-Fab-BSH(800B)-Dex and control-F(ab')2-BSH(800B)-Dex) and corresponding nonconjugated compounds were internalized very weakly. Control cell line HEK internalized the boron conjugates and nonconjugated compounds only very weakly.

**Table 3. Cell surface binding and internalization of fluorescently labeled boron conjugates (1200B or 800B) and nonconjugated compounds by HSC-2, FaDu and HEK cells. Analysis has been carried out by flow cytometry and fluorescence intensity has been normalized to the degree of labeling for each compound.**

| | **HSC-2** | **FaDu** | **HEK** |
|---|---|---|---|
| **Sample** | Normalized mean fluorescence intensity | | |
| **Anti-EGFR1-Fab** | 43006 | 6820 | 274 |
| **Anti-EGFR1-F(ab')2** | 18432 | 3461 | 168 |
| **Control-Fab** | 1165 | 970 | 555 |
| **Control-F(ab')2** | 823 | 443 | 337 |
| **Anti-EGFR1-Fab-BSH(1200)-** | 45270 | 8060 | 615 |
| **Anti-EGFR1-F(ab')2-BSH(1200)-Dex** | 10043 | 2813 | 198 |
| **Control-Fab-BSH(800)-Dex** | 1233 | 428 | 158 |
| **Control-F(ab')2-BSH(800)-** | 236 | 169 | 61 |

### Internalization assay with radiolabeled samples

HSC-2, FaDu and HEK cells (2x10⁵) were seeded on a 24 well plate and allowed to grow for 24 h. Then the cells were incubated for 3 h at +37°C in 300 µl media containing 5 µg/ml tritium labeled compounds. After incubation media was removed and cells were washed three times with PBS and lysed by adding 300 µl 1 M NaOH. The amount of radioactivity in media and cell lysates was measured with scintillation counter in the presence of scintillation fluid cocktail (Ultima Gold). The amount of internalized compounds was calculated from the total amount of radioactivity per well and normalized to 100 000 cells.

Boron conjugates (800 boron atoms) of anti-EGFR1-Fab and -F(ab')2 as well as nonconjugated anti-EGFR1-mAb were labeled with tritium to the lysine residues of a protein part. Internalization assay with radiolabeled compounds was carried out using human HNC cancer cell lines, HSC-2 and FaDu, as well as a control cell line HEK. The results represent internalized plus cell surface bound compounds that occurs when cells have been incubated at + 37°C. The results (Table 4) indicate that boron conjugates of anti-EGFR1-Fab and -F(ab')2 were internalized as efficiently as nonconjugated anti-EGFR1-mAb by HSC-2 and FaDu cells. Internalization by HSC-2 cells was 100 times stronger than by FaDu cells likely due to the higher amount of EGFR1 receptors at the cell surface in HSC-2 cells. Control cell line HEK showed only very weak internalization.

**Table 4. Internalization of radiolabeled boron conjugates by HSC-2, FaDu and HEK cells. The amount of internalized compounds has been calculated from the total amount of radioactivity per well and normalized to 100 000 cells. The results are an average of three determinations +/- S.D.**

| | **HSC-2** | **FaDu** | **HEK** |
|---|---|---|---|
| **Samples** | % internalized/ 100000 cells | | |
| **Anti-EGFR1-Fab-BSH(800B)-Dex** | 4.0±0.3 | 0.04±0.02 | 0.004±0.001 |
| **Anti-EGFR1-F(ab')2-BSH(800B)-Dex** | 5.4±1.0 | 0.06±0.02 | 0.006±0.001 |
| **Anti-EGFR1-mAb** | 5.0±0.5 | 0.04±0.02 | 0.007±0.001 |
| **Control-mAb** | 0.1±0.1 | 0.01±0.01 | 0.002±0.002 |

### EXAMPLE 8. In vivo experiments with tritium labeled conjugates

### Preparation of mouse tissues and blood samples for liquid scintillation counting

Weighted mouse organs were dissolved to 1 ml of tissue solubilizer (Solvable^{™}, Perkin Elmer) per 0.2 g tissue. Samples were incubated overnight at +60°C. Then 150 µl of H₂O₂ was added per 300 µl of dissolved organ and samples were incubated for one hour at +60°C. Bones were treated first with 1 M HCl overnight at +60°C and then with Solvable and H₂O₂. The amount of radioactivity in the organs was measured with scintillation counter in a presence of scintillation fluid cocktail (Ultima Gold^{™}, Perkin Elmer). Data is presented as percent of total injected dose in g of tissue. The results are an average of three mice +/- SEM. Since each of the mice had two tumors, the results in tumors are an average of six determinations +/- SEM.

Blood samples in clearance tests were collected in Eppendorf tubes and the volumes were measured after adding 100 µl of Solvable and overnight incubation at +60°C. Then 100 µl of H₂O₂ was added and samples were incubated for one hour at +60°C. The amount of radioactivity in the blood samples was measured with scintillation counter in the presence of scintillation fluid cocktail (Ultima Gold, Perkin Elmer). Data is presented as a percent of total injected dose. The results are an average of two mice.

### Blood clearance of boron conjugates in non-tumor mice

Female adult mice of the same age (Harlan HSD:Athymic nude Foxn1nu) were used. Radiolabeled (3H) boron conjugates of anti-EGFR1-Fab and -F(ab')2 with 800B and 300B boron load were injected i.v. via tail vein in 100 µl PBS. Injected dose was 30 µg = 1.3-2 x 106 cpm per mouse and two mice per sample were used. Blood samples of approximately 10 µl were collected before and after injection at different time points and counted for radioactivity. At the end of the experiment (48 h) mice were sacrificed and organs were collected and counted for radioactivity for determination of tissue biodistribution of the conjugates.

Blood clearance study in non-tumor mice was carried out using 3H-labeled boron conjugates of anti-EGFR1-Fab and -F(ab')2 with 800B and 300B boron load. Two different boron loads were used to see whether the boron load has an effect on the clearance rate of the conjugate from blood circulation. The results indicate that blood clearance of boron conjugates was rapid and independent on the boron load (Table 5). Clearance rate was comparable to the clearance of corresponding nonconjugated F(ab')2 and Fab fragments (not shown). Tissue distribution study indicated that the boron conjugates were not accumulated into any organs at 48 h (not shown).

**Table 5. Blood clearance of boron conjugates in non-tumor mice. The results are an average of two determinations. Time is time after administration (min) and values % of total injected dose.**

| **Time** | **Anti-EGFR-Fab-BSH(300)-Dex** | **Anti-EGFR-Fab-BSH(800)-Dex** | **Anti-EGFR -Fab2-BSH(300)-Dex** | **Anti-EGFR -Fab2-BSH(800)-Dex** |
|---|---|---|---|---|
| **0** | 100.0 | 100.0 | 100.0 | 100.0 |
| **5** | 35.4 | 31.3 | 42.9 | 40.8 |
| **15** | 31.8 | 19.9 | 34.3 | 20.2 |
| **30** | 26.7 | 10.5 | 29.3 | 16.3 |
| **60** | 13.6 | 10.7 | 22.6 | 9.7 |
| **120** | 6.8 | 5.2 | 16.1 | 6.3 |
| **240** | 4.6 | 2.5 | 9.4 | 4.3 |
| **460** | 2.4 | 2.0 | 4.1 | 1.7 |
| **1440** | 0.9 | 0.8 | 1.7 | 1.1 |
| **2880** | 0.4 | 0.4 | 0.6 | |

### Biodistribution of boron conjugates in HSC-2 tumor mice

Female adult mice of the same age (Harlan HSD:Athymic nude Foxn1nu) were used. Two and half to three million HSC-2 cells (JCRP Cellbank, Japan) in 150 µl in EME-media and 50 % Matrigel were inoculated to both flanks of nude mice. The dosing was given when at least one tumor per mouse has grown to at least 6 mm diameter in size (6 - 10 mm) corresponding roughly to tumor volume of 100-500 mm³. Radiolabeled (3H) boron conjugates (800B) of anti-EGFR1-Fab/F(ab')2 and control-Fab/F(ab')2 were injected i.v. via tail vein in 100 µl PBS. Injected dose was 50 µg = 1.3-2.6 x 106 cpm per mouse and three mice per sample were used. Mice were sacrificed at different time points (24 h, 48 h and 72 h) and organs were collected and counted for radioactivity for determination of tissue biodistribution of the conjugates.

Tissue distribution of boron conjugates (Table 6) show that boron conjugates of anti-EGFR1-Fab and -F(ab')2 accumulated into tumors but not in any other organs, whereas control boron conjugates did not significantly accumulate into tumors. Tumor accumulation of boron conjugates of anti-EGFR1-Fab and -F(ab')2 was highest at 24 h and slowly decreased at later time points (48 h and 72 h).

**Table 6. Biodistribution of boron conjugates in HSC-2 tumor mice. The results represent an average of three determinations +/- SEM except for tumors that are an average of six determinations +/- SEM. Values are % of total injected dose/g organ.**

| **24 h** | **Anti-EGFR-Fab-BSH (800) - Dex** | **Anti-EGFR-Fab2-BSH (800) - Dex** | **Control-Fab-BSH (800) - Dex** | **Control-Fab2-BSH(800)-Dex** |
|---|---|---|---|---|
| **Organ** | | | | |
| **blood** | | 0.34±0.07 | 0.23±0.05 | 0.47±0.23 |
| **urine** | 0.16±0.07 | | 0.94±0.05 | |
| **liver** | 0.34±0. 03 | 0.28±0.03 | | 0.29±0.14 |
| **kidney** | 0.23±0.02 0.28±0.01 | 2.22±0.9 0.31±0.04 | 0.26±0.07 0.24±0.05 | 3.03±1.16 0.32±0.15 |
| **lung** | 0.19±0.02 | 0.44±0.14 | 0.19±0.04 | 0.45±0.30 |
| **muscle** | 0.19±0.01 | 0.21±0.05 | 0.17±0.06 | 0.20±0.09 |
| **skin** | 0.23±0.02 | 0.31±0.03 | 0.22±0.04 | 0.29±0.15 |
| **tumor** | 1.00±0.08 | 0.75±0.15 | 0.32±0.60 | 0.57±0.27 |
| | | | | |

| **48 h** | **Anti-EGFR-Fab-BSH (800) - Dex** | **Anti-EGFR-Fab2-BSH(800)-Dex** | **Control-Fab-BSH (800) - Dex** | **Control-Fab2-BSH(800)-Dex** |
|---|---|---|---|---|
| **Organ** | | | | |
| **blood** | 0.10±0.02 | 0.10±0.01 | | |
| **urine** | 0.36±0.11 | 0.46±0.04 | 0.10±0.01 0.28±0.17 | 0.20±0.02 1.00±0.32 |
| **liver** | 0.23±0.04 | 0.18±0.03 | 0.15±0.01 | 0.14±0.03 |
| **kidney** | 0.17±0.02 | 0.14±0.01 | 0.15±0.02 | 0.17±0.01 |
| **lung** | 0.10±0.02 | 0.10±0.01 | 0.09±0.02 | 0.12±0.01 |
| **muscle** | 0.11±0.01 | 0.12±0.01 | 0.11±0.01 | 0.15±0.01 |
| **skin** | 0.12±0.01 | 0.14±0.01 | 0.11±0.04 | 0.18±0.02 |
| **tumor** | 0.41±0.06 | 0.58±0.06 | 0.21±0.03 | 0.29±0.02 |
| | | | | |

| **72 h Organ** | **Anti-EGFR-Fab-BSH (800) - Dex** | **Anti-EGFR-Fab2-BSH (800) - Dex** | **Control-Fab-BSH (800) - Dex** | **Control-Fab2-BSH (800) - Dex** |
|---|---|---|---|---|
| **blood** | 0.06±0.01 | 0.08±0.01 | 0.08±0.01 | 0.10±0.01 |
| **urine** | 0.23±0.07 | 0.24±0.10 | 0.23±0.02 | 0.30±0.05 |
| **liver** | 0.11±0.01 | 0.15±0.02 | 0.12±0.01 | 0.09±0.01 |
| **kidney** | 0.11±0.02 | 0.12±0.01 | 0.12±0.01 | 0.12±0.01 |
| **lung** | 0.05±0.01 | 0.06±0.01 | 0.05±0.01 | 0.08±0.01 |
| **muscle** | 0.07±0.01 | 0.10±0.02 | 0.09±0.01 | 0.08±0.02 |
| **skin** | 0.08±0.01 | 0.11±0.01 | 0.08±0.01 | 0.09±0.01 |
| **tumor** | 0.25±0.04 | 0.30±0.05 | 0.11±0.01 | 0.18±0.02 |

Tumor vs. blood distribution of boron conjugates in HSC-2 xenograft mice was calculated at different time points (24 h, 48 h and 72 h) (Table 7). Tumor/blood ratio was 4-5 for anti-EGFR1-Fab conjugate and 2-6 for anti-EGFR1- F(ab')2 conjugate. Anti-EGFR1-Fab-BSH-Dex reached the maximum ratio earlier (24 h) than anti-EGFR1-F(ab')2-BSH-Dex (48 h). Tumor/blood ratio of control conjugates remained at a constant level throughout the study (approximately 1-2).

**Table 7. Tumor/blood distribution of boron conjugates in HSC-2 tumor mice. The results are based on an average of three determinations for blood samples and an average of six determinations for tumors (2 tumors per mouse) +/- S.D.**

| **Boron conjugate** | **24h** | **48h** | **72h** |
|---|---|---|---|
| **Anti-EGFR-Fab-BSH (800B)** | 4.2 ± 0.3 | 4.2 ± 1.1 | 4.0 ± 0.9 |
| **Anti-EGFR-Fab2-BSH (800B) -dex** | 2.2 ± 0.3 | 6.1 ± 1.4 | 3.8 ± 1.0 |
| **Control-Fab-BSH(800B)-dex** | 1.5 ± 0.3 | 2.2 ± 0.5 | 1.5 ± 0.3 |
| **Control-Fab2-BSH(800B)-dex** | 1.5 ± 0.5 | 1.8 ± 0.2 | 1.9 ± 0.5 |

### Biodistribution of boron conjugates in FaDu tumor mice

Female adult mice of the same age (Charles River Crl:Athymic nude Foxn1nu) were used. Three million FaDu cells (ATCC) in 150 µl in EME-media and 50 % Matrigel were inoculated to both flanks of nude mice. The dosing was given when at least one tumor per mouse has grown to at least 6 mm diameter in size (6 - 10 mm) corresponding roughly to tumor volume of 100-500 mm³. Radiolabeled (3H) boron conjugates (800B or 1200B) of anti-EGFR1-Fab/F(ab')2 and control-Fab/F(ab')2 were injected i.v. via tail vein in 100 µl PBS. Injected dose was 50 µg = 2.3-2.7 x 10⁶ cpm per mouse and three mice per sample were used. Mice were sacrificed at two different time points (24 h and 48 h) and organs were collected and counted for radioactivity for determination of tissue biodistribution of the conjugates.

Biodistribution study in FaDu xenograft tumor mice was carried out using anti-EGFR1-F(ab')2-BSH(800B)-Dex and anti-EGFR1-Fab(800B or 1200B)-BSH-Dex and boron conjugates (800B) of control-F(ab')2 and -Fab. The conjugates were radiolabeled (3H) to lysine residues of a protein. Radioactivity in tissue samples, including tumors and blood, were counted at two different time points (24h and 48h). Tissue distribution of boron conjugates (Table 8) show that boron conjugates of anti-EGFR1-Fab and -F(ab')2 accumulated into tumors but not significantly in any other organs, whereas control boron conjugates did not significantly accumulate into tumors. Control-F(ab')2-BSH(800B)-Dex can be still be found in blood circulation and in all organs at 24 h, but is cleared from circulation at 48 h. Tumor accumulation of boron conjugates of anti-EGFR1-Fab and -F(ab')2 was highest at 24 h and decreased at 48 h.

**Table 8. Biodistribution of boron conjugates in FaDu tumor mice. The results represent an average of three determinations +/- SEM except for tumors that are an average of six determinations +/- SEM. values are % of total injected dose/g organ.**

| **24 h** | **Anti-EGFR-Fab-BSH (800) - Dex** | **Anti-EGFR-Fab-BSH (1200)-Dex** | **Anti-EGFR-Fab2-BSH (1200)-Dex** | **Control-Fab-BSH (800) - Dex** | **Control-Fab2-BSH (800)** - **Dex** |
|---|---|---|---|---|---|
| **Organ** | | | | | |
| **blood** | 0.34±0.03 | 0.13±0.01 | 0.10±0.01 | 0.20±0.02 | 0.52±0.05 |
| **urine** | 2.45±0.58 | 0.94±0.06 | 0.59±0.25 | 1.95±0.38 | 3.48±0.42 |
| **liver** | 0.30±0.02 | 0.35±0.01 | 0.29±0.04 | 0.30±0.04 | 0.38±0.05 |
| **kidney** | 0.29±0.01 | 0.21±0.02 | 0.15±0.02 | 0.29±0.02 | 0.44±0.05 |
| **lung** | 0.15±0.01 | 0.11±0.01 | 0.09±0.02 | 0.18±0.01 | 0.32±0.04 |
| **muscle** | 0.15±0.01 | 0.16±0.02 | 0.11±0.01 | 0.19±0.01 | 0.24±0.03 |
| **skin** | 0.20±0.02 | 0.21±0.04 | 0.16±0.01 | 0.23±0.04 | 0.53±0.09 |
| **tumor** | 1.44±0.34 | 0.93±0.23 | 0.73±0.10 | 0.41±0.06 | 0.86±0.13 |
| | | | | | |

| **48 h** | **Anti-EGFR-Fab-BSH (800) - Dex** | **Anti-EGFR-Fab-BSH (1200) - Dex** | **Anti-EGFR-Fab2-BSH (1200) - Dex** | **Control-Fab-BSH (800) - Dex** | **Control-Fab2-BSH (800) - Dex** |
|---|---|---|---|---|---|
| **Organ** | | | | | |
| **blood** | 0.14±0.04 | 0.12±0.01 | 0.08±0.01 | 0.13±0.01 | 0.22±0.04 |
| **urine** | 0.77±0.07 | 0.33±0.05 | 0.42±0.08 | 0.66±0.09 | 1.05±0.15 |
| **liver** | 0.17±0.03 | 0.14±0.03 | 0.18±0.04 | 0.16±0.01 | 0.15±0.03 |
| **kidney** | 0.14±0.01 | 0.12±0.02 | 0.12±0.02 | 0.17±0.01 | 0.17±0.02 |
| **lung** | 0.09±0.01 | 0.08±0.02 | 0.07±0.01 | 0.11±0.01 | 0.13±0.01 |
| **muscle** | 0.12±0.01 | 0.11±0.03 | 0.10±0.01 | 0.13±0.01 | 0.13±0.02 |
| **skin** | 0.11±0.01 | 0.08±0.02 | 0.09±0.01 | 0.13±0.01 | 0.16±0.01 |
| **tumor** | 0.70±0.11 | 0.39±0.13 | 0.31±0.04 | 0.19±0.02 | 0.24±0.04 |

Tumor vs. blood distribution of boron conjugates in FaDu xenograft mice was calculated at 24 h and 48 h (Table 9). Tumor/blood ratio was approximately 7 for anti-EGFR1-Fab and - F(ab')2 conjugates with 1200 borons at 24 h, and the ratio decreased to 3-4 at 48 h suggesting that the labeled protein is degraded and is secreted out of the cells. Tumor/blood ratio of anti-EGFR1-Fab conjugate with 800 borons was approximately 4-5 at both time points. The ratio of control conjugates remained at a constant level (approximately 1-2).

**Table 9. Tumor/blood distribution of boron conjugates in FaDu tumor mice. The results are based on an average of three determinations for blood samples and an average of six determinations for tumors (2 tumors per mouse) +/- S.D.**

| **Boron conjugate** | **24h** | **48h** |
|---|---|---|
| **anti-EGFR-Fab-BSH(800)-dex** | 4.4 ± 2.2 | 5.5 ± 1.5 |
| **anti-EGFR-Fab-BSH(1200)-dex** | 6.9 ± 2.8 | 3.4 ± 2.0 |
| **anti-EGFR-Fab2-BSH(1200)-dex** | 7.6 ± 1.7 | 4.2 ± 1.1 |
| **control-Fab-BSH (800) -dex** | 1.8 ± 0.5 | 1.5 ± 0.2 |
| **control-Fab2-BSH (800) -dex** | 1.7 ± 0.4 | 1.2 ± 0.4 |

### EXAMPLE 9. Quantitation of boron in BSH-Dextran by inductively coupled plasma mass spectrometry (ICP-MS) (mol boron per mol BSH-Dextran)

The boron load of BSH-dextran was estimated from proton-NMR spectrum of BSH-dextran (Figure 1) and ICP-MS was used to quantitate the amount of boron in the samples. The BSH-Dextran sample analyzed in this example was estimated to contain about 1200 borons based on NMR analysis. Approximately 2.1 µg (0.0228 nmol) of BSH-Dextran (average MW 92 kDa) was liquefied with microwave-assisted wet ashing and analyzed by ICP-MS essentially as described in Laakso et al., 2001, Clinical Chemistry 47, 1796-1803. Different dilutions of the sample were analyzed by ICP-MS and the background boron was subtracted from the samples. The results representing an average of 7 determinations indicate that the sample contains approximately 0.341 µg (31.5 nmol) of boron atoms, or one mole of the BSH-Dextran contain 1381 moles of boron atoms.

### EXAMPLE 10. In vivo experiments and boron quantitation

Female adult mice of the same age (Charles River Crl:Athymic nude Foxn1nu) were used. 2.3 million HSC-2 or 5 million FaDu cells in 150 µl in EME-media and 50 % Matrigel were inoculated to the right flank of nude mice. The dosing was given when the tumor was grown to at least 6 mm diameter in size (6 - 10 mm) corresponding roughly to tumor volume of 100-500 mm³. Anti-EGFR-Fab-BSH(1200)-dex or anti-EGFR-F(ab')2-BSH(1200)-dex (both non-labeled) conjugates were injected i.v. via tail vein in 100 µl PBS. Injected dose was 50 µg or 250 µg per mouse and three mice per sample were used. Mice were sacrificed at 24 h and 48 h and organs were collected for boron determination.

Tissue samples (including blood) were digested in closed teflon vessels in a microwave oven (Milestone, ETHOS 1200). The digestion temperature was 200 C and duration of the digestion was 50 min. Acid used in the digestions was HNO₃ (6,0 ml, E. Merck, Suprapur). After cooling the resultant solution was diluted to 25 ml with Milli-Q water. The digested samples were diluted further (1:10 or 1:50) with 1 % HNO₃ for ICP-MS analysis. The internal standard beryllium was added to the sample to gain the final concentration, 10 ppb of Be, in the samples. Standard solutions with concentrations of 1, 5, 10 and 20 µg/L for analyses were diluted from Spectrascan's single element standard solution (1000 ug/ml boron as H₃BO₃ in H₂O). Control sample for analysis was prepared from multielemental standard solution by SPEX (CLMS-4). Analyses were performed with the high resolution sector field inductively coupled plasma mass spectrometer (HR-ICP-MS, Element2, Thermo Scientific). The concentration of boron in diluted samples was defined from the peaks of 10B and 11B with both low resolution (R ≈ 300) and medium resolution (R ≈ 4000) mode. Between the samples the samples introduction system was washed first with 5 % HNO₃ and then with 1 % HNO₃ to exclude the memory effect typical for boron.

Initial boron analysis of two HSC-2 tumor mice at 24h indicated that boron tumor per muscle ratios were 5.3 and 6.3.

The muscle was used as a control tissue instead of blood because initial boron measurements from blood were inconclusive or beyond detection limit.

### EXAMPLE 11. Allylation of dextran 10

20 mg dextran 10 was dissolved in 200 µl of 0.6 M NaOH. 7.5 µl of allyl bromide was added. The reaction was allowed to proceed for 3 hours at 60°C. The reaction mixture was then neutralized with 1 M acetic acid and the product was isolated by precipitation with 10 volumes of cold acetone. Precipitate was collected by centrifugation and washed twice with acetone. The product was analyzed by NMR which implied that the allylation level was 22%.

### EXAMPLE 12. Cysteamine addition to allyl dextran 10

10 mg of allyl dextran 10kD (example 11), 5 mg ammonium persulfate and 5 mg cysteamine were dissolved in 0.25 ml MQ water. The reaction was allowed to proceed for 2 hours at 50°C. The polymer is purified with Amicon Ultra 3K concentrators (Millipore) by several additions of 0.1 M ammonium bicarbonate. The product was analyzed by NMR which implied that all allyl groups in dextran had reacted with cysteamine (Scheme 4).

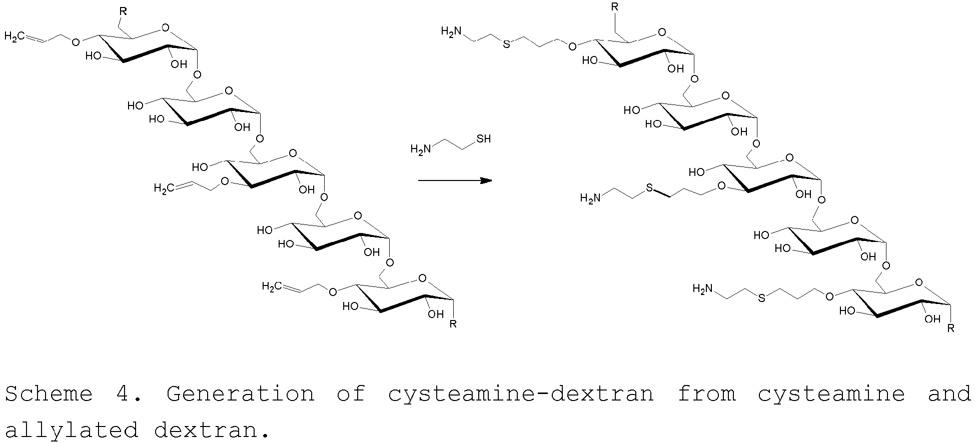

### EXAMPLE 13. NHS-PEG₄-azide addition to cysteamine-dextran

0.5 mg of cysteamine-dextran (600 nmol amino groups) was dissolved into 50 µl MQ water and 300 nmol of NHS-PEG₄-azide (Thermo Scientific) in DMSO was added. The reaction was allowed to proceed overnight at room temperature. The product (azido-dextran, Scheme 5) was purified with Amicon Ultra 3K concentrators (Millipore) by several additions of PBS.

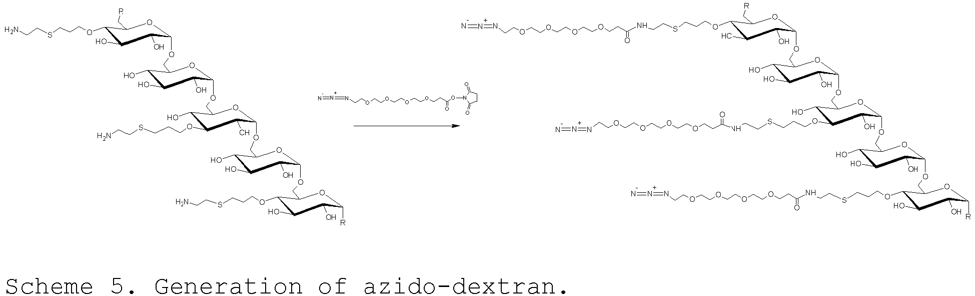

### EXAMPLE 14. Addition of DBCO units to Fab

Omalizumab Fab fragments were reacted with dibenzylcyclooctyne-NHS ester (DBCO-NHS, Jena Bioscience). 2 nmol of Fab fragment was dissolved into 50 µl PBS and 20 nmol DBCO-NHS (4 nmol/µl DMSO) was added. The reaction was allowed to proceed for 4 hours at room temperature. The DBCO-Fabs were purified with Amicon Ultra 10K concentrators (Millipore) by several additions of PBS.

The reaction was verified by analyzing the DBCO-Fabs using MALDI-TOF MS. The DBCO-Fabs were first denatured with guanidine-HCl and the disulphide bonds were reduced with dithiotreitol. Reduced Fab chains were then purified with a small Poros R1 column prepared in a pipette tip and analyzed by MALDI-TOF. MALDI analysis implied that on average 1.5 DBCO units were attached to Fab fragments.

### EXAMPLE 15. Conjugation of DBCO-Fab and azido-dextran

10 µg (ca. 0.2 nmol) of DBCO-Fab (Example 14) and i) 2, ii) 10, iii) 20 and iv) 50 nmol of azido-dextran (Example 13) were allowed to react in PBS in an overnight reaction at room temperature. The samples were divided to reduced and non-reduced SDS-PAGE gels (pre-casted 4-20%, Pierce and Mini-PROTEAN TGX, Bio-Rad, respectively). The gels were stained with Imperial Protein Stain (Pierce). This analysis showed that 2 nmol of azido-dextran was enough to modify DBCO-Fab completely to dextran-Fab, as revealed by the disappearance of Fab bands in the PAGE gels.

### EXAMPLE 16. Addition of lactose to cysteamine-dextran

5 mg of cysteamine dextran (Example 12), 300 µmol of lactose, and 47.8 mg of NaCNBH₃ were dissolved in 1.5 ml of 0.2 M ammonium bicarbonate. The mixture was incubated for 64 h at 50°C. Low-molecular weight components were eliminated by ultrafiltration on Amicon centrifugal filters (10 K cut-off). The Lac-dextran (Scheme 6) thus obtained was analyzed by 1H-NMR, which indicated that ca. 10% of the dextran glucose units carried a lactosyl unit.

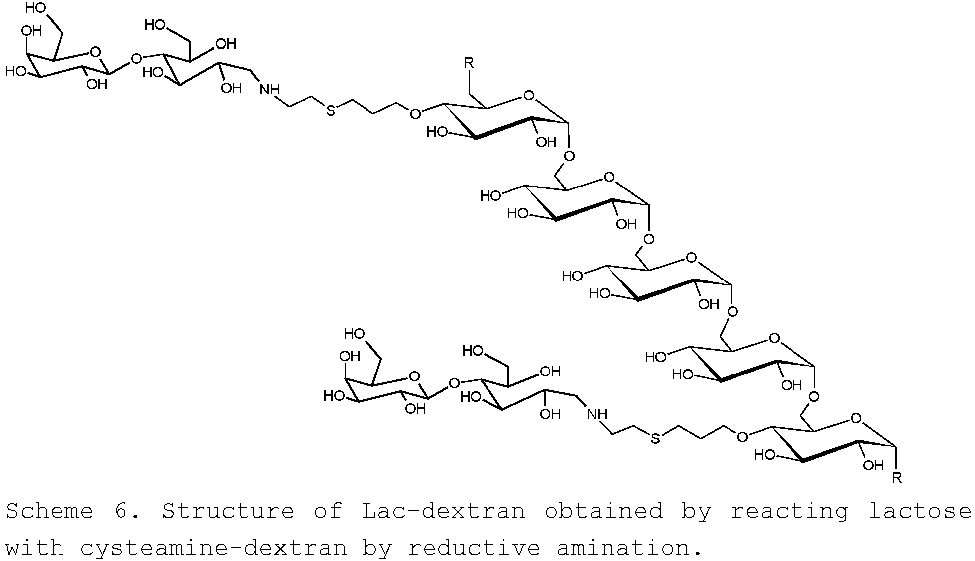

### EXAMPLE 17. Addition of DM1-DBCO to azido-dextran

~3,9 µmol DM1-S-CH₂COOH, 3.5 molar excess of DBCO-NH₂ (Sigma) in DMF (200 µl) and 26 mg (95 µmol) DMT-MM in DMF (500 µl) were stirred at RT for overnight. The crude reaction mixture was analysed by MALDI-TOF mass spectra using 2,5-dihydroxybenzoic acid matrix, showing expected mass for DM1-DBCO (m/z 1076 [M+Na]). DM1-DBCO was purified by reversed-phase chromatography using Äkta purifier (GE Healthcare) HPLC instrument with Gemini 5 µm NX- C18 reverse phase column (21.1 x 250 mm, 110 Å, AXIA (Phenomenex)) eluted with ACN gradient in aqueous ammonium acetate.

Azido-dextran 10 kD (16,7 nmol, 200 nmol azido-groups; Example 13) was dissolved in PBS (9 µl). DM1-DBCO (200 nmol) in DMSO (5 µl) was added and reaction was allowed to proceed overnight at room temperature. The DM1-DBCO-azido-dextran product (Scheme 7) was purified by HPLC with Superdex 75 gel filtration column using 100 mM ammonium hydrogen carbonate for elution.

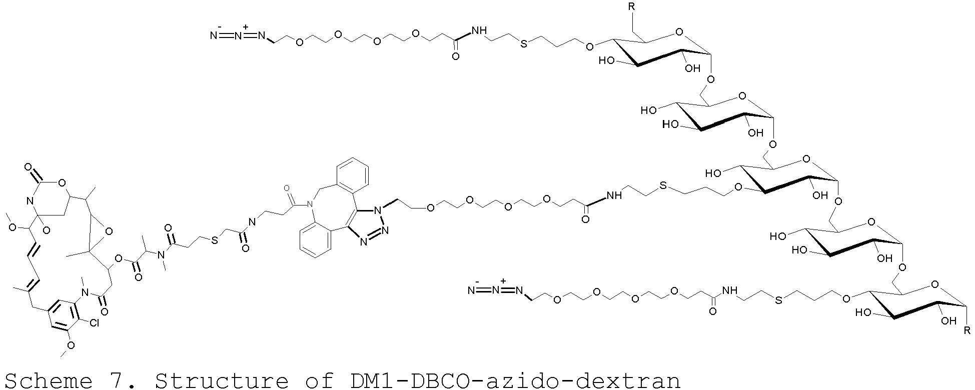

### EXAMPLE 18. Preparation of DBCO-ValCit-PABC-(NH-Gal)Modo

Synthesis of 1,2;3,4-di-O-isopropylidene-6-O-tosyl-α-D-galactopyranose (Scheme 8.2): 0.39g (1.5mmol) of (Scheme 8.1) was dissolved in 5ml of dry pyridine under an argon atmosphere. The reaction mixture was cooled on an ice bath and 0.88g (3.1 equiv.) of TsCl was added. The reaction was slowly warmed to RT and stirred overnight. After 22 hours the reaction was diluted with 30ml of CH₂Cl₂ and washed with 30ml of ice-cold water. The organic phase was washed with 20ml of 10% (w/v) aqueous CuSO₄-solution, 20ml of saturated NaHCO₃-solution and 20ml H₂O. The organic phase was separated, dried over Na₂SO₄, filtered and concentrated. The crude product was purified by column chromatography (Hexane:EtOAc 1:1) to give (Scheme 8.2) as a yellowish oil (0.49g, 81%). TLC: R*_{f}* = 0.74 (Hexane:EtOAc 1:1). ¹H NMR (600 MHz, CDCl₃, 22°C): δ = 7.81-7.32 (m, 4 H, CH₃C₆H₄SO₂), 5.45 (d, 1 H, *J*_{1,2} = 4.9 Hz, H- 1), 4.59 (dd, 1 H, *J*_{3,2} = 2.5, *J*_{3,4} = 7.9 Hz, H-3), 4.29 (dd, 1 H, H-2), 4.22-4.18 (m, 2 H, H-6a, H-4), 4.09 (dd, 1 H, *J*_{6b,5} = 6.9, J_{6b,6a} = -10.3 Hz, H-6b), 4.05 (ddd, 1 H, *J*_{5,4} = 1.9, J_{5,6}, = 6.2 Hz, H-5), 2.44 (s, 3 H, CH₃C₆H₄SO₂), 1.50, 1.34, 1.31 and 1.28 (each s, each 3 H, O₂C(CH₃)₂) ppm.

Synthesis of 1,2;3,4-di-O-isopropylidene-6-deoxy-6-azido-α-D-galactopyranose (Scheme 8.3). To a solution containing 1.5g (3.7mmol) of (Scheme 8.2) in 20ml dry DMF (under an argon atmosphere) was added 1.7g (7 equiv.) NaN₃ and the resulting mixture was stirred at 120°C overnight. After 18 hours, the reaction mixture was brought to RT, diluted with 20ml CHCl₃, filtered and concentrated. The crude product was purified by column chromatography (Hexane:EtOAc 3:1) to give (Scheme 8.3) as a colorless oil (0.7g, 68%). TLC: R*_{f}* = 0.52 (Hexane:EtOAc 3:1). ¹H NMR (600 MHz, CDCl₃, 22°C): δ = 5.55 (d, 1 H, *J*_{1,2} = 5.1 Hz, H-1), 4.63 (dd, 1 H, *J*_{3,2} = 2.5, *J*_{3,4} = 8.1 Hz, H-3), 4.33 (dd, 1H, H-2), 4.19 (dd, 1 H, *J*_{4,5} = 2.0 Hz, H-4), 3.92 (ddd, 1 H, *J*_{5,6b} = 5.3, *J*_{5,6a} = 7.8 Hz, H-5), 3.51 (dd, 1 H, *J*_{6a,6b} = -12.9 Hz, H-6a), 3.36 (dd, 1 H, H-6b), 1.55, 1.46, 1.35 and 1.34 (each s, each 3 H, O₂C(CH₃)₂) ppm.

Synthesis of 6-azido-6-deoxy-D-galactose (Scheme 1.4). 80mg (0.3mmol) of (Scheme 8.3) was dissolved in 3ml 60% TFA and the resulting mixture was stirred at 50°C for 1 hour. The mixture was then diluted with water and concentrated to give (Scheme 8.4) as a colorless oil (60mg, quantitative, furanose: pyranose 3:97, alpha_{pyranose}:beta_{pyranose} 35:65). Selected NMR-data: ¹H NMR (600 MHz, D₂O, 22°C): δ = 5.28 (d, 1 H, J_{1,2} = 4.7 Hz, H-1_{furanose}), 5.26 (d, 1 H, J_{1,2} = 3.9 Hz, H-1α_{pyranose}), 5.22 (d, 1 H, J_{1,2} = 3.4 Hz, H-1_{furanose}), 4.60 (d, 1 H, J_{1,2} = 7.8 Hz, H-1β_{pyranose}).

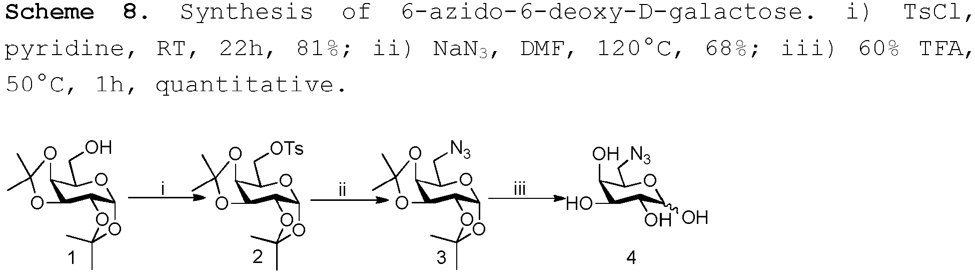

N-(6-azido-6-deoxy-D-galactosyl)-monomethyldolastatin (N-(6-azido-6-deoxy-D-galactosyl)-Modo) was prepared by adding sodium cyanoborohydride (160µmol) and 6-azido-6-deoxy-D-galactose (95µmol) to the solution of momomethyldolastatin 10 (2.5µmol) in DMSO (0.6ml). The mixture was stirred at 60°C for three days.

The azido group was reduced to amino group by catalytic hydrogenation as described in Example 23 to obtain N-(6-amino-6-deoxy-D-galactosyl)-Modo that was purified by reversed-phase chromatography using identical procedure to that of the azido-derivatised counterpart.

20 µmol of N-(6-amino-6-deoxy-D-galactosyl)-Modo, 31 mg of Fmoc-Val-Cit-PABC-pnp (Concortis Biosystems, Inc.), 1.4 mg HoBt, 16 µl DIPEA and 484 µl DMF were stirred at RT for 18h. The crude reaction mixture was analysed by MALDI-TOF mass spectra using 2,5-dihydroxybenzoic acid matrix, showing expected mass for Fmoc-ValCit-PABC-(NH-Gal)Modo (m/z 1583.8 [M+Na]⁺). Fmoc was removed by adding 500 µl of diethylamine and by stirring at room temperature for 18h. MALDI-TOF mass analysis using 2,5-dihydroxybenzoic acid matrix showed the generation of expected deprotected product (m/z 1361.7, [M+Na]⁺).

Ca. 3.7 µmol ValCit-PABC-(NH-Gal)Modo, 38 µmol DBCO-NHS ester (Jena Bioscience GmbH), and 3.5 µl DIPEA were dissolved in 400 µl DMF and stirred at RT for three hours. The crude reaction mixture was analysed by MALDI-TOF mass spectra using 2,5-dihydroxybenzoic acid matrix, showing expected mass for DBCO-ValCit-PABC-(NH-Gal)Modo (m/z 1648.9, [M+Na]⁺)

DBCO-ValCit-PABC-(NH-Gal)Modo (Scheme 9) was purified by Äkta purifier (GE Healthcare) HPLC instrument with Gemini 5 µm NX-AXIA-C18 reversed-phase column (21.2 x 250 mm, 110 Å (Phenomenex)) eluted with ACN gradient in aqueous ammonium acetate.

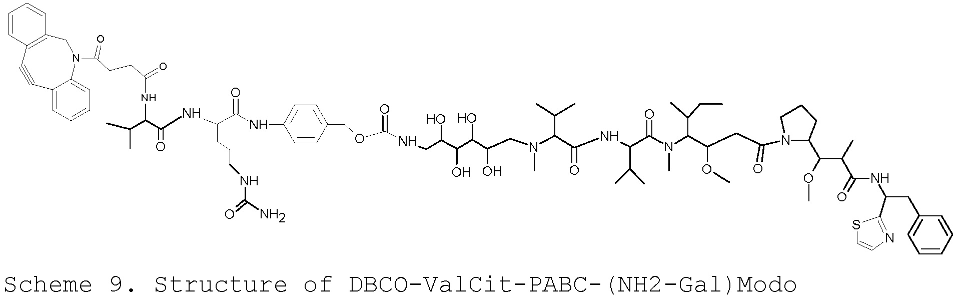

### EXAMPLE 19. Preparation of BSH-hyaluronic acid

50 mg hyaluronic acid 64 kD (Lifecore) was dissolved in 0.5 ml of 0.6 M NaOH. 63 µl of allyl bromide (Sigma) was added, and the reaction was allowed to proceed for 3 h at 60°C. The reaction mixture was then neutralized with 1M acetic acid and the product was isolated by precipitation with 10 volumes of cold acetone (-20°C). Precipitate was collected by centrifugation and washed twice with acetone. The allylated hyaluronic acid was subjected to 1H-NMR analysis, which showed that the level of allylation was ca. 50%.

20 mg of allylated hyaluronic acid was then dissolved in 400 µl water and 20 mg ammonium persulfate and 20 mg BSH were added. The reaction was allowed to proceed for 2h at 50°C, and the reacted polysaccharide was isolated with ultrafiltration using centrifugal filter (Amicon, 30K cut-off). BSH-hyaluronic acid thus obtained was subjected to 1H-NMR analysis, which showed that the product carried ca. 150 BSH units.

### EXAMPLE 20. Preparation of BSH-hydroxyethyl starch

50 mg hydroxyethyl starch (Sigma; product H6382) was dissolved in 0.5 ml of 0.6 M NaOH. 63 µl of allyl bromide (Sigma) was added, and the reaction was allowed to proceed for 3 h at 60°C. The reaction mixture was then neutralized with 1M acetic acid and the product was isolated by precipitation with 10 volumes of cold acetone (-20°C). Precipitate was collected by centrifugation and washed twice with acetone. The allylated hydroxyethyl starch was subjected to 1H-NMR analysis, which showed that the level of allylation was ca. 55%.

20 mg of allylated hydroxyethyl starch was then dissolved in 400 µl water and 20 mg ammonium persulfate and 20 mg BSH were added. The reaction was allowed to proceed for 2h at 50°C, and the reacted polysaccharide was isolated with ultrafiltration using centrifugal filter (Amicon, 30K cut-off). BSH-hydroxyethyl starch thus obtained was subjected to 1H-NMR analysis, which showed that the product carried about 14 BSH units per 100 glucose units.

### EXAMPLE 21. Conjugation of DBCO-ValCit-PABC-(NH-Gal)Modo to azido-dextran

Azido-dextran 10 kD (Example 13) is dissolved in PBS and DBCO-ValCit-PABC-(NH-Gal)Modo (Example 18)in DMSO is added. The reaction is allowed to proceed overnight at room temperature. The DBCO-ValCit-PABC-(NH-Gal)Modo-azido-dextran product is purified by HPLC with Superdex 75 gel filtration column using 100 mM ammonium hydrogen carbonate for elution.

### EXAMPLE 22. Synthesis of CMP-9-deoxy-9-azido-NeuNAc

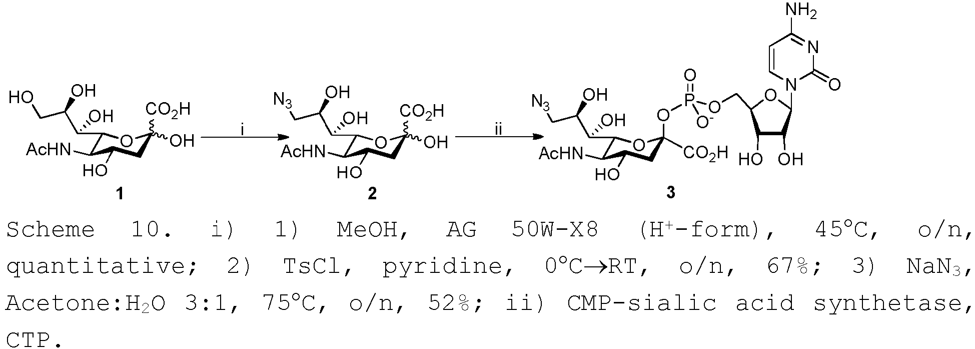

5-acetamido-9-azido-3,5,9-trideoxy-D-glycero-D-galacto-2-nonulosonic acid (2): To a solution containing 63 mg of 1 (0.2 mmol) in 5 ml dry MeOH (under argon) was added 127 mg AG 50W-×8 (2 weight equiv.) and the resulting mixture was stirred at 45 °C o/n. The mixture was then filtered and concentrated to give methyl *N*-acetyl neuraminate as a white solid (65 mg, quantitative). TLC: *R_{f}* = 0.43 (DCM:MeOH 3:1)

157 mg of methyl *N*-acetyl neuraminate (0.49 mmol) was dissolved in 5 ml of dry pyridine (under argon) and the reaction mixture was cooled to 0°C. 135 mg TsCl (0.7 mmol, 1.4 equiv.) was added and the reaction mixture was slowly warmed to RT and left to stir o/n. After 23 hours 134 mg TsCl (0.7 mmol, 1.4 equiv.) was added to the reaction mixture and it was stirred for an additional 2 hours at RT. The mixture was then cooled to 0°C and the reaction quenched with MeOH. The mixture was concentrated and the crude product was purified by column chromatography (MeOH:DCM 1:9) to give methyl 9-*O*-tosyl-*N*-acetyl-neuraminate as a yellowish oil (159 mg, 67%). TLC: *R_{f}* = 0.29 (DCM:MeOH 9:1). ¹H NMR (600MHz, CD₃OD, 22°C): δ Selected NMR-data; 7.80-7.43 (m, 4 H, CH₃C₆*H*₄SO₂), 4.28 (dd, 1 H, *J* = 2.2, 10.1 Hz), 4.06-3.99 (m, 2 H), 3.93 (dd, 1 H, *J* = 1.5, 10.6 Hz), 3.85 (ddd, 1 H, *J* = 2.0, 5.7, 8.5 Hz), 3.77 (s, 3 H, CO₂C*H*₃), 3.43 (dd, 1 H, *J* = 1.5, 9.0 Hz), 2.46 (s, 3 H, C*H*₃C₆H₄SO₂), 2.19 (dd, 1 H, *J* = 4.9, 12.9 Hz, H-3eq), 2.00 (s, 3 H, NHCOC*H*₃), 1.86 (dd, 1 H, *J* = 11.5, 12.9 Hz, H-3ax). HRMS: calcd. for C₁₉H₂₇O₁₁NNaS [M+Na]⁺ 500.12; found 500.20.

110 mg of methyl 9-*O*-tosyl-*N*-acetyl-neuraminate (0.23 mmol) was dissolved in 2 ml acetone:H₂O 3:1 and 70 mg NaN₃ (1.1 mmol, 4.3 equiv.) was added. The resulting mixture was heated to 75°C and stirred o/n. The reaction mixture was then concentrated and the crude product purified by gel filtration chromatography to give **2** as a yellowish foam (40 mg, 52%). Selected NMR-data; ¹H NMR (600MHz, D₂O, 22°C): δ 4.03 (ddd, 1 H, *J* = 5.1, 10.1, 10.3 Hz), 3.99 (dd, 1 H, *J* = 0.9, 10.6 Hz), 3.94-3.89 (m, 2 H), 3.61 (dd, 1 H, *J* = 2.8, 13.1 Hz), 3.53 (ap d, 1 H, *J* = 9.4 Hz), 3.49 (dd, 1 H, *J* = 6.0, 13.1 Hz), 2.22 (dd, 1 H, *J* = 4.9, 12.9 Hz, H-3eq), 2.07 (s, 3 H, NHCOC*H*₃), 1.83 (dd, 1 H, *J* = 11.7, 12.9 Hz, H-3ax). HRMS: calcd. for C₁₁H₁₈O₈N₄Na [M+Na]⁺ 357.10; found 357.12; calcd. for C₁₁H₁₇O₈N₄Na₂ [M+2Na-H]⁺ 379.08; found 379.10.

Cytidine-5'-monophospho-5-acetamido-9-azido-3,5,9-trideoxy-D-glycero-D-galacto-2-nonulosonic acid (CMP-9'-azido-NeuAc) (3): Enzymatic synthesis of CMP-9'-azido-NeuAc was carried out in 2 ml of 100 mM Tris-HCl buffer pH 8.5 containing 20 mM MgCl₂, 15 mM CTP, 10 mg (15 mM) of 9'-azido-NeuAc and 100 mU of CMP-sialic acid synthetase (Sigma Aldrich). All reagents except 9'-azido-NeuAc were of commercial origin. Reaction was allowed to proceed for 2.5 hours at +37°C. After 1 hour CTP was added to reach final CTP- concentration of 30 mM and pH was adjusted to 8.5 with NaOH. The reaction was monitored at time points 1 h and 2.5 h by taking samples to MALDI-TOF MS analysis. MALDI-TOF MS analyses were performed using 2',4',6'-trihydroxyacetophenone (THAP) as the matrix in reflector negative ion mode with Bruker Ultraflex III instrument (Bruker Daltonics, Germany). After 2.5 hours the enzyme was removed from the mixture by running the reaction mixture through Bond Elute C₁₈-column (Varian Inc.). CMP-9'-azido-NeuAc-sample eluted from Bond Elute-column was purified by gel filtration chromatography with Superdex peptide column (GE Healthcare) using 0.1 M ammonium bicarbonate as eluent. Two consecutive chromatographic runs resulted in sample containing mainly CMP-9'-azido-NeuAc with minor proportion of CTP as exemplified by MALDI-spectrum in Fig 2: CMP-9'-azido-NeuAc, m/z 637; CTP, m/z 479. Final yield of CMP-9'-azido-NeuAc based on absorbance at 280 nm (against CTP-standard) was 5.7 mg.

### EXAMPLE 23. Synthesis of 9-modified NeuNAc

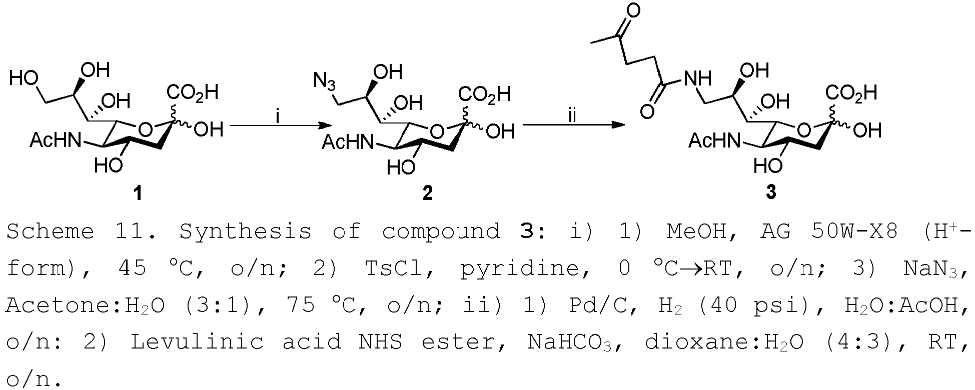

### Levulinic acid NHS ester

0.3 ml (2.93 mmol) Levulinic acid was dissolved in 7 ml dry DMF (under argon atmosphere) and 0.84 g (4.4 mmol, 1.5 equiv.) EDC×HCl and 0.41 g (3.5 mmol, 1.2 equiv.) NHS were added. The resulting mixture was stirred o/n at RT, then diluted with 20 ml EtOAc and washed with 20 ml of a satd. ammonium chloride solution, 20 ml H₂O and 20 ml brine. The organic phase was separated and dried with Na₂SO₄, filtered and concentrated to give the crude product as a white powder (0.45 g, 71 %). The crude product was utilized as such in the following step.

### 5-acetamido-9-azido-3,5,9-trideoxy-D-glycero-D-galacto-2-nonulosonic acid (2)

To a solution containing 63 mg of **1** (0.2 mmol) in 5 ml dry MeOH (under argon) was added 127 mg AG 50W×8 (H⁺-form, 2 weight equiv.) and the resulting mixture was stirred at 45 °C o/n. The mixture was then filtered and concentrated to give methyl *N*-acetyl neuraminate as a white solid (65 mg, quantitative). TLC: *R_{f}* = 0.43 (DCM:MeOH 3:1)

157 mg of methyl *N*-acetyl neuraminate (0.49 mmol) was dissolved in 5 ml of dry pyridine (under argon) and the reaction mixture was cooled to 0°C. 135 mg TsCl (0.7 mmol, 1.4 equiv.) was added and the reaction mixture was slowly warmed to RT and left to stir o/n. After 23 hours 134 mg TsCl (0.7 mmol, 1.4 equiv.) was added to the reaction mixture and it was stirred for an additional 2 hours at RT. The mixture was then cooled to 0 °C and the reaction quenched with MeOH. The mixture was concentrated and the crude product was purified by column chromatography (MeOH:DCM 1:9) to give methyl 9-*O*-tosyl-*N*-acetyl-neuraminate as a yellowish oil (159 mg, 67%). TLC: R*_{f}* = 0.29 (DCM:MeOH 9:1). Selected NMR-data; ¹H NMR (600MHz, CD₃OD, 22°C): δ 7.80-7.43 (m, 4 H, CH₃C₆*H*₄SO₂), 4.28 (dd, 1 H, *J* = 2.2, 10.1 Hz), 4.06-3.99 (m, 2 H), 3.93 (dd, 1 H, *J* = 1.5, 10.6 Hz), 3.85 (ddd, 1 H, *J* = 2.0, 5.7, 8.5 Hz), 3.77 (s, 3 H, CO₂CH₃), 3.43 (dd, 1 H, *J* = 1.5, 9.0 Hz), 2.46 (s, 3 H, C*H*₃C₆H₄SO₂), 2.19 (dd, 1 H, *J* = 4.9, 12.9 Hz, H-3eq), 2.00 (s, 3 H, NHCOC*H*₃) and 1.86 (dd, 1 H, *J* = 11.5, 12.9 Hz, H-3ax) ppm. HRMS: calcd. for C₁₉H₂₇O₁₁NNaS [M+Na]⁺ 500.12; found 500.20.

110 mg of methyl 9-*O*-tosyl-*N*-acetyl-neuraminate (0.23 mmol) was dissolved in 2 ml acetone:H₂O (3:1) and 70 mg NaN₃ (1.1 mmol, 4.3 equiv.) was added. The resulting mixture was heated to 75 °C and stirred o/n. The reaction mixture was then concentrated and the crude product purified by gel filtration chromatography to give **2** as a yellowish foam (40 mg, 52%). Selected NMR-data; ¹H NMR (600MHz, D₂O, 22°C): δ 4.03 (ddd, 1 H, *J* = 5.1, 10.1, 10.3 Hz), 3.99 (dd, 1 H, *J* = 0.9, 10.6 Hz), 3.94-3.89 (m, 2 H), 3.61 (dd, 1 H, *J* = 2.8, 13.1 Hz), 3.53 (ap d, 1 H, *J* = 9.4 Hz), 3.49 (dd, 1 H, *J* = 6.0, 13.1 Hz), 2.22 (dd, 1 H, *J* = 4.9, 12.9 Hz, H-3eq), 2.07 (s, 3 H, NHCOC*H*₃) and 1.83 (dd, 1 H, *J* = 11.7, 12.9 Hz, H-3ax) ppm. HRMS: calcd. for C₁₁H₁₈O₈N₄Na [M+Na]⁺ 357.10; found 357.12; calcd. for C₁₁H₁₇O₈N₄Na₂ [M+2Na-H]⁺ 379.08; found 379.10.

### 5-Acetamido-3,5,9-trideoxy-9-[(1,4-dioxopentyl)amino]-D-glycero-D-galacto-2-nonulosonic acid (3)

26 mg (0.08 mmol) of **2** was dissolved in 2.5 ml H₂O and the pH was adjusted to 1/3 with AcOH. 7.9 mg (0.3 weight equiv.) Pd/C (10 % Pd) was added and the resulting mixture was placed inside a hydrogenation reactor. The hydrogen pressure was set to 40 psi (~ 2.7 bar) and the mixture was stirred o/n, then filtered through celite and concentrated to give the crude product 5-acetamido-3,5,9-trideoxy-9-amino-D-glycero-D-galacto-2-nonulosonic acid as a yellowish oil. This product was utilized as such in the following step.

22 mg (0.07 mmol) of 5-acetamido-3,5,9-trideoxy-9-amino-D-glycero-D-galacto-2-nonulosonic acid was dissolved in 3 ml H₂O and the pH was adjusted to 8/9 with a satd. NaHCO₃-solution. 23 mg (0.11 mmol, 1.5 equiv.) levulinic acid NHS ester was dissolved in 4 ml dioxane and slowly added to the solution containing the sialic acid in H₂O. The reaction mixture was then stirred at RT o/n in the dark and concentrated. The crude product was purified by gel filtration chromatography to give the title compound. HRMS: calcd. for C₁₆H₂₆O₁₀N₂Na [M+Na]⁺ 429.15; found 429.19; calcd. for C₁₆H₂₅O₁₀N₂Na₂ [M+2Na-H]⁺ 451.13; found 451.17.

### Synthesis of other 9-modified NeuNAc analogues

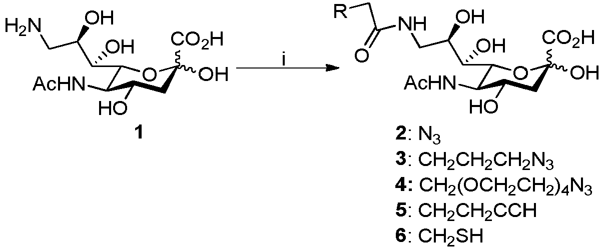

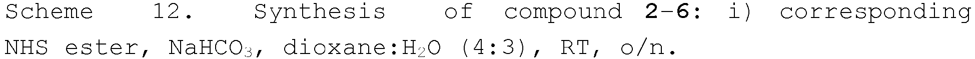

### General procedure for synthesis of carboxylic acid NHS esters

The corresponding carboxylic acid was dissolved in 2 ml dry DMF /mmol acid (under argon atmosphere) and 1.5 equiv. EDC×HCl and 1.2 equiv. NHS were added. The resulting mixture was stirred o/n at RT, then diluted with 7 ml EtOAc/mmol acid and washed with 7 ml of a satd. ammonium chloride solution/mmol acid, 7 ml H₂O/mmol acid and 7 ml brine/mmol acid. The organic phase was separated and dried with Na₂SO₄, filtered and concentrated to give the crude product. The crude product was utilized as such in the following step.

### General procedure for synthesis of 9-amido modified NeuNAc

5-acetamido-3,5,9-trideoxy-9-amino-D-glycero-D-galacto-2-nonulosonic acid was dissolved in 2 ml H₂O/30 mg **1** and the pH was adjusted to 8/9 with a satd. NaHCO₃-solution. 1.5 equiv. of the corresponding carboxylic acid NHS ester was dissolved in 2 ml dioxane/30 mg of NHS ester and slowly added to the solution containing the sialic acid in H₂O. The reaction mixture was then stirred at RT o/n in the dark and concentrated. The crude product was purified by gel filtration chromatography to give the corresponding 9-amido NeuNAc.

### Hexynoic acid NHS ester

The synthesis commenced according to the general procedure for synthesis of carboxylic acid NHS esters to give the title compound as a yellowish oil in quantitative yield.

### 5-azidopentanoic acid NHS ester

The synthesis commenced according to the general procedure for synthesis of carboxylic acid NHS esters to give the title compound as a colorless oil in quantitative yield.

### Compound 2

The synthesis commenced according to the general procedure for synthesis of 9-amido modified NeuNAc. HRMS: calcd. for C₁₃H₂₁O₉N₅Na [M+Na]⁺ 414.12; found 413.97; calcd. for C₁₃H₂₀O₉N₅Na₂ [M+2Na-H]⁺ 436.11; found 435.97. NMR in agreement with the data published by J. C. Paulson et. al. in Angew. Chem. Int. Ed. 2012, 51, 11014.

### Compound 3

The synthesis commenced according to the general procedure for synthesis of 9-amido modified NeuNAc. Selected NMR-data; ¹H NMR (600MHz, D₂O, 22°C): δ 3.56 (dd, 1 H, *J* = 3.0, 14.1 Hz), 3.40 (dd, 1 H, *J* = 1.0, 9.0 Hz), 3.25 (dd, 1 H, *J* = 7.8, 14.1 Hz), 2.03 (s, 3 H, NHCOC*H*₃) and 1.68-1.55 (m, 4 H, NHCOCH₂C*H*₂C*H*₂CH₂N₃) ppm. HRMS: calcd. for C₁₆H₂₇O₉N₅Na [M+Na]⁺ 456.17; found 456.21; calcd. for C₁₆H₂₆O₉N₅Na₂ [M+2Na-H]⁺ 478.15; found 478.17.

### Compound 4

The synthesis commenced according to the general procedure for synthesis of 9-amido modified NeuNAc. HRMS: calcd. for C₂₂H₃₉O₁₃N₅Na [M+Na]⁺ 604.22; found 604.23; calcd. for C₂₂H₃₈O₁₃N₅Na₂ [M+2Na-H]⁺ 626.23; found 626.21.

### Compound 5

The synthesis commenced according to the general procedure for synthesis of 9-amido modified NeuNAc. Selected NMR-data; ¹H NMR (600MHz, D₂O, 22°C): δ 3.55 (dd, 1 H, *J* = 2.9, 14.2 Hz), 3.40 (dd, 1 H, *J* = 1.0, 9.1 Hz), 3.27 (dd, 1 H, *J* = 7.6, 14.2 Hz), 2.03 (s, 3 H, NHCOC*H*₃) and 1.83-1.76 (m, 2 H) ppm. HRMS: calcd. for C₁₇H₂₆O₉N₂Na [M+Na]⁺ 425.15; found 425.11; calcd. for C₁₇H₂₅O₉N₂Na₂ [M+2Na-H]⁺ 447.14; found 447.10.

### Compound 6

The synthesis commenced according to the general procedure for synthesis of 9-amido modified NeuNAc starting from **1** and SPDP (pyridyldithiol protective group is partially cleaved under the reaction conditions to give **6**). HRMS: calcd. for C₁₄H₂₄O₉N₂SNa [M+Na]⁺ 419.11; found 419.16; calcd. for C₁₄H₂₃O₉N₂SNa₂ [M+2Na-H]⁺ 441.09; found 441.13.

### Synthesis of 5-modified NeuNAc

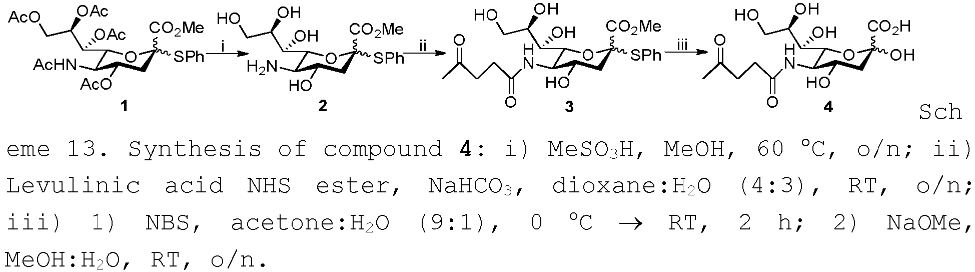

### Phenyl 5-amino-2-thio-D-neuraminic acid methyl ester (2)

96.3 mg (0.17 mmol) of **1** was dissolved in 7 ml dry MeOH (under argon atmosphere) and 0.45 ml MeSO₃H was added. The resulting mixture was stirred at 60 °C o/n and concentrated to give the crude product. This product was utilized as such in the following step. Selected analytical data; HRMS: calcd. for C₁₆H₂₄O₇NS [M+H]⁺ 374.13; found 374.15; calcd. for C₁₆H₂₃O₇NSNa₂ [M+Na]⁺ 396.11; found 396.13.

### Phenyl 5-[(1,4-dioxopentyl)amino]-2-thio-D-neuraminic acid methyl ester (3)

The crude product from the previous step (63 mg, 0.17 mmol) was dissolved in 3 ml H₂O and the pH was adjusted to 8/9 with a satd. NaHCO₃-solution. 0.1 g (0.51 mmol, 3 equiv.) of levulinic acid NHS ester dissolved in 4 ml dioxane was slowly added to the reaction mixture. The resulting mixture was stirred o/n at RT in the dark and then concentrated. The crude product was purified by column chromatography (MeOH:DCM 1:5 → 1:3) to give the title compound as a colorless oil (80 mg, quant.). TLC: R*_{f}* = 0.43 (DCM:MeOH 5:1). Selected NMR-data; ¹H NMR (600MHz, CD₃OD, 22°C): δ 7.62-7.32 (m, 5 H, arom. *H*)*,* 4.53 (dd, 1 H, *J* = 0.7, 10.6 Hz), 4.13 (m, 1 H, H-4), 3.87 (t, 1 H, *J* = 10.2 Hz), 3.82 (dd, 1 H, *J* = 2.9, 11.3 Hz), 3.78 (m, 1 H), 3.67 (dd, 1 H, , *J* = 5.5, 11.3 Hz), 3.57 (d, 1 H, 9.4 Hz), 3.50 (s, 3 H, CO₂C*H*₃) and 2.19 (s, 3 H, NHCOCH₂CH₂COC*H*₃) ppm.

HRMS: calcd. for C₂₁H₂₉O₉NSNa [M+Na]⁺ 494.15; found 494.16.

### 5-[(1,4-dioxopentyl)amino]-D-neuraminic acid (4)

80 mg (0.17 mmol) of **3** was dissolved in 5 ml acetone:H₂O( 9:1) and cooled on an ice bath. 127 mg (0.72 mmol, 4.2 equiv.) NBS was added and the resulting mixture was stirred for 2 h (0 °C → RT; TLC monitoring) and concentrated. The crude product was purified by column chromatography (MeOH:DCM 1:5 → MeOH:EtOAc 1:3) to give 5-[(1,4-dioxopentyl)amino]-D-neuraminic acid methyl ester as a colorless oil (36 mg, 56 %). TLC: R*_{f}* = 0.17 (DCM:MeOH 5:1). HRMS: calcd. for C₁₅H₂₅O₁₀NNa [M+Na]⁺ 402.14; found 402.16.

36 mg (0.096 mmol) 5-[(1,4-dioxopentyl)amino]-D-neuraminic acid methyl ester was dissolved in 4 ml dry MeOH (under argon atmosphere) and 70 µl of a 5 M solution of NaOMe in MeOH was added. A few drops of H₂O was added and the resulting mixture was left to stir o/n at RT. The reaction mixture was then neutralized with AG 50W×8 (H⁺-form), filtered and concentrated to give the crude product. The crude product was purified by gel filtration chromatography to give the title compound. HRMS: calcd. for C₁₄H₂₃O₁₀NNa [M+Na]⁺ 388.12; found 388.17; calcd. for C₁₄H₂₂O₁₀NNa₂ [M+2Na-H]⁺ 410.10; found 410.15.

### EXAMPLE 24. In vivo experiments with ¹⁴C labelled anti-EGFR1 Fab BSH-dextran

### Preparation of anti-EGFR1 Fab BSH-dextran

BSH-dextran was prepared as described in Examples 1 and 2, respectively. According to NMR analysis the BSH-dextran contained approximately 650 borons. The oxidation was made as described in Example 3 but in two batches; one with 50 mg and the other with 100 mg BSH-dextran.

Anti-EGFR1 Fab fragments were prepared by papain digestion as described in Example 5. Conjugation reactions were carried out as in Example 4 but in four batches: 1) 29 mg oxidized BSH-dextran and 10.4 mg anti-EGFR1 Fab, 2) 16.5 mg oxidized BSH-dextran and 5.9 mg anti-EGFR1 Fab, 3) 50 mg oxidized BSH-dextran and 19.8 mg anti-EGFR1 Fab, 4) 50 mg oxidized BSH-dextran and 19.7 mg anti-EGFR1 Fab yielding together 55.8 mg of anti-EGFR1 Fab. All were analyzed in SDS-PAGE as in Example 6 and samples of each were labeled with Alexa Fluor 488-NHS. Internalization assay with Alexa Fluor 488 labeled molecules was performed with HSC-2 cells as described in Example 7.

Unlabeled Fab-BSH-dextran batches were combined to yield 39 mg of Anti-EGFR1 Fab BSH-dextran. The sample buffer was changed to 5% Mannitol - 0.1% Tween80 in PBS prior to combining unlabeled and ¹⁴C labelled anti-EGFR1 Fab BSH-dextran and subsequent sterile filtration.

### Preparation of ¹⁴C labelled anti-EGFR1 Fab BSH-dextran

3 mg Fab-BSH-dextran (before ethanolamine capping) was ¹⁴C labelled by incubation with 66 µCi ¹⁴C-ethanolamine (American Radiolabeled Chemicals Inc.) in PBS containing NaCNBH₃ (as in Example 4) o/n after which the capping was finished with nonradioactive ethanolamine for 2 hours, and the low molecular weight reagents were removed as described in Example 4. This reaction resulted in ¹⁴C labelled anti-EGFR1 Fab BSH-dextran containing 9.21 µCi radioactivity.

For the animal study ¹⁴C labeled anti-EGFR1 Fab BSH dextran was mixed with unlabeled "cold" anti-EGFR1 Fab BSH dextran in portions shown in Table 10.

**Table 10. Preparation of test materials.**

| **Group** | **Amount of ¹⁴C labelled anti-EGFR1 Fab BSH-dextran (µg of Fab)** | **Amount of "cold" anti-EGFR1 Fab BSH-dextran (µg of Fab)** |
|---|---|---|
| I | 250 | 750 |
| II | 250 | 1750 |
| III | 250 | 3750 |
| IV | 250 | 5750 |
| V | 250 | 7750 |
| X | 250 | 750 |
| VIII | 250+250 | 1500 |
| IX | 250+250 | 1500 |

### In vivo experiment with ¹⁴C labelled anti-EGFR1 Fab BSH-dextran

Xenograft mice were generated as described in Example 8 except that HSC-2 cells were inoculated in right flank and the dosing was given the tumor had grown to at least 8 mm diameter in size (8 - 12 mm) corresponding roughly to tumor volume of 200-800 mm³. Radiolabeled (¹⁴C) anti-EGFR1-Fab boron conjugateswere injected either i.v. via tail vein or by intratumoral injection (Group X) in 100 µl PBS containing 5% mannitol and 0.1% polysorbate (study groups are listed in Table 10).Three mice per sample were used. Each mouse were administered about 400000 cpm of the conjugate (see above the preparation of the anti-EGFR1 Fab BSH dextran conjugates for the animal study; Table 10). Mice were sacrificed at 24h or 48h (Group IX) and organs were collected and counted for radioactivity for determination of tissue biodistribution of the conjugates. Blood samples were also collected at 30 min, 2h, and 8h after administration of boron conjugates.

Tissues were prepared for ¹⁴C quantitation as described in Example 8. Blood samples in clearance tests were prepared as in Example 8 with the exception that 200 µl of Solvable and 90 µl of H₂O₂ were used. The results are an average of three mice.

Table 11 shows tumor to blood ratios for the mice administered with ¹⁴C labelled anti-EGFR1 Fab dextran conjugate.

**Table 11. Tumor/blood ratio of ¹⁴C boron conjugate in HSC-2 tumor mice. The value for G IX is tumor/brain ratio as radioactivity in blood was determined to be 0% (all blood samples were negative after deduction of background levels). Group I: 250 µg; Group II: 500 µg; Group III 1000 µg; Group IV: 1500 µg; Group V: 2000 µg; Group X: 250 µg; Group VIII: 250 µg + 250 µg after 2 h; and Group IX: 250 µg + 250 µg after 24 h. All Groups i.v. except Group X intratumoral administration. Organs collected at 24h except Group IX at 48h. Tumor/blood ratio of Group VIII from one mouse (due to presence of one blood cpm value in the group).**

| G I | G II | G III | G IV | G V | G X | G VIII | G IX |
|---|---|---|---|---|---|---|---|
| 11.2 | 12.8 | 9.7 | 23.8 | 28.8 | 4394.3 | 9.3 | 6.2 |

**Table 12. Blood clearance of ¹⁴C boron conjugates in the three groups. Left column shows time after administration (min/h) and values are % of total injected dose / g blood.**

| | **G I** | **G III** | **G V** |
|---|---|---|---|
| **30 min** | 6.546±0.991% | 9.80910.876% | 7.486±0.235% |
| **2 h** | 1.461±0.256% | 2.80210.416% | 1.854±0.608% |
| **8 h** | 0.489±0.034% | 0.74±0.055% | 1.76±1.109% |
| **24 h** | 0.089±0.016% | 0.122±0.014% | 0.086±0.051% |

### EXAMPLE 25. In vivo experiments with anti-EGFR1 Fab BSH-dextran by direct boron quantitation

### Preparation of anti-EGFR1 Fab BSH-dextran

Anti-EGFR1 Fab BSH-dextran was prepared as described in Examples 1 and 2, respectively. The oxidation was made as described in Example 3 but in two batches; one with 80 mg, the other with 96 mg BSH-dextran. According to NMR analyses the BSH-dextran samples contained approximately 880 and 500 borons, respectively.

Anti- EGFR1 Fab fragments were prepared by papain digestion as described in Example 5. Conjugation reactions were carried out as in Example 4 but in four batches: two with 15.7 mg Anti-EGFR1 Fab and 40 mg ox-BSH-dextran, other two with 18.8 mg Anti-EGFR1 Fab and 48 mg ox-BSH-dextran.

All boron conjugates were analyzed in SDS-PAGE as in Example 6 and were labeled with Alexa Fluor^{®} 488-NHS. Internalization assay with HSC-2 cells was performed with the Alexa Fluor labelled molecules as described in Example 7.

The sample buffer was changed to 5% Mannitol - 0.1% Tween80 in PBS prior to mouse trial sample preparation and sterile filtration.

### In vivo experiment with anti-EGFR Fab BSH-dextran

Xenograft mice were generated as in Example 24. Anti-EGFR Fab BSH-dextran was administered in 100 µl of mannitol/Tween/PBS solution i.v. or in 40 µl of mannitol/Tween/PBS solution intratumorally (i.t.). In i.t. administration the needle was passed into the tumor through a single injection site and moved in a fanning technique to distribute the test substance throughout the tumor. Depending on tumor size and shape, a total of three or four passes was used.

Organs were collected at 24h and blood samples were collected at 30 min, 2h, and 8h (study groups II and V).

### Quantitation of boron

Tissues were prepared for direct boron quantitation by ICP-MS as described above. Three control samples containing ~150 mg NIST reference standard 1573 tomato leaves were also digested. The digested samples were diluted to 1:10 or 1:100.

Table 13 illustrates boron in selected organs and Table 14 shows tumor to blood ratios. Intratumoral administration shows considerably higher tumor boron concentration compared to i.v. administration.

**Table 13. Biodistribution of anti-EGFR1 Fab BSH-dextran conjugates in HSC-2 tumor mice by boron quantitation. The results represent an average of four determinations +/- SEM. Study groups were: Group I: buffer only (mannitol/Tween/PBS) i.v.; Group II: 2 mg i.v.; Group III: 2 mg + dextran i.v.; Group IV: 250 µg i.t.; Group V: 2 mg i.t. Values are µg boron in g of organ. Students t-test was performed (using Statistica 12 software [StatSoft]]) for tumor boron values of Groups II vs III and for Groups IV vs V. Groups IV and V showed significant difference between boron quantities (p-value=0.009).**

| | **Group II** | **Group III** | **Group IV** | **Group V** | **Group I** |
|---|---|---|---|---|---|
| **Blood** | 0.56±0.18 | 0.87±0.14 | 0.1±0.05 | 0.22±0. 01 | 0.32±0.21 |
| **Liver** | 18.3±1.25 | 17.54±1.15 | 1.02±0.33 | 6.97±0.86 | 0.27±0.09 |
| **Kidney** | 6.57±0.57 | 6.44±0.41 | 0.87±0.27 | 3.78±0.24 | 0.76±0.31 |
| **Muscle** | 1. 87±0.34 | 1.51±0.9 | 0.56±0.25 | 0.7±0.31 | 0.87±0.51 |
| **Skin** | 2.11±0.16 | 1.46±0.14 | 0.43±0.13 | 1.27±0.85 | 0.17±0.09 |
| **Tumor** | 2.19±1.01 | 9.54±8.59 | 9.22±2.3 | 53.09±11.45 | 0.62±0.53 |
| **Spleen** | 4.95±0.9 | 5.91±0.88 | 2.01±0.5 | 1.88±0.59 | 1.34±0.53 |

**Table 14. Tumor to blood ratios +/- SEM.**

| **Group II** | **Group III** | **Group IV** | **Group V** | **Group I** |
|---|---|---|---|---|
| 10.8±7.1 | 13.6±12.5 | 131.3±40.7 | 240.8±49.4 | 4.6±3.6 |

It is obvious to a person skilled in the art that with the advancement of technology, the basic idea of the invention may be implemented in various ways. The invention and its embodiments are thus not limited to the examples described above, instead they may vary within the scope of the claims.

## Claims

1. A conjugate represented by the formula
[D-L-Y-(CH₂)ₙ-O]ₘ-P-T
wherein
T is an antibody;
P is a polymer selected from the group consisting of dextran, hyaluronic acid, hydroxyethyl starch, and cyclodextrin;
m is at least 1;
n is in the range of 2 to 10;
each Y is independently selected from the group consisting of S, NH and 1,2,3-triazolyl, wherein 1,2,3-triazolyl is optionally substituted;
each L is independently absent or comprises a linker group covalently joining D and Y; and
each D is a toxic payload molecule or a boron compound;
wherein saccharide units of the polymer have been cleaved by oxidative cleavage of a bond between two adjacent carbons substituted by a hydroxyl group; and
wherein optionally free aldehyde groups in the conjugate have been capped, reacted with an amino group of the antibody or reacted with a tracking molecule.

2. The conjugate according to claim 1, wherein the linker group comprises a peptide from 2 to 5 amino acids in length; a linker group cleavable by a lysosomal hydrolase; a peptide cleavable by cathepsin; a cleavable carbohydrate unit; a self-immolative group between the peptide and the payload molecule; at least one moiety derived from one or more saccharide units; or a linker group represented by formula -(O-CH₂-CH₂)_{q}-, wherein q is in the range of 1 to 20.

3. The conjugate according to claim 1 or 2, wherein the toxic payload molecule is selected from the group consisting of dolastatin, auristatin, doxorubicin, maytansinoid, epirubicin, and duocarmycin.

4. The conjugate according to any one of claims 1 - 3, wherein the polymer derivative is bound to the antibody via a bond formed by a reaction between at least one aldehyde group formed by oxidative cleavage of a saccharide unit of the polymer derivative and an amino group of the antibody.

5. The conjugate according to any one of claims 1 - 4, wherein the conjugate is represented by the formula
[D-L-Y'-(CH₂)_{∘}-S-(CH₂)ₙ-O]ₘ-P-T
wherein o is in the range of 1 to 10; and
each Y' is independently selected from the group consisting of NH and 1,2,3-triazolyl, wherein 1,2,3-triazolyl is optionally substituted.

6. The conjugate according to any one of claims 1 - 5, wherein the polymer is a dextran derivative comprising at least one D-glucosyl unit;
n is in the range of 3 to 10;
Y is S;
L is absent;
D is B₁₂H₁₁²⁻; and
the dextran derivative is bound to the antibody via a bond formed by a reaction between at least one aldehyde group formed by oxidative cleavage of a D-glucosyl unit of the dextran derivative and an amino group of the antibody.

7. The conjugate according to any one of claims 1 - 6, wherein the antibody is capable of binding to a target molecule selected from the group consisting of CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, epidermal growth factor receptor 1 (EGFR1), epidermal growth factor receptor 2 (HER2/neu), HER3, HER4 receptor, LFA-1, Mac1, p150.95, VLA-4, ICAM-1, VCAM, EpCAM, alpha₄/beta₇ integrin, alpha v/beta3 integrin including either alpha or beta subunits thereof, tissue factor (TF), tumor necrosis factor alpha (TNF-α), epidermal growth factor (EGF), human vascular endothelial growth factor (VEGF), glycoprotein IIb/IIIa, TGF-beta, alpha interferon (alpha-IFN), IL-8, IL-2 receptor, IgE, HIV-1 envelope glycoprotein gp120, cancer-associated high-mannose type N-glycans, blood group antigen Apo2, death receptor, flk2/flt3 receptor, obesity (OB) receptor, mpl receptor, CTLA-4, transferrin receptor, Lewis y, GD3, and a target molecule binding fragment thereof.

8. A method for preparing the conjugate according to any one of claims 1 - 7, comprising the steps of:
a) reacting at least one hydroxyl group of a polymer selected from the group consisting of dextran, hyaluronic acid, hydroxyethyl starch, and cyclodextrin, with an alkylating agent to obtain alkylated polymer,
wherein the alkylating agent has a structure represented by the formula
X-(CH₂)ₙCH=CH₂ or X-(CH₂)ₙC≡CH
wherein n is in the range from 1 to 8, and X is Br, Cl, or I;
b) conjugating a payload molecule and optionally a linker group with the modified polymer obtainable from step a) to obtain a payload molecule-modified polymer conjugate;
c1) oxidatively cleaving at least one saccharide residue of the payload molecule-modified polymer conjugate obtainable from step b) so that aldehyde groups are formed; and
c2) reacting the oxidatively cleaved payload molecule-modified polymer conjugate obtainable from step c1) with an antibody to obtain a conjugate.

9. The method according to claim 8, wherein the method comprises the step
b1) reacting the modified polymer obtainable from step a) with a compound comprising a functional group capable of reacting with an alkene or an alkyne and a secondary functional group;
wherein the compound optionally comprises a linker group, and
wherein the functional group capable of reacting with an alkene or an alkyne is optionally selected from the group consisting of sulfhydryl, amino, and azidyl;
wherein the secondary functional group is optionally independently selected from the group consisting of sulfhydryl, amino, alkenyl, alkynyl, azidyl, aldehyde, carboxyl, maleimidyl, succinimidyl and hydroxylamino; and
wherein the compound comprising a functional group capable of reacting with an alkene or an alkyne optionally has a structure according to formula Z-(CH₂)ₚ-SH, wherein Z is sulfhydryl, amino, alkenyl, alkynyl, azidyl, aldehyde, carboxyl, maleimidyl, succinimidyl or hydroxylamino, and p is in the range from 1 to 10.

10. The method according to claim 8 or 9, wherein the method comprises the step
b1') reacting the modified polymer obtainable from step b1) with a secondary compound comprising a functional group capable of reacting with the secondary functional group and a tertiary functional group; and the secondary compound optionally comprises a linker group; and
wherein the tertiary functional group is optionally selected from the group consisting of sulfhydryl, amino, and azidyl, and the secondary functional group is independently selected from the group consisting of sulfhydryl, amino, alkenyl, alkynyl, azidyl, aldehyde, carboxyl, maleimidyl, succinimidyl and hydroxylamino; and
wherein the method optionally further comprises the step
b2) reacting the modified polymer obtainable from step a), b1 or b1') with a compound comprising
a functional group capable of reacting with the alkyl group of the alkylated polymer or with the secondary functional group, and a linker group and/or a payload molecule.

11. The method according to any one of claims 8 - 10, wherein the linker group comprises a peptide from 2 to 5 amino acids in length; a linker group cleavable by a lysosomal hydrolase; a peptide cleavable by cathepsin; a cleavable carbohydrate unit; a self-immolative group between the peptide and the payload molecule; at least one moiety derived from one or more saccharide units; or a linker group represented by formula -(O-CH₂-CH₂)_{q}-, wherein q is in the range of 1 to 20.

12. The method according to any one of claims 8 - 11, wherein the toxic payload molecule is selected from the group consisting of dolastatin, auristatin, doxorubicin, maytansinoid, epirubicin, duocarmycin.

13. The method according to any one of claims 8 - 12, wherein the method comprises the steps
c1) oxidatively cleaving at least one saccharide residue of the payload molecule-modified polymer conjugate obtainable from step b), b1), b1') or b2) so that aldehyde groups are formed, wherein the at least one saccharide residue of the payload molecule-modified polymer conjugate obtainable from step b) is optionally oxidatively cleaved in step c1) using an oxidizing agent selected from the group consisting of sodium periodate, periodic acid and lead(IV) acetate; and
c2) reacting the oxidatively cleaved payload molecule-modified polymer conjugate obtainable from step c1) with an antibody to obtain a conjugate, and
optionally the steps of
c3) modifying the antibody by adding a functional group capable of reacting with the modified polymer obtainable from step a) or with the payload molecule-modified polymer conjugate obtainable from step b), b1) or b2), and
c4) reacting the antibody or the modified antibody obtainable from step c3) with the modified polymer obtainable from step a) or with the payload molecule-modified polymer conjugate obtainable from step b), b1) or b2); and
optionally the step of e) reacting the modified polymer obtainable from step a), the payload molecule-modified polymer conjugate obtainable from step b), b1) or b2) or the conjugate obtainable from step c) or c1) with a tracking molecule; and
optionally the step of f) capping unreacted aldehyde groups of the oxidatively cleaved payload molecule-modified polymer conjugate obtainable from step c2) or e), wherein optionally capping the unreacted aldehyde groups using a hydrophilic capping agent, such as ethanolamine, lysine, glycine or Tris.

14. The method according to any one of claims 8 - 13, wherein the antibody is capable of binding to a target molecule selected from the group consisting of CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, epidermal growth factor receptor 1 (EGFR1), epidermal growth factor receptor 2 (HER2/neu), HER3, HER4 receptor, LFA-1, Mac1, p150.95, VLA-4, ICAM-1, VCAM, EpCAM, alpha₄/beta₇ integrin, alpha v/beta3 integrin including either alpha or beta subunits thereof, tissue factor (TF), tumor necrosis factor alpha (TNF-α), epidermal growth factor (EGF), human vascular endothelial growth factor (VEGF), glycoprotein IIb/IIIa, TGF-beta, alpha interferon (alpha-IFN), IL-8, IL-2 receptor, IgE, HIV-1 envelope glycoprotein gp120, cancer-associated high-mannose type N-glycans, blood group antigen Apo2, death receptor, flk2/flt3 receptor, obesity (OB) receptor, mpl receptor, CTLA-4, transferrin receptor, Lewis y, GD3, and a target molecule binding fragment thereof.

15. A conjugate obtainable by the method according to any one of claims 8 - 14.

16. A pharmaceutical composition comprising the conjugate according to any one of claims 1 - 7 or 15.

17. The conjugate according to any one of claims 1 - 7 or 15 or the pharmaceutical composition according to claim 16 for use as a medicament or for use in the treatment of cancer.

## Patentansprüche

1. Konjugat, dargestellt durch die Formel
[D-L-Y-(CH₂)ₙ-O]ₘ-P-T,
wobei
T ein Antikörper ist;
P ein Polymer ist, das aus der Gruppe bestehend aus Dextran, Hyaluronsäure, Hydroxyethylstärke und Cyclodextrin ausgewählt ist;
m mindestens 1 ist;
n im Bereich von 2 bis 10 liegt;
jedes Y unabhängig aus der Gruppe bestehend aus S, NH und 1,2,3-Triazolyl ausgewählt ist, wobei 1,2,3-Triazolyl optional substituiert ist;
jedes L unabhängig fehlt oder eine Linkergruppe umfasst, die D und Y kovalent verknüpft; und jedes D ein toxisches Beladungsmolekül oder eine Borverbindung ist;
wobei Saccharid-Einheiten des Polymers durch oxidative Spaltung einer Bindung zwischen zwei benachbarten Kohlenstoffen, die durch eine Hydroxylgruppe substituiert sind, gespalten wurden; und wobei optional freie Aldehydgruppen in dem Konjugat gecappt, mit einer Aminogruppe des Antikörpers umgesetzt oder mit einem Tracking-Molekül umgesetzt wurden.

2. Konjugat nach Anspruch 1, wobei die Linkergruppe ein Peptid mit einer Länge von 2 bis 5 Aminosäuren; eine Linkergruppe, die durch eine lysosomale Hydrolase spaltbar ist; ein Peptid, das durch Cathepsin spaltbar ist; eine spaltbare Kohlenhydrat-Einheit; eine selbstimmolative Gruppe zwischen dem Peptid und dem Beladungsmolekül; mindestens einen Teil, der von einer oder mehreren Saccharid-Einheiten abgeleitet ist; oder eine Linkergruppe, die durch die Formel -(O-CH₂-CH₂)_{q}- dargestellt ist, wobei q im Bereich von 1 bis 20 liegt, umfasst.

3. Konjugat nach Anspruch 1 oder 2, wobei das toxische Beladungsmolekül aus der Gruppe bestehend aus Dolastatin, Auristatin, Doxorubicin, Maytansinoid, Epirubicin und Duocarmycin ausgewählt ist.

4. Konjugat nach einem der Ansprüche 1-3, wobei das Polymerderivat mittels einer Bindung, die durch eine Reaktion zwischen mindestens einer Aldehydgruppe, die durch oxidative Spaltung einer Saccharid-Einheit des Polymerderivats gebildet wird, und einer Aminogruppe des Antikörpers gebildet wird, an den Antikörper gebunden ist.

5. Konjugat nach einem der Ansprüche 1-4, wobei das Konjugat durch die Formel
[D-L-Y'-(CH₂)ₒ-S-(CH₂)ₙ-O]ₘ-P-T
dargestellt ist, wobei o im Bereich von 1 bis 10 liegt und
jedes Y' unabhängig aus der Gruppe bestehend aus NH und 1,2,3-Triazolyl ausgewählt ist, wobei 1,2,3-Triazolyl optional substituiert ist.

6. Konjugat nach einem der Ansprüche 1-5, wobei das Polymer ein Dextranderivat ist, das mindestens eine D-Glucosyl-Einheit umfasst;
n im Bereich von 3 bis 10 liegt;
Y S ist;
L fehlt;
D B₁₂H₁₁²⁻ ist und
das Dextranderivat mittels einer Bindung, die durch eine Reaktion zwischen mindestens einer Aldehydgruppe, die durch oxidative Spaltung einer D-Glucosyl-Einheit des Dextranderivats gebildet wird, und einer Aminogruppe des Antikörpers gebildet wird, an den Antikörper gebunden ist.

7. Konjugat nach einem der Ansprüche 1-6, wobei der Antikörper dazu fähig ist, an ein Zielmolekül zu binden, das aus der Gruppe bestehend aus CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, epidermalem Wachstumsfaktorrezeptor 1 (EGFR1), epidermalen Wachstumsfaktorrezeptor 2 (HER2/neu), HER3, HER4-Rezeptor, LFA-1, Mac1, p150.95, VLA-4, ICAM-1, VCAM, EpCAM, Alpha₄/Beta₇-Integrin, Alpha-v/Beta3-Integrin, einschließlich entweder Alpha- oder Beta-Untereinheiten davon, Gewebefaktor (TF), Tumornekrosefaktor-Alpha (TNF-α), epidermalem Wachstumsfaktor (EGF), humanem vaskulärem endothelialem Wachstumsfaktor (VEGF), Glykoprotein Ilb/Illa, TGF-Beta, Alpha-Interferon (Alpha-IFN), IL-8, IL-2-Rezeptor, IgE, HIV-1-Hüllglykoprotein gp120, krebsassoziierten N-Glykanen mit hohem Mannosegehalt vom Antigen-Typ, Blutgruppenantigen Apo2, Todesrezeptor, flk2/flt3-Rezeptor, Leptinrezeptor (OB-Rezeptor), mp1-Rezeptor, CTLA-4, Transferrinrezeptor, Lewis-γ, GD3 und einem zielmolekülbindenden Fragment davon ausgewählt ist.

8. Verfahren zur Herstellung des Konjugats nach einem der Ansprüche 1-7, umfassend die Schritte:
a) Umsetzen mindestens einer Hydroxylgruppe eines Polymers, das aus der Gruppe bestehend aus Dextran, Hyaluronsäure, Hydroxyethylstärke und Cyclodextrin ausgewählt ist, mit einem Alkylierungsmittel, um ein alkyliertes Polymer zu erhalten,
wobei das Alkylierungsmittel eine Struktur aufweist, die durch die Formel
X-(CH₂)ₙCH=CH₂ oder X-(CH₂)ₙ-C≡CH
dargestellt ist, wobei n im Bereich von 1 bis 8 liegt und X Br, Cl oder I ist;
b) Konjugieren eines Beladungsmoleküls und optional einer Linkergruppe mit dem aus Schritt a) erhältlichen modifizierten Polymer, um ein Konjugat von Beladungsmolekül und modifiziertem Polymer zu erhalten;
c1) oxidatives Spalten mindestens eines Saccharidrests des aus Schritt b) erhältlichen Konjugats von Beladungsmolekül und modifiziertem Polymer, so dass Aldehydgruppen gebildet werden; und
c2) Umsetzen des aus Schritt c1) erhältlichen oxidativ gespaltenen Konjugats von Beladungsmolekül und modifiziertem Polymer mit einem Antikörper, um ein Konjugat zu erhalten.

9. Verfahren nach Anspruch 8, wobei das Verfahren den Schritt umfasst:
b1) Umsetzen des aus Schritt a) erhältlichen modifizierten Polymers mit einer Verbindung, die eine funktionelle Gruppe umfasst, die dazu fähig ist, mit einem Alken oder einem Alkin zu reagieren, und eine sekundäre funktionelle Gruppe umfasst;
wobei die Verbindung optional eine Linkergruppe umfasst und
wobei die funktionelle Gruppe, die dazu fähig ist, mit einem Alken oder einem Alkin zu reagieren, optional aus der Gruppe bestehend aus Sulfhydryl, Amino und Azidyl ausgewählt ist;
wobei die sekundäre funktionelle Gruppe optional unabhängig aus der Gruppe bestehend aus Sulfhydryl, Amino, Alkenyl, Alkinyl, Azidyl, Aldehyd, Carboxyl, Maleinimidyl, Succinimidyl und Hydroxylamino ausgewählt ist und
wobei die Verbindung, die eine funktionelle Gruppe umfasst, die dazu fähig ist, mit einem Alken oder einem Alkin zu reagieren, optional eine Struktur gemäß der Formel Z-(CH₂)ₚ-SH aufweist, wobei Z Sulfhydryl, Amino, Alkenyl, Alkinyl, Azidyl, Aldehyd, Carboxyl, Maleinimidyl, Succinimidyl oder Hydroxylamino ist und p im Bereich von 1 bis 10 liegt.

10. Verfahren nach Anspruch 8 oder 9, wobei das Verfahren den Schritt umfasst:
b1') Umsetzen des aus Schritt b1) erhältlichen modifizierten Polymers mit einer sekundären Verbindung, die eine funktionelle Gruppe umfasst, die dazu fähig ist, mit der sekundären funktionellen Gruppe und einer tertiären funktionellen Gruppe zu reagieren; und wobei die sekundäre Verbindung optional eine Linkergruppe umfasst und
wobei die tertiäre funktionelle Gruppe optional aus der Gruppe bestehend aus Sulfhydryl, Amino und Azidyl ausgewählt ist und die sekundäre funktionelle Gruppe unabhängig aus der Gruppe bestehend aus Sulfhydryl, Amino, Alkenyl, Alkinyl, Azidyl, Aldehyd, Carboxyl, Maleinimidyl, Succinimidyl und Hydroxylamino ausgewählt ist; und
wobei das Verfahren optional weiterhin den Schritt umfasst:
b2) Umsetzen des aus Schritt a), b1) oder b1') erhältlichen modifizierten Polymers mit einer Verbindung, umfassend
eine funktionelle Gruppe, die dazu fähig ist, mit der Alkylgruppe des alkylierten Polymers oder mit der sekundären funktionellen Gruppe zu reagieren, und eine Linkergruppe und/oder ein Beladungsmolekül.

11. Verfahren nach einem der Ansprüche 8-10, wobei die Linkergruppe ein Peptid mit einer Länge von 2 bis 5 Aminosäuren; eine Linkergruppe, die durch eine lysosomale Hydrolase spaltbar ist; ein Peptid, das durch Cathepsin spaltbar ist; eine spaltbare Kohlenhydrat-Einheit; eine selbstimmolative Gruppe zwischen dem Peptid und dem Beladungsmolekül; mindestens einen Teil, der von einer oder mehreren Saccharid-Einheiten abgeleitet ist; oder eine Linkergruppe, die durch die Formel -(O-CH₂-CH₂)_{q}- dargestellt ist, wobei q im Bereich von 1 bis 20 liegt, umfasst.

12. Verfahren nach einem der Ansprüche 8-11, wobei das toxische Beladungsmolekül aus der Gruppe bestehend aus Dolastatin, Auristatin, Doxorubicin, Maytansinoid, Epirubicin, Duocarmycin ausgewählt ist.

13. Verfahren nach einem der Ansprüche 8-12, wobei das Verfahren die Schritte umfasst:
c1) oxidatives Spalten mindestens eines Saccharidrests des aus Schritt b), b1), b1') oder b2) erhältlichen Konjugats von Beladungsmolekül und modifiziertem Polymer, so dass Aldehydgruppen gebildet werden, wobei der mindestens eine Saccharidrest des aus Schritt b) erhältlichen Konjugats von Beladungsmolekül und modifiziertem Polymer optional in Schritt c1) unter Verwendung eines Oxidationsmittels, das aus der Gruppe bestehend aus Natriumperiodat, Periodsäure und Blei(IV)-acetat ausgewählt ist, oxidativ gespalten wird; und
c2) Umsetzen des aus Schritt c1) erhältlichen oxidativ gespaltenen Konjugats von Beladungsmolekül und modifiziertem Polymer mit einem Antikörper, um ein Konjugat zu erhalten, und
optional die Schritte:
c3) Modifizieren des Antikörpers durch Hinzufügen einer funktionellen Gruppe, die dazu fähig ist, mit dem aus Schritt a) erhältlichen modifizierten Polymer oder mit dem aus Schritt b), b1) oder b2) erhältlichen Konjugat von Beladungsmolekül und modifiziertem Polymer zu reagieren, und
c4) Umsetzen des Antikörpers oder des aus Schritt c3) erhältlichen modifizierten Antikörpers mit dem aus Schritt a) erhältlichen modifizierten Polymer oder mit dem aus Schritt b), b1) oder b2) erhältlichen Konjugat von Beladungsmolekül und modifiziertem Polymer; und
optional den Schritt e) Umsetzen des aus Schritt a) erhältlichen modifizierten Antikörpers, des aus Schritt b), b1) oder b2) erhältlichen Konjugats von Beladungsmolekül und modifiziertem Polymer oder des aus Schritt c) oder c1) erhältlichen Konjugats mit einem Tracking-Molekül; und
optional den Schritt f) Cappen von nicht umgesetzten Aldehydgruppen des aus Schritt c2) oder e) erhältlichen oxidativ gespaltenen Konjugats von Beladungsmolekül und modifiziertem Polymer, wobei die nicht umgesetzten Aldehydgruppen optional unter Verwendung eines hydrophilen Capping-Mittels, wie Ethanolamin, Lysin, Glycin oder Tris, gecappt werden.

14. Verfahren nach einem der Ansprüche 8-13, wobei der Antikörper dazu fähig ist, an ein Zielmolekül zu binden, das aus der Gruppe bestehend aus CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, epidermalem Wachstumsfaktorrezeptor 1 (EGFR1), epidermalen Wachstumsfaktorrezeptor 2 (HER2/neu), HER3, HER4-Rezeptor, LFA-1, Mac1, p150.95, VLA-4, ICAM-1, VCAM, EpCAM, Alpha₄/Beta₇-Integrin, Alpha-v/Beta3-Integrin, einschließlich entweder Alpha- oder Beta-Untereinheiten davon, Gewebefaktor (TF), Tumornekrosefaktor-Alpha (TNF-α), epidermalem Wachstumsfaktor (EGF), humanem vaskulärem endothelialem Wachstumsfaktor (VEGF), Glykoprotein Ilb/Illa, TGF-Beta, Alpha-Interferon (Alpha-IFN), IL-8, IL-2-Rezeptor, IgE, HIV-1-Hüllglykoprotein gp120, krebsassoziierten N-Glykanen mit hohem Mannosegehalt vom Antigen-Typ, Blutgruppenantigen Apo2, Todesrezeptor, flk2/flt3-Rezeptor, Leptinrezeptor (OB-Rezeptor), mp1-Rezeptor, CTLA-4, Transferrinrezeptor, Lewis-γ, GD3 und einem zielmolekülbindenden Fragment davon ausgewählt ist.

15. Konjugat, das durch das Verfahren nach einem der Ansprüche 8-14 erhältlich ist.

16. Pharmazeutische Zusammensetzung, die das Konjugat nach einem der Ansprüche 1-7 oder 15 umfasst.

17. Konjugat nach einem der Ansprüche 1-7 oder 15 oder pharmazeutische Zusammensetzung nach Anspruch 16 zur Verwendung als ein Arzneimittel oder zur Verwendung bei der Behandlung von Krebs.

## Revendications

1. Conjugué représenté par la formule
[D-L-Y-(CH₂)ₙ-O]ₘ-P-T
dans laquelle
T est un anticorps ;
P est un polymère choisi dans le groupe constitué par le dextrane, l'acide hyaluronique, l'hydroxyéthylamidon, et la cyclodextrine ;
m est au moins 1 ;
n est dans la plage de 2 à 10 ;
chaque Y est choisi indépendamment dans le groupe constitué par S, NH et 1,2 3-triazolyle, le groupe 1,2 3-triazolyle étant en option substitué ;
chaque L indépendamment est absent ou comprend un groupe de liaison liant D et Y par liaison covalente ; et
chaque D est une molécule toxique de charge utile ou un composé de bore ;
dans lequel des unités saccharidiques du polymère ont été clivées par coupure oxydante d'une liaison entre deux atomes de carbone adjacents substitués par un groupe hydroxy ; et
dans lequel en option des groupes aldéhyde libres dans le conjugué ont été coiffés, mis à réagir avec un groupe amino de l'anticorps ou mis en réaction avec une molécule de traçage.

2. Conjugué selon la revendication 1, dans lequel le groupe de liaison comprend un peptide ayant une longueur de 2 à 5 acides aminés ; un groupe de liaison clivable par une hydrolase lysosomale ; un peptide clivable par une cathepsine ; une unité glucidique clivable ; un groupe auto-immolateur entre le peptide et la molécule de charge utile ; au moins un fragment dérivé d'une ou de plusieurs unités saccharidiques ; ou un groupe de liaison représenté par la formule -(O-CH₂-CH₂)_{q}-, dans laquelle q est dans la plage de 1 à 20.

3. Conjugué selon la revendication 1 ou 2, dans lequel la molécule toxique de charge utile est choisie dans le groupe constitué par la dolastatine, l'auristatine, la doxorubicine, le maytansinoïde, l'épirubicine, et la duocarmycine.

4. Conjugué selon l'une quelconque des revendications 1 - 3, dans lequel le dérivé polymère est lié à l'anticorps via une liaison formée par une réaction entre au moins un groupe aldéhyde formé par coupure oxydante d'une unité saccharidique du dérivé polymère et un groupe amino de l'anticorps.

5. Conjugué selon l'une quelconque des revendications 1 - 4, le conjugué étant représenté par la formule
[D-L-Y'-(CH₂)ₒ-S-(CH₂)ₙ-O]ₘ-P-T
dans laquelle o est dans la plage de 1 à 10 ; et
chaque Y' est choisi indépendamment dans le groupe constitué par NH et 1,2 3-triazolyle, le groupe 1,2 3-triazolyle étant en option substitué.

6. Conjugué selon l'une quelconque des revendications 1 - 5, dans lequel le polymère est un dérivé de dextrane comprenant au moins une unité D-glucosyle ;
n est dans la plage de 3 à 10 ;
Y est S ;
L est absent ;
D est B₁₂H₁₁²⁻ ; et
le dérivé de dextrane est lié à l'anticorps via une liaison formée par une réaction entre au moins un groupe aldéhyde formé par coupure oxydante d'une unité D-glucosyle du dérivé de dextrane et un groupe amino de l'anticorps.

7. Conjugué selon l'une quelconque des revendications 1 - 6, dans lequel l'anticorps est capable de se lier à une molécule cible choisie dans le groupe constitué par CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, le récepteur de facteur de croissance épidermique 1 (EGFR1), le récepteur de facteur de croissance épidermique 2 (HER2/neu), HER3, le récepteur HER4, LFA-1, Mac1, p150.95, VLA-4, ICAM-1, VCAM, EpCAM, l'intégrine alpha₄/bêta₇, l'intégrine alpha v/bêta3 incluant ses sous-unités soit alpha, soit bêta, le facteur tissulaire (TF), le facteur de nécrose tumorale alpha (TNF-α), le facteur de croissance épidermique (EGF), le facteur de croissance de l'endothélium vasculaire humain (VEGF), la glycoprotéine Ilb/Illa, TGF-bêta, l'interféron alpha (alpha-IFN), IL-8, le récepteur d'IL-2, IgE, la glycoprotéine d'enveloppe de HIV-1 gp120, les N-glycanes de type à forte teneur en mannose, associés au cancer, l'antigène de groupe sanguin Apo2, le récepteur de mort cellulaire, le récepteur flk2/flt3, le récepteur d'obésité (OB), le récepteur mpl, CTLA-4, le récepteur de transferrine, Lewis y, GD3, et un fragment de liaison de molécule cible de telles molécules.

8. Procédé de préparation du conjugué selon l'une quelconque des revendications 1 - 7, comprenant les étapes consistant à :
a) faire réagir au moins un groupe hydroxy d'un polymère choisi dans le groupe constitué par le dextrane, l'acide hyaluronique, l'hydroxyéthylamidon, et la cyclodextrine, avec un agent d'alkylation, pour obtenir un polymère alkylé,
l'agent d'alkylation ayant une structure représentée par la formule
X-(CH₂)ₙCH=CH₂ ou X-(CH₂)ₙC≡CH
où n est dans la plage de 1 à 8, et X est Br, Cl, ou I ;
b) conjuguer une molécule de charge utile et en option un groupe de liaison au polymère modifié pouvant être obtenu dans l'étape a), pour obtenir un conjugué molécule de charge utile-polymère modifié ;
c1) cliver par oxydation au moins un résidu saccharidique du conjugué molécule de charge utile-polymère modifié, pouvant être obtenu dans l'étape b), de sorte que des groupes aldéhyde sont formés ; et
c2) faire réagir le conjugué molécule de charge utile-polymère modifié, clivé par oxydation, pouvant être obtenu dans l'étape c1), avec un anticorps pour obtenir un conjugué.

9. Procédé selon la revendication 8, le procédé comprenant l'étape
b1) faire réagir le polymère modifié, pouvant être obtenu dans l'étape a), avec un composé comprenant un groupe fonctionnel capable de réagir avec un alcène ou un alcyne et un groupe fonctionnel secondaire ;
dans lequel le composé comprend en option un groupe de liaison,
dans lequel le groupe fonctionnel capable de réagir avec un alcène ou un alcyne est choisi en option dans le groupe constitué par les groupes sulfhydryle, amino, et azidyle ;
dans lequel le groupe fonctionnel secondaire est en option choisi dans le groupe constitué par les groupes sulfhydryle, amino, alcényle, alcynyle, azidyle, aldéhyde, carboxy, maléimidyle, succinimidyle et hydroxylamino ; et
dans lequel le composé comprenant un groupe fonctionnel capable de réagir avec un alcène ou un alcyne a en option une structure selon la formule Z-(CH₂)ₚ-SH, dans laquelle Z est un groupe sulfhydryle, amino, alcényle, alcynyle, azidyle, aldéhyde, carboxy, maléimidyle, succinimidyle ou hydroxylamino, et p est dans la plage de 1 à 10.

10. Procédé selon la revendication 8 ou 9, le procédé comprenant l'étape
b1') faire réagir le polymère modifié, pouvant être obtenu dans l'étape b1), avec un composé secondaire comprenant un groupe fonctionnel capable de réagir avec le groupe fonctionnel secondaire et un groupe fonctionnel tertiaire ; et le composé secondaire comprend en option un groupe de liaison ; et
dans lequel le groupe fonctionnel tertiaire est en option choisi dans le groupe constitué par les groupes sulfhydryle, amino, et azidyle, et le groupe fonctionnel secondaire est choisi indépendamment dans le groupe constitué par les groupes sulfhydryle, amino, alcényle, alcynyle, azidyle, aldéhyde, carboxy, maléimidyle, succinimidyle et hydroxylamino ; et
le procédé en outre comprenant en outre en option l'étape
b2) faire réagir le polymère modifié, pouvant être obtenu dans l'étape a), b1) ou b1'), avec un composé comprenant
un groupe fonctionnel capable de réagir avec le groupe alkyle du polymère alkylé ou avec le groupe fonctionnel secondaire, et un groupe de liaison et/ou une molécule de charge utile.

11. Procédé selon l'une quelconque des revendications 8 - 10, dans lequel le groupe de liaison comprend un peptide ayant une longueur de 2 à 5 acides aminés ; un groupe de liaison clivable par une hydrolase lysosomale ; un peptide clivable par une cathepsine ; une unité glucidique clivable ; un groupe auto-immolateur entre le peptide et la molécule de charge utile ; au moins un fragment dérivé d'une ou de plusieurs unités saccharidiques ; ou un groupe de liaison représenté par la formule -(O-CH₂-CH₂)_{q}-, dans laquelle q est dans la plage de 1 à 20.

12. Procédé selon l'une quelconque des revendications 8 - 11, dans lequel la molécule toxique de charge utile est choisie dans le groupe constitué par la dolastatine, l'auristatine, la doxorubicine, le maytansinoïde, l'épirubicine, la duocarmycine.

13. Procédé selon l'une quelconque des revendications 8 - 12, le procédé comprenant les étapes
c1) cliver par oxydation au moins un résidu saccharidique du conjugué molécule de charge utile-polymère modifié, pouvant être obtenu dans l'étape b), b1), b1') ou b2), de sorte que des groupes aldéhyde sont formés, ledit au moins un résidu saccharidique du conjugué molécule de charge utile-polymère modifié, pouvant être obtenu dans l'étape b), étant en option clivé par oxydation dans l'étape c1) à l'aide d'un agent d'oxydation choisi dans le groupe constitué par le periodate de sodium, l'acide periodique et l'acétate de plomb(IV) ; et
c2) faire réagir le conjugué molécule de charge utile-polymère modifié, clivé par oxydation, pouvant être obtenu dans l'étape c1), avec un anticorps pour obtenir un conjugué, et
en option les étapes consistant à
c3) modifier l'anticorps en ajoutant un groupe fonctionnel capable de réagir avec le polymère modifié pouvant être obtenu dans l'étape a) ou avec le conjugué molécule de charge utile-polymère modifié, pouvant être obtenu dans l'étape b), b1) ou b2), et
c4) faire réagir l'anticorps ou l'anticorps modifié pouvant être obtenu dans l'étape c3) avec le polymère modifié pouvant être obtenu dans l'étape a) ou avec le conjugué molécule de charge utile-polymère modifié pouvant être obtenu dans l'étape b), b1) ou b2) ; et
en option l'étape consistant à e) faire réagir le polymère modifié pouvant être obtenu dans l'étape a), le conjugué molécule de charge utile-polymère modifié, pouvant être obtenu dans l'étape b), b1), ou b2) ou le conjugué pouvant être obtenu dans l'étape c) ou c1), avec une molécule de traçage ; et
en option l'étape consistant à f) coiffer les groupes aldéhyde n'ayant pas réagi du conjugué molécule de charge utile-polymère modifié, clivé par oxydation, pouvant être obtenu dans l'étape c2) ou e), où en option coiffer les groupes aldéhyde n'ayant pas réagi s'effectue en utilisant un agent de coiffage hydrophile, tel que l'éthanolamine, la lysine, la glycine ou le Tris.

14. Procédé selon l'une quelconque des revendications 8 - 13, dans lequel l'anticorps est capable de se lier à une molécule cible choisie dans le groupe constitué par CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, le récepteur de facteur de croissance épidermique 1 (EGFR1), le récepteur de facteur de croissance épidermique 2 (HER2/neu), HER3, le récepteur HER4, LFA-1, Mac1, p150.95, VLA-4, ICAM-1, VCAM, EpCAM, l'intégrine alpha₄/bêta₇, l'intégrine alpha v/bêta3 incluant ses sous-unités soit alpha, soit bêta, le facteur tissulaire (TF), le facteur de nécrose tumorale alpha (TNF-α, le facteur de croissance épidermique (EGF), le facteur de croissance de l'endothélium vasculaire humain (VEGF), la glycoprotéine Ilb/Illa, TGF-bêta, l'interféron alpha (alpha-IFN), IL-8, le récepteur d'IL-2, IgE, la glycoprotéine d'enveloppe de HIV-1 gp120, les N-glycanes de type à forte teneur en mannose, associés au cancer, l'antigène de groupe sanguin Apo2, le récepteur de mort cellulaire, le récepteur flk2/flt3, le récepteur d'obésité (OB), le récepteur mpl, CTLA-4, le récepteur de transferrine, Lewis y, GD3, et un fragment de liaison de molécule cible d'une telles molécules.

15. Conjugué pouvant être obtenu par le procédé selon l'une quelconque des revendications 8 - 14.

16. Composition pharmaceutique comprenant le conjugué selon l'une quelconque des revendications 1 - 7 ou 15.

17. conjugué selon l'une quelconque des revendications 1 - 7 ou 15 ou composition pharmaceutique selon la revendication 16 pour utilisation en tant que médicament ou pour utilisation dans le traitement de cancer.
